# EUROPEAN PATENT APPLICATION

(11) **EP 4 736 958 A2**
(43) Date of publication of application: **06.05.2026**
(21) Application number: 26165672.2
(22) Date of filing: 21.02.2023
(51) Int. Cl.: A61P 35/00

(54) **ANTI-5T4 ANTIBODIES AND USES THEREOF**

(30) Priority: 21.02.2022 WO PCT/CN2022/077113
(62) Divisional of application: 23755925.7
(71) Applicant: Lepu Biopharma Co., Ltd., Shanghai 201100 (CN)
(72) Inventor: LIU, Fang, Shanghai, 201112 (CN); GONG, Wenci, Shanghai, 201112 (CN); CAI, Zhijian, Shanghai, 201112 (CN); YANG, Liu, Shanghai, 201112 (CN); GAO, Shan, Shanghai, 201112 (CN); JIANG, Wenqing, Shanghai, 201112 (CN); FANG, Lei, Shanghai, 201112 (CN)
(74) Representative: Gleiss Große Schrell und Partner mbB

(57) **Abstract**

This disclosure provides anti-5T4 antibodies, variants thereof and humanized versions. The newly disclosed antibodies exhibited high affinity to the 5T4 protein and can be used to treat cancers.

## Description

### BACKGROUD

5T4 (also known as trophoblast glycoprotein, TPBG; 5T4 oncofetal trophoblast glycoprotein; and Wnt-activated inhibitory factor 1, WAIF1) is a vertebrate-specific, single-pass transmembrane protein first identified in human placental tissues. 5T4 contains a highly glycosylated, rigid core, comprising eight leucine-rich repeats (LRRs) in the extracellular domain, a transmembrane helix and a cytoplasmic region. The cytoplasmic PDZ-binding motif Ser-Asp-Val of 5T4 has been reported to interact with the PDZ domain of TIP-2/GIPC, a cytoplasmic protein that associates with vesicles located near the cell membrane. Further downstream mechanisms of signal transduction remain unknown. 5T4 has also been found to inhibit the Wnt/β-catenin signaling pathway, a key pathway in embryonic development and a major target for anticancer therapeutics.

5T4 is rarely expressed in normal adult tissues, but is present at high levels in placenta and in most common tumors, typically more than 80% of carcinomas of the kidney, breast, colon, prostate, and ovary. Thus, 5T4 has the characteristics of an oncofetal antigen, highlighting it as a possible candidate for use as a diagnostic marker or target for cancer treatment.

### SUMMARY

The present disclosure, in various embodiments, provides antibodies and antigen-binding fragments specific to the human 5T4 protein. Experimental testing shows that these newly identified antibodies can bind to the human 5T4 protein potently and specifically. Contrary to naptumomab, which is a fusion protein containing the Fab-fragment targeting 5T4 and has been evaluated in clinical trials, these newly identifies antibodies can bind to the cynomolgus monkey 5T4 protein with a comparable potency, as well.

In accordance with one embodiment of the present disclosure, provided is an antibody or antigen-binding fragment thereof which has specificity to the human 5T4 oncofetal trophoblast glycoprotein (5T4) protein and comprises a heavy chain variable region (VH) comprising a VH CDR1, a VH CDR2 and a VH CDR3, and a light chain variable region (VL) comprising a VL CDR1, a VL CDR2, and a VL CDR3.

In some embodiments, the VH CDR1, VH CDR2, VH CDR3, VL CDR1, VL CDR2 and VL CDR3 comprise, respectively, the amino acid sequences of SEQ ID NO: 41, 42 (or any one of 47-53), 43, 44, 45 and 46 (or 264 or 265). In some embodiments, the VH CDR1 comprises the amino acid sequence of SEQ ID NO: 41; the VH CDR2 comprises the amino acid sequence of SEQ ID NO: 42; the VH CDR3 comprises the amino acid sequence of SEQ ID NO: 43; the VL CDR1 comprises the amino acid sequence of SEQ ID NO: 44; the VL CDR2 comprises the amino acid sequence of SEQ ID NO: 45; and the VL CDR3 comprises the amino acid sequence of SEQ ID NO: 264 or 265. In some embodiments, the VH comprises the amino acid sequence of SEQ ID NO: 197, and the VL comprises the amino acid sequence of SEQ ID NO: 262 or 263.

In some embodiments, the VH CDR1, VH CDR2, VH CDR3, VL CDR1, VL CDR2 and VL CDR3 comprise, respectively, the amino acid sequences of SEQ ID NO: 54, 55 (or any one of 60-63, or 266 or 267), 56, 57, 58 and 59. In some embodiments, the VH CDR1, VH CDR2, VH CDR3, VL CDR1, VL CDR2 and VL CDR3 comprise, respectively, the amino acid sequences of SEQ ID NO: 64-69. In some embodiments, the VH CDR1, VH CDR2, VH CDR3, VL CDR1, VL CDR2 and VL CDR3 comprise, respectively, the amino acid sequences of SEQ ID NO: 70, 71 or 76, 72, 73, 74 or 75. In some embodiments, the VH CDR1, VH CDR2, VH CDR3, VL CDR1, VL CDR2 and VL CDR3 comprise, respectively, the amino acid sequences of SEQ ID NO: 77-82.

In some embodiments, the VH CDR1, VH CDR2, VH CDR3, VL CDR1, VL CDR2 and VL CDR3 comprise, respectively, the amino acid sequences of SEQ ID NO: 83-88. In some embodiments, the VH CDR1, VH CDR2, VH CDR3, VL CDR1, VL CDR2 and VL CDR3 comprise, respectively, the amino acid sequences of SEQ ID NO: 89-94. In some embodiments, the VH CDR1, VH CDR2, VH CDR3, VL CDR1, VL CDR2 and VL CDR3 comprise, respectively, the amino acid sequences of SEQ ID NO: 95, 96 (or any one of 101-104), 97, 98, 99 and 100. In some embodiments, the VH CDR1, VH CDR2, VH CDR3, VL CDR1, VL CDR2 and VL CDR3 comprise, respectively, the amino acid sequences of SEQ ID NO: 105-110. In some embodiments, the VH CDR1, VH CDR2, VH CDR3, VL CDR1, VL CDR2 and VL CDR3 comprise, respectively, the amino acid sequences of SEQ ID NO: 111-116.

In some embodiments, the VH CDR1, VH CDR2, VH CDR3, VL CDR1, VL CDR2 and VL CDR3 comprise, respectively, the amino acid sequences of SEQ ID NO: 117-122. In some embodiments, the VH CDR1, VH CDR2, VH CDR3, VL CDR1, VL CDR2 and VL CDR3 comprise, respectively, the amino acid sequences of SEQ ID NO: 123, 124 or 129, 125, 126, 127 and 128. In some embodiments, the VH CDR1, VH CDR2, VH CDR3, VL CDR1, VL CDR2 and VL CDR3 comprise, respectively, the amino acid sequences of SEQ ID NO: 130-135. In some embodiments, the VH CDR1, VH CDR2, VH CDR3, VL CDR1, VL CDR2 and VL CDR3 comprise, respectively, the amino acid sequences of SEQ ID NO: 136-141. In some embodiments, the VH CDR1, VH CDR2, VH CDR3, VL CDR1, VL CDR2 and VL CDR3 comprise, respectively, the amino acid sequences of SEQ ID NO: 142-147.

In some embodiments, the VH CDR1, VH CDR2, VH CDR3, VL CDR1, VL CDR2 and VL CDR3 comprise, respectively, the amino acid sequences of SEQ ID NO: 148-153. In some embodiments, the VH CDR1, VH CDR2, VH CDR3, VL CDR1, VL CDR2 and VL CDR3 comprise, respectively, the amino acid sequences of SEQ ID NO: 154-159. In some embodiments, the VH CDR1, VH CDR2, VH CDR3, VL CDR1, VL CDR2 and VL CDR3 comprise, respectively, the amino acid sequences of SEQ ID NO: 160, 161 or 166, 162, 163, 164 and 165. In some embodiments, the VH CDR1, VH CDR2, VH CDR3, VL CDR1, VL CDR2 and VL CDR3 comprise, respectively, the amino acid sequences of SEQ ID NO: 167, 168 or 173, 169, 170, 171 and 172. In some embodiments, the VH CDR1, VH CDR2, VH CDR3, VL CDR1, VL CDR2 and VL CDR3 comprise, respectively, the amino acid sequences of SEQ ID NO: 174, 175 or 180, 176, 177, 178 and 179.

Also provided is an antibody-drug conjugate comprising an antibody or fragment thereof of the present disclosure conjugated to a drug moiety. In some embodiments the drug moiety is a cytotoxic or cytostatic agent. In some embodiments, the drug moiety is a maytansinoid, an auristatin, or a macrocyclic ketone analogue. In some embodiments, the drug moiety comprises monomethyl auristatin E (MMAE) or monomethyl auristatin F (MMAF). In some embodiments, the drug moiety is attached to the antibody or fragment thereof through a linker that is hydrolyzable under acidic conditions.

In some embodiments, the VH CDR1 comprises the amino acid sequence of SEQ ID NO: 41; the VH CDR2 comprises the amino acid sequence of SEQ ID NO: 42; the VH CDR3 comprises the amino acid sequence of SEQ ID NO: 43; the VL CDR1 comprises the amino acid sequence of SEQ ID NO: 44; the VL CDR2 comprises the amino acid sequence of SEQ ID NO: 45; and the VL CDR3 comprises the amino acid sequence of SEQ ID NO: 264 or 265. In some embodiments, the VH comprises the amino acid sequence of SEQ ID NO: 197, and the VL comprises the amino acid sequence of SEQ ID NO: 262 or 263.

Also provided are multispecific antibodies comprising an antigen-binding fragment of the present disclosure and one or more antibody or antigen-binding fragment having binding specificity to a target antigen that is not 5T4.

Also provided, in another embodiment, is a chimeric antigen receptor (CAR) comprising an antigen-binding fragment of the present disclosure, a transmembrane domain, a costimulatory domain, and a CD3ξ intracellular domain.

Polynucleotides are also provided, encoding the antibody or antigen-binding fragment thereof or the CAR of the present disclosure. In some embodiments, the polynucleotide is mRNA, which is optionally chemically modified.

Methods and uses for treating cancer and inflammatory conditions are also provided, with the antibody or antigen-binding fragment thereof of the present disclosure.

### BRIEF DESCRIPTION OF THE DRAWINGS

**FIG. 1** shows the ELISA binding activity of tested anti-5T4 chimeric monospecific antibodies against human 5T4 protein.
**FIG. 2** shows the ELISA binding activity of tested anti-5T4 chimeric monospecific antibodies against cynomolgus 5T4 protein.
**FIG. 3-8** show the epitope binning of tested anti-5T4 chimeric monospecific antibodies by competitive ELISA assay.
**FIG. 9** shows that the binding activity of tested anti-5T4 chimeric mAbs against human 5T4 expressed on the surface of CHO-K1.
**FIG. 10** shows that most of humanized 14G12 antibodies have similar binding activity to human 5T4 antigen, compared to chimeric 14G12 mAb.
**FIG. 11** shows that most of tested humanized 14G12 antibodies have similar binding activity against CHOK1 overexpressing human 5T4, compared to chimeric 14G12 antibody.
**FIG. 12** shows that tested humanized 393E9 antibodies and their chimeric mAb have comparable binding activity to human 5T4 antigen.
**FIG. 13** shows that some of tested humanized 393E9 antibodies have similar or even stronger binding activity than the chimeric antibody on CHOK1-hu5T4 cells.
**FIG. 14** shows that tested humanized 159D5 antibodies and their chimeric mAb have comparable binding activity to human 5T4 antigen.
**FIG. 15** shows that tested humanized 159D5 antibodies and their chimeric antibody have comparable binding activity against CHOK1 overexpressing human 5T4.
**FIG. 16** shows that some of tested humanized 286B4 antibodies have similar binding activity to human 5T4 antigen, compared with chimeric antibody.
**FIG. 17** shows that some of tested humanized 286B4 antibodies have similar to or even stronger binding activity than their chimeric antibody on CHOK1-hu5T4 cells or MCF-7 cells.
**FIG. 18** shows that the PTM-removed antibodies have enhanced binding capacity on 5T4-expressing cells than their chimeric antibodies.
**FIG. 19** shows that the affinity maturated antibodies have stronger binding activity than their parental Hu14G12-28 antibody to human 5T4 antigen.
**FIG. 20** shows that the affinity maturated antibodies have enhanced binding capacity on 5T4-expressing cells than their parental Hu14G12-28 antibody.

### DETAILED DESCRIPTION

### Definitions

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

As used herein, an "antibody" or "antigen-binding polypeptide" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

The terms "antibody fragment" or "antigen-binding fragment", as used herein, is a portion of an antibody such as F(ab')2, F(ab)2, Fab', Fab, Fv, scFv and the like. Regardless of structure, an antibody fragment binds with the same antigen that is recognized by the intact antibody. The term "antibody fragment" includes aptamers, spiegeleisen, and diabodies. The term "antibody fragment" also includes any synthetic or genetically engineered protein that acts like an antibody by binding to a specific antigen to form a complex.

The term antibody encompasses various broad classes of polypeptides that can be distinguished biochemically. Those skilled in the art will appreciate that heavy chains are classified as gamma, mu, alpha, delta, or epsilon (γ, µ, α, δ, ε) with some subclasses among them (*e.g.,* γ1-γ4). It is the nature of this chain that determines the "class" of the antibody as IgG, IgM, IgA IgG, or IgE, respectively.

The immunoglobulin subclasses (isotypes) e.g., IgG1, IgG2, IgG3, IgG4, IgG5, etc. are well characterized and are known to confer functional specialization. Modified versions of each of these classes and isotypes are readily discernable to the skilled artisan in view of the instant disclosure and, accordingly, are within the scope of the instant disclosure. All immunoglobulin classes are clearly within the scope of the present disclosure, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000 Daltons. The four chains are typically joined by disulfide bonds in a "Y" configuration wherein the light chains bracket the heavy chains starting at the mouth of the "Y" and continuing through the variable region.

Antibodies, antigen-binding polypeptides, variants, or derivatives thereof of the disclosure include, but are not limited to, polyclonal, monoclonal, multispecific, human, humanized, primatized, or chimeric antibodies, single chain antibodies, epitope-binding fragments, *e.g.,* Fab, Fab' and F(ab')2, Fd, Fvs, single-chain Fvs (scFv), single-chain antibodies, disulfide-linked Fvs (sdFv), fragments comprising either a VK or VH domain, fragments produced by a Fab expression library, and anti- idiotypic (anti-Id) antibodies (including, *e.g.,* anti-Id antibodies to LIGHT antibodies disclosed herein). Immunoglobulin or antibody molecules of the disclosure can be of any type (*e.g.,* IgG, IgE, IgM, IgD, IgA, and IgY), class *(e.g.,* IgGl, IgG2, IgG3, IgG4, IgAl and IgA2) or subclass of immunoglobulin molecule.

As used herein, the term "chimeric antibody" will be held to mean any antibody wherein the immunoreactive region or site is obtained or derived from a first species and the constant region (which may be intact, partial or modified in accordance with the instant disclosure) is obtained from a second species. In certain embodiments the target binding region or site will be from a non-human source (*e.g.* mouse or primate) and the constant region is human.

Antibodies disclosed herein can be from any animal origin including birds and mammals. Preferably, the antibodies are human, murine, donkey, rabbit, goat, guinea pig, camel, llama, horse, or chicken antibodies. In some embodiments, the variable region may be condricthoid in origin (*e.g.,* from sharks).

As used herein, the term "recombinant" as it pertains to polypeptides or polynucleotides intends a form of the polypeptide or polynucleotide that does not exist naturally, a non-limiting example of which can be created by combining polynucleotides that would not normally occur together.

Hybridoma technology can be performed under conditions of different "stringency". In general, a low stringency hybridization reaction is carried out at about 40°C in about 10 x SSC or a solution of equivalent ionic strength/temperature. A moderate stringency hybridization is typically performed at about 50°C in about 6 x SSC, and a high stringency hybridization reaction is generally performed at about 60°C in about 1 x SSC. Hybridization reactions can also be performed under "physiological conditions" which is well known to one of skill in the art. A nonlimiting example of a physiological condition is the temperature, ionic strength, pH and concentration of Mg²⁺ normally found in a cell.

### Anti-5T4 Antibodies

As demonstrated in the appended experimental examples, the instant inventors were able to generate anti-5T4 antibodies 14G12, 393E9, 113H5, 159D5, 24F10, 493E10, 257F1, 353H11, 367B8, 389G2, 109H7, 286B4, 37G6, 267B5, 425G1, 449H9, 49C5, 119G5, 85B10 and 95F10 **(Table 1).** Also importantly, many of these antibodies exhibited greater biological activities than naptumomab (NeoTX), a benchmark fusion protein comprising a Fab portion against 5T4. Moreover, these antibodies showed cross-reactivity to cynomolgus monkey 5T4, which enables the pre-clinical evaluation moving forward.

Competitive binding experiments showed that these newly identified antibodies, along with naptumomab, can be classified into four different bins based on where they bind on the 5T4 antigen. Interestingly, only 14G12, 393E9, and 113H5 compete with naptumomab in binding to 5T4 (referred to as "Bin A", **Table 4**). Bin B includes 159D5, 24F10, and 493E10, Bin C includes 257F1, 353H11, 367B8, 389G2, and 109H7, and Bin D includes 286B4, 37G6, 267B5, 425G1, 449H9, 49C5, 119G5, 85B10 and 95F10. Sequence examination shows that certain CDRs of the antibodies in each of these bins are highly homologous and thus are contemplated to be interchangeable.

In accordance with one embodiment of the present disclosure, provided is an antibody or antigen-binding fragment thereof. In some embodiments, the antibody or antigen-binding fragment thereof has binding specificity to the human 5T4 protein. In some embodiments, the antibody or antigen-binding fragment thereof includes a heavy chain variable region (VH) that includes a VH CDR1, a VH CDR2 and a VH CDR3, and a light chain variable region (VL) that includes a VL CDR1, a VL CDR2, and a VL CDR3.

Sequence analysis revealed that some of the CDRs can potentially be modified post-translationally. To avoid post-translational modification (PTM) risks and thus simplify manufacturing, the instant disclosure designed and tested certain de-risked versions of the CDRs.

In some embodiments, provided is an antibody or antigen-binding fragment that is derived from antibody 393E9. In some embodiments, the VH CDR1 includes the amino acid sequence of SEQ ID NO: 54; the VH CDR2 includes the amino acid sequence of SEQ ID NO: 55; the VH CDR3 includes the amino acid sequence of SEQ ID NO: 56; the VL CDR1 includes the amino acid sequence of SEQ ID NO: 57; the VL CDR2 includes the amino acid sequence of SEQ ID NO: 58; and the VL CDR3 includes an amino acid sequence selected from the group consisting SEQ ID NO: 59.

In some embodiments, the VH CDR2 is PTM de-risked. In some embodiments, the VH CDR1 includes the amino acid sequence of SEQ ID NO: 54; the VH CDR2 includes the amino acid sequence of SEQ ID NO: 55, or any one of SEQ ID NO: 60-63 or 266-267; the VH CDR3 includes the amino acid sequence of SEQ ID NO: 56; the VL CDR1 includes the amino acid sequence of SEQ ID NO: 57; the VL CDR2 includes the amino acid sequence of SEQ ID NO: 58; and the VL CDR3 includes an amino acid sequence selected from the group consisting SEQ ID NO: 59.

In some embodiments, the VH CDR2 is PTM de-risked. In some embodiments, the VH CDR1 includes the amino acid sequence of SEQ ID NO: 54; the VH CDR2 includes the amino acid sequence of SEQ ID NO: 266; the VH CDR3 includes the amino acid sequence of SEQ ID NO: 56; the VL CDR1 includes the amino acid sequence of SEQ ID NO: 57; the VL CDR2 includes the amino acid sequence of SEQ ID NO: 58; and the VL CDR3 includes an amino acid sequence selected from the group consisting SEQ ID NO: 59. In some embodiments, the VH CDR2 is PTM de-risked. In some embodiments, the VH CDR1 includes the amino acid sequence of SEQ ID NO: 54; the VH CDR2 includes the amino acid sequence of SEQ ID NO: 267; the VH CDR3 includes the amino acid sequence of SEQ ID NO: 56; the VL CDR1 includes the amino acid sequence of SEQ ID NO: 57; the VL CDR2 includes the amino acid sequence of SEQ ID NO: 58; and the VL CDR3 includes an amino acid sequence selected from the group consisting SEQ ID NO: 59. Interestingly, as shown in **Example 12,** the PTM de-risked versions, in particular Hu393E9-45-P2 and Hu393E9-62-P2 both of which contained SEQ ID NO:267 as the VH CDR2, had enhanced affinity as compared to the chimeric version.

An example VH sequence includes an amino acid sequence selected from the group consisting of SEQ ID NO: 3, 215-221 and 248-256. An example VL sequence includes an amino acid sequence selected from the group consisting of SEQ ID NO: 4 and 222-226.

In some embodiments, the VH includes an amino acid sequence of any one of SEQ ID NO: 3, 215-221 and 248-256, or a sequence having at least 75%, 80%, 85%, 90%, 95% or 99% sequence identity to any one of SEQ ID NO: 3, 215-221 and 248-256, while retaining the corresponding VH CDRs or the PTM de-risked versions thereof. In some embodiments, the VL includes an amino acid sequence of any one of SEQ ID NO: 4 and 222-226, or a sequence having at least 75%, 80%, 85%, 90%, 95% or 99% sequence identity to any one of SEQ ID NO: 4 and 222-226, while retaining the corresponding VL CDRs or the PTM de-risked versions thereof.

Also provided, in some embodiments, are antibodies and antigen-binding fragments therefore that bind to the same epitope on 5T4 as 393E9. Also provided, in some embodiments, are antibodies and antigen-binding fragments therefore that competes with 393E9 in binding to 5T4.

In some embodiments, provided is an antibody or antigen-binding fragment that is derived from antibody 286B4. In some embodiments, the VH CDR1 includes the amino acid sequence of SEQ ID NO: 123; the VH CDR2 includes the amino acid sequence of SEQ ID NO: 124; the VH CDR3 includes the amino acid sequence of SEQ ID NO: 125; the VL CDR1 includes the amino acid sequence of SEQ ID NO: 126; the VL CDR2 includes the amino acid sequence of SEQ ID NO: 127; and the VL CDR3 includes an amino acid sequence selected from the group consisting SEQ ID NO: 128.

In some embodiments, the VH CDR2 is PTM de-risked. In some embodiments, the VH CDR1 includes the amino acid sequence of SEQ ID NO: 123; the VH CDR2 includes the amino acid sequence of SEQ ID NO: 124, or SEQ ID NO: 129; the VH CDR3 includes the amino acid sequence of SEQ ID NO: 125; the VL CDR1 includes the amino acid sequence of SEQ ID NO: 126; the VL CDR2 includes the amino acid sequence of SEQ ID NO: 127; and the VL CDR3 includes an amino acid sequence selected from the group consisting SEQ ID NO: 128. Interestingly, as shown in **Example 12,** the PTM de-risked versions had enhanced affinity as compared to the chimeric version.

An example VH sequence includes an amino acid sequence selected from the group consisting of SEQ ID NO: 23, 236-241 and 259-261. An example VL sequence includes an amino acid sequence selected from the group consisting of SEQ ID NO: 24 and 242-247.

In some embodiments, the VH includes an amino acid sequence of any one of SEQ ID NO: 23, 236-241 and 259-261, or a sequence having at least 75%, 80%, 85%, 90%, 95% or 99% sequence identity to any one of SEQ ID NO: 23, 236-241 and 259-261, while retaining the corresponding VH CDRs or the PTM de-risked versions thereof. In some embodiments, the VL includes an amino acid sequence of any one of SEQ ID NO: 24 and 242-247, or a sequence having at least 75%, 80%, 85%, 90%, 95% or 99% sequence identity to any one of SEQ ID NO: 24 and 242-247, while retaining the corresponding VL CDRs or the PTM de-risked versions thereof.

Also provided, in some embodiments, are antibodies and antigen-binding fragments therefore that bind to the same epitope on 5T4 as 286B4. Also provided, in some embodiments, are antibodies and antigen-binding fragments therefore that competes with 286B4 in binding to 5T4.

In some embodiments, provided is an antibody or antigen-binding fragment that is derived from antibody 14G12. In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46.

In some embodiments, the VH CDR2 is PTM de-risked. In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46.

In some embodiments, the VL CDR3 is affinity-maturated. As shown in **Example 20,** the affinity maturated antibodies Hu14G12-28-88# and Hu14G12-28-108# significantly increased binding affinity against human 5T4 protein by 9.45 fold and 7.41 fold respectively, compared to the parental 14G12-28 antibody.

In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 264. In some embodiments, the VH CDR2 is PTM de-risked. In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 265.

In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46, 264 and 265.

An example VH sequence includes an amino acid sequence selected from the group consisting of SEQ ID NO: 1, 181-184, 189-192, and 195-204. An example VL sequence includes an amino acid sequence selected from the group consisting of SEQ ID NO: 2, 185-188, 193-194, 205-214 and 262-263. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 262. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 263. Yet another example VH sequence includes an amino acid sequence selected from the group consisting of SEQ ID NO: 197. An example VL sequence includes an amino acid sequence selected from the group consisting of SEQ ID NO: 262. Yet another example VH sequence includes an amino acid sequence selected from the group consisting of SEQ ID NO: 197. An example VL sequence includes an amino acid sequence selected from the group consisting of SEQ ID NO: 263.

In some embodiments, the VH includes an amino acid sequence of any one of SEQ ID NO: 1, 181-184, 189-192, and 195-204, or a sequence having at least 75%, 80%, 85%, 90%, 95% or 99% sequence identity to any one of SEQ ID NO: 1, 181-184, 189-192, and 195-204, while retaining the corresponding VH CDRs or the PTM de-risked versions thereof. In some embodiments, the VL includes an amino acid sequence of any one of SEQ ID NO: 2, 185-188, 193-194, 205-214 and 262-263, or a sequence having at least 75%, 80%, 85%, 90%, 95% or 99% sequence identity to any one of SEQ ID NO: 2, 185-188, 193-194, 205-214 and 262-263, while retaining the corresponding VL CDRs or the PTM de-risked versions thereof. In some embodiments, the VH includes an amino acid sequence of any one of SEQ ID NO: 197, or a sequence having at least 75%, 80%, 85%, 90%, 95% or 99% sequence identity to any one of SEQ ID NO: 197, while retaining the corresponding VH CDRs or the PTM de-risked versions thereof. In some embodiments, the VL includes an amino acid sequence of any one of SEQ ID NO: 262, or a sequence having at least 75%, 80%, 85%, 90%, 95% or 99% sequence identity to any one of SEQ ID NO: 262, while retaining the corresponding VL CDRs or the affinity-maturated versions thereof. In some embodiments, the VL includes an amino acid sequence of any one of SEQ ID NO: 263, or a sequence having at least 75%, 80%, 85%, 90%, 95% or 99% sequence identity to any one of SEQ ID NO: 263, while retaining the corresponding VL CDRs or the affinity-maturated versions thereof.

Also provided, in some embodiments, are antibodies and antigen-binding fragments therefore that bind to the same epitope on 5T4 as 14G12. Also provided, in some embodiments, are antibodies and antigen-binding fragments therefore that competes with 14G12 in binding to 5T4.

In some embodiments, provided is an antibody or antigen-binding fragment that is derived from antibody 159D5. In some embodiments, the VH CDR1 includes the amino acid sequence of SEQ ID NO: 70; the VH CDR2 includes the amino acid sequence of SEQ ID NO: 71; the VH CDR3 includes the amino acid sequence of SEQ ID NO: 72; the VL CDR1 includes the amino acid sequence of SEQ ID NO: 73; the VL CDR2 includes the amino acid sequence of SEQ ID NO: 74; and the VL CDR3 includes an amino acid sequence selected from the group consisting SEQ ID NO: 75.

In some embodiments, the VH CDR2 is PTM de-risked. In some embodiments, the VH CDR1 includes the amino acid sequence of SEQ ID NO: 70; the VH CDR2 includes the amino acid sequence of SEQ ID NO: 71, or SEQ ID NO: 76; the VH CDR3 includes the amino acid sequence of SEQ ID NO: 72; the VL CDR1 includes the amino acid sequence of SEQ ID NO: 73; the VL CDR2 includes the amino acid sequence of SEQ ID NO: 74; and the VL CDR3 includes an amino acid sequence selected from the group consisting SEQ ID NO: 75. As shown in **Example 12,** the PTM de-risked version (159D50-P1) had similar performance to the chimeric antibody.

An example VH sequence includes an amino acid sequence selected from the group consisting of SEQ ID NO: 7, 227-229, and 257. An example VL sequence includes an amino acid sequence selected from the group consisting of SEQ ID NO: 8, 230-235 and 258.

In some embodiments, the VH includes an amino acid sequence of any one of SEQ ID NO: 7, 227-229, and 257, or a sequence having at least 75%, 80%, 85%, 90%, 95% or 99% sequence identity to any one of SEQ ID NO: 7, 227-229, and 257, while retaining the corresponding VH CDRs or the PTM de-risked versions thereof. In some embodiments, the VL includes an amino acid sequence of any one of SEQ ID NO: 8, 230-235 and 258, or a sequence having at least 75%, 80%, 85%, 90%, 95% or 99% sequence identity to any one of SEQ ID NO: 8, 230-235 and 258, while retaining the corresponding VL CDRs or the PTM de-risked versions thereof.

Also provided, in some embodiments, are antibodies and antigen-binding fragments therefore that bind to the same epitope on 5T4 as 159D5. Also provided, in some embodiments, are antibodies and antigen-binding fragments therefore that competes with 159D5 in binding to 5T4.

In some embodiments, provided is an antibody or antigen-binding fragment that is derived from antibody 353H11. In some embodiments, the VH CDR1 includes the amino acid sequence of SEQ ID NO: 95; the VH CDR2 includes the amino acid sequence of SEQ ID NO: 96; the VH CDR3 includes the amino acid sequence of SEQ ID NO: 97; the VL CDR1 includes the amino acid sequence of SEQ ID NO: 98; the VL CDR2 includes the amino acid sequence of SEQ ID NO: 99; and the VL CDR3 includes an amino acid sequence selected from the group consisting SEQ ID NO: 100.

In some embodiments, the VH CDR2 is PTM de-risked. In some embodiments, the VH CDR1 includes the amino acid sequence of SEQ ID NO: 95; the VH CDR2 includes the amino acid sequence of SEQ ID NO: 96, or any one of SEQ ID NO: 101-104; the VH CDR3 includes the amino acid sequence of SEQ ID NO: 97; the VL CDR1 includes the amino acid sequence of SEQ ID NO: 98; the VL CDR2 includes the amino acid sequence of SEQ ID NO: 99; and the VL CDR3 includes an amino acid sequence selected from the group consisting SEQ ID NO: 100.

An example VH sequence includes the amino acid sequence of SEQ ID NO: 15 and an example VL sequence includes the amino acid sequence of SEQ ID NO: 16. In some embodiments, the VH includes the amino acid sequence of SEQ ID NO: 15, or a sequence having at least 75%, 80%, 85%, 90%, 95% or 99% sequence identity to SEQ ID NO: 15, while retaining the corresponding VH CDRs or the PTM de-risked versions thereof. In some embodiments, the VL includes the amino acid sequence of SEQ ID NO: 16, or a sequence having at least 75%, 80%, 85%, 90%, 95% or 99% sequence identity to SEQ ID NO: 16, while retaining the corresponding VL CDRs or the PTM de-risked versions thereof.

Also provided, in some embodiments, are antibodies and antigen-binding fragments therefore that bind to the same epitope on 5T4 as 353H11. Also provided, in some embodiments, are antibodies and antigen-binding fragments therefore that competes with 353H11 in binding to 5T4.

In some embodiments, provided is an antibody or antigen-binding fragment that is derived from antibody 109H7. In some embodiments, the VH CDR1 includes the amino acid sequence of SEQ ID NO: 117; the VH CDR2 includes the amino acid sequence of SEQ ID NO: 118; the VH CDR3 includes the amino acid sequence of SEQ ID NO: 119; the VL CDR1 includes the amino acid sequence of SEQ ID NO: 120; the VL CDR2 includes the amino acid sequence of SEQ ID NO: 121; and the VL CDR3 includes an amino acid sequence selected from the group consisting SEQ ID NO: 122.

An example VH sequence includes the amino acid sequence of SEQ ID NO: 21 and an example VL sequence includes the amino acid sequence of SEQ ID NO: 22. In some embodiments, the VH includes the amino acid sequence of SEQ ID NO: 21, or a sequence having at least 75%, 80%, 85%, 90%, 95% or 99% sequence identity to SEQ ID NO: 21, while retaining the corresponding VH CDRs or the PTM de-risked versions thereof. In some embodiments, the VL includes the amino acid sequence of SEQ ID NO: 22, or a sequence having at least 75%, 80%, 85%, 90%, 95% or 99% sequence identity to SEQ ID NO: 22, while retaining the corresponding VL CDRs or the PTM de-risked versions thereof.

Also provided, in some embodiments, are antibodies and antigen-binding fragments therefore that bind to the same epitope on 5T4 as 109H7. Also provided, in some embodiments, are antibodies and antigen-binding fragments therefore that competes with 109H7 in binding to 5T4.

In some embodiments, provided is an antibody or antigen-binding fragment that is derived from antibody 49C5. In some embodiments, the VH CDR1 includes the amino acid sequence of SEQ ID NO: 154; the VH CDR2 includes the amino acid sequence of SEQ ID NO: 155; the VH CDR3 includes the amino acid sequence of SEQ ID NO: 156; the VL CDR1 includes the amino acid sequence of SEQ ID NO: 157; the VL CDR2 includes the amino acid sequence of SEQ ID NO: 158; and the VL CDR3 includes an amino acid sequence selected from the group consisting SEQ ID NO: 159.

An example VH sequence includes the amino acid sequence of SEQ ID NO: 33 and an example VL sequence includes the amino acid sequence of SEQ ID NO: 34. In some embodiments, the VH includes the amino acid sequence of SEQ ID NO: 33, or a sequence having at least 75%, 80%, 85%, 90%, 95% or 99% sequence identity to SEQ ID NO: 33, while retaining the corresponding VH CDRs or the PTM de-risked versions thereof. In some embodiments, the VL includes the amino acid sequence of SEQ ID NO: 34, or a sequence having at least 75%, 80%, 85%, 90%, 95% or 99% sequence identity to SEQ ID NO: 34, while retaining the corresponding VL CDRs or the PTM de-risked versions thereof.

Also provided, in some embodiments, are antibodies and antigen-binding fragments therefore that bind to the same epitope on 5T4 as 49C5. Also provided, in some embodiments, are antibodies and antigen-binding fragments therefore that competes with 49C5 in binding to 5T4.

Also provided are antibodies that include a set of CDR (VH CDR1, VH CDR2, VH CDR3, VL CDR1, VL CDR2 and VL CDR3) of any of the antibodies disclosed herein, such as 14G12, 393E9, 113H5, 159D5, 24F10, 493E10, 257F1, 353H11, 367B8, 389G2, 109H7, 286B4, 37G6, 267B5, 425G1, 449H9, 49C5, 119G5, 85B10 and 95F10 (**Table 1),** or PTM de-risked versions thereof. The CDR sequences are described in **Tables 1-1 to 1-41.**

In some embodiments, the VH CDR1, VH CDR2, VH CDR3, VL CDR1, VL CDR2 and VL CDR3 comprise, respectively, the amino acid sequences of SEQ ID NO: 41, 42 (or any one of 47-53), 43, 44, 45 and 46 (or 264 or 265). In some embodiments, the VH CDR1, VH CDR2, VH CDR3, VL CDR1, VL CDR2 and VL CDR3 comprise, respectively, the amino acid sequences of SEQ ID NO: 54, 55 (or any one of 60-63, or 266 or 267), 56, 57, 58 and 59. In some embodiments, the VH CDR1, VH CDR2, VH CDR3, VL CDR1, VL CDR2 and VL CDR3 comprise, respectively, the amino acid sequences of SEQ ID NO: 64-69. In some embodiments, the VH CDR1, VH CDR2, VH CDR3, VL CDR1, VL CDR2 and VL CDR3 comprise, respectively, the amino acid sequences of SEQ ID NO: 70, 71 or 76, 72, 73, 74 or 75. In some embodiments, the VH CDR1, VH CDR2, VH CDR3, VL CDR1, VL CDR2 and VL CDR3 comprise, respectively, the amino acid sequences of SEQ ID NO: 77-82.

In some embodiments, the VH CDR1, VH CDR2, VH CDR3, VL CDR1, VL CDR2 and VL CDR3 comprise, respectively, the amino acid sequences of SEQ ID NO: 83-88. In some embodiments, the VH CDR1, VH CDR2, VH CDR3, VL CDR1, VL CDR2 and VL CDR3 comprise, respectively, the amino acid sequences of SEQ ID NO: 89-94. In some embodiments, the VH CDR1, VH CDR2, VH CDR3, VL CDR1, VL CDR2 and VL CDR3 comprise, respectively, the amino acid sequences of SEQ ID NO: 95, 96 (or any one of 101-104), 97, 98, 99 and 100. In some embodiments, the VH CDR1, VH CDR2, VH CDR3, VL CDR1, VL CDR2 and VL CDR3 comprise, respectively, the amino acid sequences of SEQ ID NO: 105-110. In some embodiments, the VH CDR1, VH CDR2, VH CDR3, VL CDR1, VL CDR2 and VL CDR3 comprise, respectively, the amino acid sequences of SEQ ID NO: 111-116.

In some embodiments, the VH CDR1, VH CDR2, VH CDR3, VL CDR1, VL CDR2 and VL CDR3 comprise, respectively, the amino acid sequences of SEQ ID NO: 117-122. In some embodiments, the VH CDR1, VH CDR2, VH CDR3, VL CDR1, VL CDR2 and VL CDR3 comprise, respectively, the amino acid sequences of SEQ ID NO: 123, 124 or 129, 125, 126, 127 and 128. In some embodiments, the VH CDR1, VH CDR2, VH CDR3, VL CDR1, VL CDR2 and VL CDR3 comprise, respectively, the amino acid sequences of SEQ ID NO: 130-135. In some embodiments, the VH CDR1, VH CDR2, VH CDR3, VL CDR1, VL CDR2 and VL CDR3 comprise, respectively, the amino acid sequences of SEQ ID NO: 136-141. In some embodiments, the VH CDR1, VH CDR2, VH CDR3, VL CDR1, VL CDR2 and VL CDR3 comprise, respectively, the amino acid sequences of SEQ ID NO: 142-147.

In some embodiments, the VH CDR1, VH CDR2, VH CDR3, VL CDR1, VL CDR2 and VL CDR3 comprise, respectively, the amino acid sequences of SEQ ID NO: 148-153. In some embodiments, the VH CDR1, VH CDR2, VH CDR3, VL CDR1, VL CDR2 and VL CDR3 comprise, respectively, the amino acid sequences of SEQ ID NO: 154-159. In some embodiments, the VH CDR1, VH CDR2, VH CDR3, VL CDR1, VL CDR2 and VL CDR3 comprise, respectively, the amino acid sequences of SEQ ID NO: 160, 161 or 166, 162, 163, 164 and 165. In some embodiments, the VH CDR1, VH CDR2, VH CDR3, VL CDR1, VL CDR2 and VL CDR3 comprise, respectively, the amino acid sequences of SEQ ID NO: 167, 168 or 173, 169, 170, 171 and 172. In some embodiments, the VH CDR1, VH CDR2, VH CDR3, VL CDR1, VL CDR2 and VL CDR3 comprise, respectively, the amino acid sequences of SEQ ID NO: 174, 175 or 180, 176, 177, 178 and 179.

In some embodiments, the VH includes the amino acid sequence of SEQ ID NO: 5, or a sequence having at least 75%, 80%, 85%, 90%, 95% or 99% sequence identity to SEQ ID NO: 5, while retaining the corresponding VH CDRs or the PTM de-risked versions thereof. In some embodiments, the VL includes the amino acid sequence of SEQ ID NO: 6, or a sequence having at least 75%, 80%, 85%, 90%, 95% or 99% sequence identity to SEQ ID NO: 6, while retaining the corresponding VL CDRs or the PTM de-risked versions thereof.

In some embodiments, the VH includes the amino acid sequence of SEQ ID NO: 9, or a sequence having at least 75%, 80%, 85%, 90%, 95% or 99% sequence identity to SEQ ID NO: 9, while retaining the corresponding VH CDRs or the PTM de-risked versions thereof. In some embodiments, the VL includes the amino acid sequence of SEQ ID NO: 10, or a sequence having at least 75%, 80%, 85%, 90%, 95% or 99% sequence identity to SEQ ID NO: 10, while retaining the corresponding VL CDRs or the PTM de-risked versions thereof.

In some embodiments, the VH includes the amino acid sequence of SEQ ID NO: 11, or a sequence having at least 75%, 80%, 85%, 90%, 95% or 99% sequence identity to SEQ ID NO: 11, while retaining the corresponding VH CDRs or the PTM de-risked versions thereof. In some embodiments, the VL includes the amino acid sequence of SEQ ID NO: 12, or a sequence having at least 75%, 80%, 85%, 90%, 95% or 99% sequence identity to SEQ ID NO: 12, while retaining the corresponding VL CDRs or the PTM de-risked versions thereof.

In some embodiments, the VH includes the amino acid sequence of SEQ ID NO: 13, or a sequence having at least 75%, 80%, 85%, 90%, 95% or 99% sequence identity to SEQ ID NO: 13, while retaining the corresponding VH CDRs or the PTM de-risked versions thereof. In some embodiments, the VL includes the amino acid sequence of SEQ ID NO: 14, or a sequence having at least 75%, 80%, 85%, 90%, 95% or 99% sequence identity to SEQ ID NO: 14, while retaining the corresponding VL CDRs or the PTM de-risked versions thereof.

In some embodiments, the VH includes the amino acid sequence of SEQ ID NO: 17, or a sequence having at least 75%, 80%, 85%, 90%, 95% or 99% sequence identity to SEQ ID NO: 17, while retaining the corresponding VH CDRs or the PTM de-risked versions thereof. In some embodiments, the VL includes the amino acid sequence of SEQ ID NO: 18, or a sequence having at least 75%, 80%, 85%, 90%, 95% or 99% sequence identity to SEQ ID NO: 18, while retaining the corresponding VL CDRs or the PTM de-risked versions thereof.

In some embodiments, the VH includes the amino acid sequence of SEQ ID NO: 19, or a sequence having at least 75%, 80%, 85%, 90%, 95% or 99% sequence identity to SEQ ID NO: 19, while retaining the corresponding VH CDRs or the PTM de-risked versions thereof. In some embodiments, the VL includes the amino acid sequence of SEQ ID NO: 20, or a sequence having at least 75%, 80%, 85%, 90%, 95% or 99% sequence identity to SEQ ID NO: 20, while retaining the corresponding VL CDRs or the PTM de-risked versions thereof.

In some embodiments, the VH includes the amino acid sequence of SEQ ID NO: 25, or a sequence having at least 75%, 80%, 85%, 90%, 95% or 99% sequence identity to SEQ ID NO: 25, while retaining the corresponding VH CDRs or the PTM de-risked versions thereof. In some embodiments, the VL includes the amino acid sequence of SEQ ID NO: 26, or a sequence having at least 75%, 80%, 85%, 90%, 95% or 99% sequence identity to SEQ ID NO: 26, while retaining the corresponding VL CDRs or the PTM de-risked versions thereof.

In some embodiments, the VH includes the amino acid sequence of SEQ ID NO: 27, or a sequence having at least 75%, 80%, 85%, 90%, 95% or 99% sequence identity to SEQ ID NO: 27, while retaining the corresponding VH CDRs or the PTM de-risked versions thereof. In some embodiments, the VL includes the amino acid sequence of SEQ ID NO: 28, or a sequence having at least 75%, 80%, 85%, 90%, 95% or 99% sequence identity to SEQ ID NO: 28, while retaining the corresponding VL CDRs or the PTM de-risked versions thereof.

In some embodiments, the VH includes the amino acid sequence of SEQ ID NO: 29, or a sequence having at least 75%, 80%, 85%, 90%, 95% or 99% sequence identity to SEQ ID NO: 29, while retaining the corresponding VH CDRs or the PTM de-risked versions thereof. In some embodiments, the VL includes the amino acid sequence of SEQ ID NO: 30, or a sequence having at least 75%, 80%, 85%, 90%, 95% or 99% sequence identity to SEQ ID NO: 30, while retaining the corresponding VL CDRs or the PTM de-risked versions thereof.

In some embodiments, the VH includes the amino acid sequence of SEQ ID NO: 31, or a sequence having at least 75%, 80%, 85%, 90%, 95% or 99% sequence identity to SEQ ID NO: 31, while retaining the corresponding VH CDRs or the PTM de-risked versions thereof. In some embodiments, the VL includes the amino acid sequence of SEQ ID NO: 32, or a sequence having at least 75%, 80%, 85%, 90%, 95% or 99% sequence identity to SEQ ID NO: 32, while retaining the corresponding VL CDRs or the PTM de-risked versions thereof.

In some embodiments, the VH includes the amino acid sequence of SEQ ID NO: 35, or a sequence having at least 75%, 80%, 85%, 90%, 95% or 99% sequence identity to SEQ ID NO: 35, while retaining the corresponding VH CDRs or the PTM de-risked versions thereof. In some embodiments, the VL includes the amino acid sequence of SEQ ID NO: 36, or a sequence having at least 75%, 80%, 85%, 90%, 95% or 99% sequence identity to SEQ ID NO: 36, while retaining the corresponding VL CDRs or the PTM de-risked versions thereof.

In some embodiments, the VH includes the amino acid sequence of SEQ ID NO: 37, or a sequence having at least 75%, 80%, 85%, 90%, 95% or 99% sequence identity to SEQ ID NO: 37, while retaining the corresponding VH CDRs or the PTM de-risked versions thereof. In some embodiments, the VL includes the amino acid sequence of SEQ ID NO: 38, or a sequence having at least 75%, 80%, 85%, 90%, 95% or 99% sequence identity to SEQ ID NO: 38, while retaining the corresponding VL CDRs or the PTM de-risked versions thereof.

In some embodiments, the VH includes the amino acid sequence of SEQ ID NO: 39, or a sequence having at least 75%, 80%, 85%, 90%, 95% or 99% sequence identity to SEQ ID NO: 39, while retaining the corresponding VH CDRs or the PTM de-risked versions thereof. In some embodiments, the VL includes the amino acid sequence of SEQ ID NO: 40, or a sequence having at least 75%, 80%, 85%, 90%, 95% or 99% sequence identity to SEQ ID NO: 40, while retaining the corresponding VL CDRs or the PTM de-risked versions thereof.

Also provided, in some embodiments, are antibodies and antigen-binding fragments that include CDR sequences derived from the presently disclosed CDR sequences, with one, two or three amino acid substitutions, deletions, and/or additions.

### Antibody-Drug Conjugates

In some embodiments, the antibodies or fragments may be conjugated to therapeutic agents, prodrugs, peptides, proteins, enzymes, viruses, lipids, biological response modifiers, pharmaceutical agents, or PEG.

In one embodiment, the antibodies or fragments of the disclosure are covalently attached to a drug moiety. The drug moiety may be, or be modified to include, a group reactive with a conjugation point on the antibody. For example, a drug moiety can be attached by alkylation (*e.g.,* at the epsilon-amino group lysines or the N-terminus of antibodies), reductive amination of oxidized carbohydrate, transesterification between hydroxyl and carboxyl groups, amidation at amino groups or carboxyl groups, and conjugation to thiols.

In some embodiments, the number of drug moieties, p, conjugated per antibody molecule ranges from an average of 1 to 8; 1 to 7, 1 to 6, 1 to 5, 1 to 4, 1 to 3, or 1 to 2. In some embodiments, p ranges from an average of 2 to 8, 2 to 7, 2 to 6, 2 to 5, 2 to 4 or 2 to 3. In other embodiments, p is an average of 1, 2, 3, 4, 5, 6, 7 or 8. In some embodiments, p ranges from an average of about 1 to about 20, about 1 to about 10, about 2 to about 10, about 2 to about 9, about 1 to about 8, about 1 to about 7, about 1 to about 6, about 1 to about 5, about 1 to about 4, about 1 to about 3, or about 1 to about 2. In some embodiments, p ranges from about 2 to about 8, about 2 to about 7, about 2 to about 6, about 2 to about 5, about 2 to about 4 or about 2 to about 3.

For example, when chemical activation of the protein results in formation of free thiol groups, the protein may be conjugated with a sulfhydryl reactive agent. In one aspect, the agent is one which is substantially specific for free thiol groups. Such agents include, for example, malemide, haloacetamides (*e.g.,* iodo, bromo or chloro), haloesters (*e.g.,* iodo, bromo or chloro), halomethyl ketones (*e.g.,* iodo, bromo or chloro), benzylic halides (*e.g*., iodide, bromide or chloride), vinyl sulfone and pyridylthio.

The drug can be linked to the antibody or fragment by a linker. Suitable linkers include, for example, cleavable and non-cleavable linkers. A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit), a phenylalanine-lysine (phe-lys) linker, or maleimidocapronic-valine-citruline-p-aminobenzyloxycarbonyl (mc-Val-Cit-PABA) linker. Another linker is Sulfosuccinimidyl-4-[N-maleimidomethyl]cyclohexane-1-carboxylate (smcc). Sulfo-smcc conjugation occurs via a maleimide group which reacts with sulfhydryls (thiols, -SH), while its Sulfo-NHS ester is reactive toward primary amines (as found in Lysine and the protein or peptide N-terminus). Yet another linker is maleimidocaproyl (mc). Other suitable linkers include linkers hydrolyzable at a specific pH or a pH range, such as a hydrazone linker. Additional suitable cleavable linkers include disulfide linkers. The linker may be covalently bound to the antibody to such an extent that the antibody must be degraded intracellularly in order for the drug to be released *e.g.* the mc linker and the like.

A linker can include a group for linkage to the antibody. For example, linker can include an amino, hydroxyl, carboxyl or sulfhydryl reactive groups (*e.g*., malemide, haloacetamides (*e.g.,* iodo, bromo or chloro), haloesters (*e.g.,* iodo, bromo or chloro), halomethyl ketones (*e.g.,* iodo, bromo or chloro), benzylic halides (*e.g.,* iodide, bromide or chloride), vinyl sulfone and pyridylthio).

In some embodiments, the drug moiety is a cytotoxic or cytostatic agent, an immunosuppressive agent, a radioisotope, a toxin, or the like. The conjugate can be used for inhibiting the multiplication of a tumor cell or cancer cell, causing apoptosis in a tumor or cancer cell, or for treating cancer in a patient. The conjugate can be used accordingly in a variety of settings for the treatment of animal cancers. The conjugate can be used to deliver a drug to a tumor cell or cancer cell. Without being bound by theory, in some embodiments, the conjugate binds to or associates with a cancer cell expressing CLDN6, and the conjugate and/or drug can be taken up inside a tumor cell or cancer cell through receptor-mediated endocytosis.

In some embodiments, the drug moiety is a maytansinoid or an auristatin. In some embodiments, the drug moiety is a macrocyclic ketone analogue such as eribulin. In some embodiments, the drug moiety is a topoisomerase inhibitor, such as exatecan and exatecan derivatives.

Once inside the cell, one or more specific peptide sequences within the conjugate (*e.g.,* in a linker) are hydrolytically cleaved by one or more tumor-cell or cancer-cell-associated proteases, resulting in release of the drug. The released drug is then free to migrate within the cell and induce cytotoxic or cytostatic or other activities. In some embodiments, the drug is cleaved from the antibody outside the tumor cell or cancer cell, and the drug subsequently penetrates the cell, or acts at the cell surface.

Examples of drug moieties or payloads are selected from the group consisting of eribulin (2-(3-Amino-2-hydroxypropyl)hexacosahydro-3-methoxy- 26-methyl-20,27-bis(methylene)11,15-18,21-24,28-triepoxy- 7,9-ethano-12,15-methano-9H,15H-furo(3,2-i)furo(2',3'-5,6) pyrano(4,3-b)(1,4)dioxacyclopentacosin-5-(4H)-one), DM1 (maytansine, N2'-deacetyl-N2'-(3-mercapto-1-oxopropyl)- or N2'-deacetyl-N2'-(3-mercapto-1-oxopropyl)-maytansine), mc-MMAD (6-maleimidocaproyl-monomethylauristatin-D or N-methyl-L-valyl-N-[(1S,2R)-2-methoxy-4-[(2S)-2-[(1R,2R)-1-methoxy-2-methyl-3-oxo-3-[[(1S)-2-phenyl-1-(2-thiazolyl)ethyl]amino]propyl]-1-pyr rolidinyl]-1-[(1S)-1-methylpropyl]-4-oxobutyl]-N-methyl-(9Cl)-L-valinamide), mc-MMAF (maleimidocaproyl-monomethylauristatin F or N-[6-(2,5-dihydro-2,5-dioxo-1H-pyrrol-1-yl)-1-oxohexyl]-N-methyl-L-valyl-L-valyl-(3R,4S,5S)-3-methoxy-5-methyl-4-(methylamino)heptanoyl-(αR, βR,2S)-β-methoxy-α-methyl-2-pyrrolidinepropanoyl-L-phenylalanine) and mc-Val-Cit-PABA-MMAE (6-maleimidocaproyl-ValcCit-(p-aminobenzyloxycarbonyl)-monomethylauristatin E or N-[[[4-[[N-[6-(2,5-dihydro-2,5-dioxo-1H-pyrrol-1-yl)-1-oxohexyl]-L-valyl-N5-(aminocarbonyl)-L-ornithyl]amino]phenyl]methoxy]carbonyl]-N-meth yl-L-valyl-N-[(1S,2R)-4-[(2S)-2-[(1R,2R)-3-[[(1R,2S)-2-hydroxy-1-methyl-2-phenylethyl]amino]-1-methoxy-2-methyl-3-oxopropyl]-1-pyrrolidinyl] -2-methoxy-1-[(1S)-1-methylpropyl]-4-oxobutyl]-N-methyl-L-valinamide). DM1 is a derivative of the tubulin inhibitor maytansine while MMAD, MMAE, and MMAF are auristatin derivatives. In some embodiments, the drug moiety is selected from the group consisting of mc-MMAF and mc-Val-Cit-PABA-MMAE.

The antibodies or fragments may be conjugated or fused to a therapeutic agent, which may include detectable labels such as radioactive labels, an immunomodulator, a hormone, an enzyme, an oligonucleotide, a photoactive therapeutic or diagnostic agent, a cytotoxic agent, which may be a drug or a toxin, an ultrasound enhancing agent, a nonradioactive label, a combination thereof and other such agents known in the art.

The antibodies can be detectably labeled by coupling it to a chemiluminescent compound. The presence of the chemiluminescent-tagged antigen-binding polypeptide is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The antibodies can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA). Techniques for conjugating various moieties to an antibody are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. (1985); Hellstrom et al., "Antibodies For Drug Delivery", in Controlled Drug Delivery (2nd Ed.), Robinson et al., (eds.), Marcel Dekker, Inc., pp. 623- 53 (1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), Academic Press pp. 303-16 (1985), and Thorpe et al., "The Preparation And Cytotoxic Properties Of Antibody-Toxin Conjugates", Immunol. Rev. (52:119-58 (1982)).

It is understood that any antibody or antigen-binding fragment of the present disclosure is suitable for inclusion in an antibody-drug conjugate (ADC) as currently disclosed. In one embodiment, the antibody or fragment includes the VH and VL CDRs of any of antibodies 14G12, 393E9, 113H5, 159D5, 24F10, 493E10, 257F1, 353H11, 367B8, 389G2, 109H7, 286B4, 37G6, 267B5, 425G1, 449H9, 49C5, 119G5, 85B10 or 95F10, or their PTM de-risked or affinity-maturated versions.

In some embodiments, the antibody or fragment includes the VH and VL CDRs of any of antibodies of Bin A (14G12, 393E9, and 113H5). In some embodiments, the antibody or fragment includes the VH and VL CDRs of any of antibodies of Bin B (159D5, 24F10, and 493E10). In some embodiments, the antibody or fragment includes the VH and VL CDRs of any of antibodies of Bin C (257F1, 353H11, 367B8, 389G2, and 109H7). In some embodiments, the antibody or fragment includes the VH and VL CDRs of any of antibodies of Bin D (286B4, 37G6, 267B5, 425G1, 449H9, 49C5, 119G5, 85B10 and 95F10).

In some embodiments, the antibody or antigen-binding fragment of the ADC includes a VH CDR1 that includes the amino acid sequence of SEQ ID NO: 41; a VH CDR2 that includes the amino acid sequence of SEQ ID NO: 42; a VH CDR3 that includes the amino acid sequence of SEQ ID NO: 43; a VL CDR1 that includes the amino acid sequence of SEQ ID NO: 44; a VL CDR2 that includes the amino acid sequence of SEQ ID NO: 45; and a VL CDR3 that includes an amino acid sequence selected from the group consisting SEQ ID NO: 46.

In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46.

In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 264. In some embodiments, the VH CDR2 is PTM de-risked. In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 265.

In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46, 264 and 265.

An example VH sequence includes an amino acid sequence selected from the group consisting of SEQ ID NO: 1, 181-184, 189-192, and 195-204. An example VL sequence includes an amino acid sequence selected from the group consisting of SEQ ID NO: 2, 185-188, 193-194, 205-214 and 262-263. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 262. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 263. Yet another example VH sequence includes an amino acid sequence selected from the group consisting of SEQ ID NO: 197. An example VL sequence includes an amino acid sequence selected from the group consisting of SEQ ID NO: 262. Yet another example VH sequence includes an amino acid sequence selected from the group consisting of SEQ ID NO: 197. An example VL sequence includes an amino acid sequence selected from the group consisting of SEQ ID NO: 263.

In some embodiments, the VH includes an amino acid sequence of any one of SEQ ID NO: 1, 181-184, 189-192, and 195-204, or a sequence having at least 75%, 80%, 85%, 90%, 95% or 99% sequence identity to any one of SEQ ID NO: 1, 181-184, 189-192, and 195-204, while retaining the corresponding VH CDRs or the PTM de-risked versions thereof. In some embodiments, the VL includes an amino acid sequence of any one of SEQ ID NO: 2, 185-188, 193-194, 205-214 and 262-263, or a sequence having at least 75%, 80%, 85%, 90%, 95% or 99% sequence identity to any one of SEQ ID NO: 2, 185-188, 193-194, 205-214 and 262-263, while retaining the corresponding VL CDRs or the PTM de-risked or affinity-maturated versions thereof. In some embodiments, the VH includes an amino acid sequence of any one of SEQ ID NO: 197, or a sequence having at least 75%, 80%, 85%, 90%, 95% or 99% sequence identity to any one of SEQ ID NO: 197, while retaining the corresponding VH CDRs or the PTM de-risked versions thereof. In some embodiments, the VL includes an amino acid sequence of any one of SEQ ID NO: 262, or a sequence having at least 75%, 80%, 85%, 90%, 95% or 99% sequence identity to any one of SEQ ID NO: 262, while retaining the corresponding VL CDRs or the affinity-maturated versions thereof. In some embodiments, the VL includes an amino acid sequence of any one of SEQ ID NO: 263, or a sequence having at least 75%, 80%, 85%, 90%, 95% or 99% sequence identity to any one of SEQ ID NO: 263, while retaining the corresponding VL CDRs or the affinity-maturated versions thereof.

### Multi-functional Molecules

Multi-functional molecules that include an antibody or antigen-binding fragment specific to 5T4, such as those disclosed herein, and one or more antibody or antigen-binding fragment having specificity to a second antigen.

In some embodiments, the second antigen is a protein expressed on an immune cell, such as a T cell, a B cell, a monocyte, a macrophage, a neutrophil, a dendritic cell, a phagocyte, a natural killer cell, an eosinophil, a basophil, and a mast cell.

In some embodiments, the second antigen is CD3, CD47, PD1, PD-L1, LAG3, TIM3, CTLA4, VISTA, CSFR1, A2AR, CD73, CD39, CD40, CEA, HER2, CMET, 4-1BB, OX40, SIRPA CD16, CD28, ICOS, CTLA4, BTLA, TIGIT, HVEM, CD27, VEGFR, or VEGF.

Different formats of bispecific antibodies are also provided. In some embodiments, each of the anti-5T4 fragment and the second fragment each is independently selected from a Fab fragment, a single-chain variable fragment (scFv), or a single-domain antibody. In some embodiments, the bispecific antibody further includes a Fc fragment.

Bifunctional molecules that include not just antibody or antigen binding fragment are also provided. As a tumor antigen targeting molecule, an antibody or antigen-binding fragment specific to 5T4, such as those described here, can be combined with an immune cytokine or ligand optionally through a peptide linker. The linked immune cytokines or ligands include, but not limited to, IL-2, IL-3, IL-4, IL-5, IL-6, IL-7, IL-10, IL-12, IL-13, IL-15, GM-CSF, TNF-α, CD40L, OX40L, CD27L, CD30L, 4-1BBL, LIGHT and GITRL. Such bi-functional molecules can combine the immune checkpoint blocking effect with tumor site local immune modulation.

In some embodiments, the anti-5T4 fragment includes the VH and VL CDRs of any of antibodies of Bin A (14G12, 393E9, and 113H5). In some embodiments, the anti-5T4 fragment includes the VH and VL CDRs of any of antibodies of Bin B (159D5, 24F10, and 493E10). In some embodiments, the anti-5T4 fragment includes the VH and VL CDRs of any of antibodies of Bin C (257F1, 353H11, 367B8, 389G2, and 109H7). In some embodiments, the anti-5T4 fragment includes the VH and VL CDRs of any of antibodies of Bin D (286B4, 37G6, 267B5, 425G1, 449H9, 49C5, 119G5, 85B10 and 95F10).

In some embodiments, the anti-5T4 fragment includes a VH CDR1 that includes the amino acid sequence of SEQ ID NO: 41; a VH CDR2 that includes the amino acid sequence of SEQ ID NO: 42; a VH CDR3 that includes the amino acid sequence of SEQ ID NO: 43; a VL CDR1 that includes the amino acid sequence of SEQ ID NO: 44; a VL CDR2 that includes the amino acid sequence of SEQ ID NO: 45; and a VL CDR3 that includes an amino acid sequence selected from the group consisting SEQ ID NO: 46.

In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46.

In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 264. In some embodiments, the VH CDR2 is PTM de-risked. In some embodiments, the VH CDR1 includes the amino acid sequence of SEQ ID NO: 41; the VH CDR2 includes the amino acid sequence of SEQ ID NO: 42; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 265.

In some embodiments, the VH CDR1 includes the amino acid sequence of SEQ ID NO: 41; the VH CDR2 includes the amino acid sequence of SEQ ID NO: 42, or any one of SEQ ID NO: 47-53; the VH CDR3 includes the amino acid sequence of SEQ ID NO: 43; the VL CDR1 includes the amino acid sequence of SEQ ID NO: 44; the VL CDR2 includes the amino acid sequence of SEQ ID NO: 45; and the VL CDR3 includes an amino acid sequence selected from the group consisting SEQ ID NO: 46, 264 and 265.

An example VH sequence includes an amino acid sequence selected from the group consisting of SEQ ID NO: 1, 181-184, 189-192, and 195-204. An example VL sequence includes an amino acid sequence selected from the group consisting of SEQ ID NO: 2, 185-188, 193-194, 205-214 and 262-263. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 262. Another example VH sequence includes an amino acid sequence selected from the group consisting of SEQ ID NO: 1, 181-184, 189-192, and 195-204. An example VL sequence includes an amino acid sequence selected from the group consisting of SEQ ID NO: 263. Yet another example VH sequence includes an amino acid sequence selected from the group consisting of SEQ ID NO: 197. An example VL sequence includes an amino acid sequence selected from the group consisting of SEQ ID NO: 262. Yet another example VH sequence includes an amino acid sequence selected from the group consisting of SEQ ID NO: 197. An example VL sequence includes an amino acid sequence selected from the group consisting of SEQ ID NO: 263.

In some embodiments, the VH includes an amino acid sequence of any one of SEQ ID NO: 1, 181-184, 189-192, and 195-204, or a sequence having at least 75%, 80%, 85%, 90%, 95% or 99% sequence identity to any one of SEQ ID NO: 1, 181-184, 189-192, and 195-204, while retaining the corresponding VH CDRs or the PTM de-risked versions thereof. In some embodiments, the VL includes an amino acid sequence of any one of SEQ ID NO: 2, 185-188, 193-194, 205-214 and 262-263, or a sequence having at least 75%, 80%, 85%, 90%, 95% or 99% sequence identity to any one of SEQ ID NO: 2, 185-188, 193-194, 205-214 and 262-263, while retaining the corresponding VL CDRs or the PTM de-risked or affinity-maturated versions thereof. In some embodiments, the VH includes an amino acid sequence of any one of SEQ ID NO: 197, or a sequence having at least 75%, 80%, 85%, 90%, 95% or 99% sequence identity to any one of SEQ ID NO: 197, while retaining the corresponding VH CDRs or the PTM de-risked versions thereof. In some embodiments, the VL includes an amino acid sequence of any one of SEQ ID NO: 262, or a sequence having at least 75%, 80%, 85%, 90%, 95% or 99% sequence identity to any one of SEQ ID NO: 262, while retaining the corresponding VL CDRs or the affinity-maturated versions thereof. In some embodiments, the VL includes an amino acid sequence of any one of SEQ ID NO: 263, or a sequence having at least 75%, 80%, 85%, 90%, 95% or 99% sequence identity to any one of SEQ ID NO: 263, while retaining the corresponding VL CDRs or the affinity-maturated versions thereof.

### Chimeric Antigen Receptors

Also provided, in one embodiment, is a chimeric antigen receptor (CAR) that includes the antibody or fragment thereof of the present disclosure as a targeting unit. In some embodiments, the CAR includes an antibody or fragment thereof of the present disclosure, a transmembrane domain, a costimulatory domain, and a CD3ξ intracellular domain.

A transmembrane domain can be designed to be fused to the extracellular domain which includes the antibody or fragment, optionally through a hinge domain. It can similarly be fused to an intracellular domain, such as a costimulatory domain. In some embodiments, the transmembrane domain can include the natural transmembrane region of a costimulatory domain (*e.g.,* the TM region of a CD28T or 4- 1BB employed as a costimulatory domain) or the natural transmembrane domain of a hinge region (*e.g.,* the TM region of a CD8 alpha or CD28T employed as a hinge domain).

In some embodiments, the transmembrane domain can include a sequence that spans a cell membrane, but extends into the cytoplasm of a cell and/or into the extracellular space. For example, a transmembrane can include a membrane-spanning sequence which itself can further include 1, 2, 3, 4, 5, 6, 7, 8, 9, 10 or more amino acids that extend into the cytoplasm of a cell, and/or the extracellular space. Thus, a transmembrane domain includes a membrane-spanning region, yet can further comprise an amino acid(s) that extend beyond the internal or external surface of the membrane itself; such sequences can still be considered to be a "transmembrane domain".

In some embodiments, the transmembrane domain is fused to the cytoplasmic domain through a short linker. Optionally, the short peptide or polypeptide linker, preferably between 2 and 10 amino acids in length can form the linkage between the transmembrane domain and a proximal cytoplasmic signaling domain of the chimeric receptor. A glycine-serine doublet (GS), glycine-serine-glycine triplet (GSG), or alanine- alanine-alanine triplet (AAA) provides a suitable linker.

In some embodiments, the CAR further includes a costimulatory domain. In some embodiments, the costimulatory domain is positioned between the transmembrane domain and an activating domain. Example costimulatory domains include, but are not limited to, CD2, CD3 delta, CD3 epsilon, CD3 gamma, CD4, CD7, CD8a, CD8, CD11a (ITGAL), CD11b (ITGAM), CD11c (ITGAX), CD11d (ITGAD), CD18 (ITGB2), CD19 (B4), CD27 (T FRSF7), CD28, CD28T, CD29 (ITGB1), CD30 (TNFRSF8), CD40 (TNFRSF5), CD48 (SLAMF2), CD49a (ITGA1), CD49d (ITGA4), CD49f (ITGA6), CD66a (CEACAM1), CD66b (CEACAM8), CD66c (CEACAM6), CD66d (CEACAM3), CD66e (CEACAM5), CD69 (CLEC2), CD79A (B-cell antigen receptor complex-associated alpha chain), CD79B (B-cell antigen receptor complex- associated beta chain), CD84 (SLAMF5), CD96 (Tactile), CD 100 (SEMA4D), CD 103 (ITGAE), CD134 (OX40), CD137 (4-1BB), CD150 (SLAMF1), CD158A(KIR2DL1), CD158B1 (KIR2DL2), CD158B2 (KIR2DL3), CD158C (KIR3DP1), CD158D (KIRDL4), CD158F1 (KIR2DL5A), CD158F2 (KIR2DL5B), CD158K (KTR3DL2), CD160 (BY55), CD162 (SELPLG), CD226 (DNAM1), CD229 (SLAMF3), CD244 (SLAMF4), CD247 (CD3-zeta), CD258 (LIGHT), CD268 (BAFFR), CD270 (T FSF14), CD272 (BTLA), CD276 (B7-H3), CD279 (PD-1), CD314 ( KG2D), CD319 (SLAMF7), CD335 ( K-p46), CD336 ( K-p44), CD337 ( K-p30), CD352 (SLAMF6), CD353 (SLAMF8), CD355 (CRTAM), CD357 (TNFRSF 18), inducible T cell co-stimulator (ICOS), LFA-1 (CD 1 la/CD 18), KG2C, DAP- 10, ICAM-1, Kp80 (KLRF1), IL-2R beta, IL-2R gamma, IL-7R alpha, LFA-1, SLAMF9, LAT, GADS (GrpL), SLP-76 (LCP2), PAG1/CBP, a CD83 ligand, Fc gamma receptor, MHC class 1 molecule, MHC class 2 molecule, a TNF receptor protein, an immunoglobulin protein, a cytokine receptor, an integrin, activating NK cell receptors, a Toll ligand receptor, and fragments or combinations thereof.

In some embodiments, the cytoplasmic portion of the CAR also includes a signaling/activation domain. In one embodiment, the signaling/activation domain is the CD3ξ domain, or is an amino acid sequence having at least about 80%, 85%, 90%, 95%, 98% or 99% sequence identity to the CD3ξ domain.

### Polynucleotides, mRNA, and Methods of Expressing or Preparing Antibodies

The present disclosure also provides polynucleotides or nucleic acid molecules encoding the antibodies, variants or derivatives thereof of the disclosure, or the CAR. The polynucleotides of the present disclosure may encode the entire heavy and light chain variable regions of the antigen-binding polypeptides, variants or derivatives thereof on the same polynucleotide molecule or on separate polynucleotide molecules. Additionally, the polynucleotides of the present disclosure may encode portions of the heavy and light chain variable regions of the antigen-binding polypeptides, variants or derivatives thereof on the same polynucleotide molecule or on separate polynucleotide molecules.

In some embodiments, the polynucleotide is an mRNA molecule. In some embodiments, the mRNA can be introduced into a target cell for expressing the antibody or fragment thereof.

mRNAs may be synthesized according to any of a variety of known methods. For example, the mRNAs may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (*e.g.,* T3, T7 or SP6 RNA polymerase), DNAse I, pyrophosphatase, and/or RNAse inhibitor. The exact conditions will vary according to the specific application.

In some embodiments, for the preparation of antibody-coding mRNA, a DNA template is transcribed *in vitro.* A suitable DNA template typically has a promoter, for example a T3, T7 or SP6 promoter, for in vitro transcription, followed by desired nucleotide sequence for desired antibody encoding (*e.g.,* heavy chain or light chain encoding) mRNA and a termination signal.

Desired antibody encoding (*e.g.,* heavy chain or light chain encoding) mRNA sequence may be determined and incorporated into a DNA template using standard methods. For example, starting from a desired amino acid sequence (*e.g.,* a desired heavy chain or light chain sequence), a virtual reverse translation is carried out based on the degenerated genetic code. Optimization algorithms may then be used for selection of suitable codons. Typically, the G/C content can be optimized to achieve the highest possible G/C content on one hand, taking into the best possible account the frequency of the tRNAs according to codon usage on the other hand. The optimized RNA sequence can be established and displayed, for example, with the aid of an appropriate display device and compared with the original (wild-type) sequence. A secondary structure can also be analyzed to calculate stabilizing and destabilizing properties or, respectively, regions of the RNA.

The mRNA may be synthesized as unmodified or modified mRNA. Typically, mRNAs are modified to enhance stability. Modifications of mRNA can include, for example, modifications of the nucleotides of the RNA. A modified mRNA can thus include, for example, backbone modifications, sugar modifications or base modifications. In some embodiments, antibody encoding mRNAs (*e.g.,* heavy chain and light chain encoding mRNAs) may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as *e.g*. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, 13-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art *e.g.* from the U.S. Pat. Nos. 4,373,071, 4,401,796, 4,415,732, 4,458,066, 4,500,707, 4,668,777, 4,973,679, 5,047,524, 5,132,418, 5,153,319, 5,262,530 and 5,700,642, the disclosure of which is included here in its full scope by reference.

In some embodiments, the mRNAs (*e.g.,* heavy chain and light chain encoding mRNAs) may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (*e.g.* cytidine 5'-O-(1-thiophosphate)), boranophosphates, positively charged guanidinium groups etc., which means by replacing the phosphodiester linkage by other anionic, cationic or neutral groups.

In some embodiments, the mRNAs (*e.g.,* heavy chain and light chain encoding mRNAs) may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, the mRNAs (e.g., heavy chain and light chain encoding mRNAs) may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

Thus, in some embodiments, the mRNAs (e.g., heavy chain and light chain encoding mRNAs) include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5)A and G(5)ppp(5')G.

In some embodiments, the mRNAs (*e.g.,* heavy chain and light chain encoding mRNAs) include a 3' poly(A) tail structure. A poly-A tail on the 3' terminus of mRNA typically includes about 10 to 300 adenosine nucleotides (*e.g.,* about 10 to 200 adenosine nucleotides, about 10 to 175 adenosine nucleotides, about 10 to 150 adenosine nucleotides, about 10 to 125 adenosine nucleotides, 10 to 100 adenosine nucleotides, about 10 to 75 adenosine nucleotides, about 20 to 70 adenosine nucleotides, or about 20 to 60 adenosine nucleotides). In some embodiments, antibody encoding mRNAs (*e.g.,* heavy chain and light chain encoding mRNAs) include a 3' poly(C) tail structure. A suitable poly-C tail on the 3' terminus of mRNA typically include about 10 to 200 cytosine nucleotides (*e.g.,* about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). The poly-C tail may be added to the poly-A tail or may substitute the poly-A tail.

In some embodiments, the mRNAs (*e.g.,* heavy chain and light chain encoding mRNAs) include a 5' and/or 3' untranslated region. In some embodiments, a 5' untranslated region includes one or more elements that affect an mRNA's stability or translation, for example, an iron responsive element. In some embodiments, a 5' untranslated region may be between about 50 and 500 nucleotides in length (*e.g.,* about 50 and 400 nucleotides in length, about 50 and 300 nucleotides in length, about 50 and 200 nucleotides in length, or about 50 and 100 nucleotides in length).

In some embodiments, a 5' region of an mRNA (*e.g.,* heavy chain and light chain encoding mRNAs) includes a sequence encoding a signal peptide, such as those described herein. In particular embodiments, a signal peptide derived from human growth hormone (hGH) is incorporated in the 5' region. Typically, a signal peptide encoding sequence is linked, directly or indirectly, to the heavy chain or light chain encoding sequence at the N-terminus.

The present technology may be used to deliver any antibody known in the art and antibodies that can be produced against desired antigens using standard methods. The present invention may be used to deliver monoclonal antibodies, polyclonal antibodies, antibody mixtures or cocktails, human or humanized antibodies, chimeric antibodies, or bi-specific antibodies.

Methods of making antibodies are well known in the art and described herein. In certain embodiments, both the variable and constant regions of the antigen-binding polypeptides of the present disclosure are fully human. Fully human antibodies can be made using techniques described in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in U.S. patents: 6,150,584; 6,458,592; 6,420,140 which are incorporated by reference in their entireties.

### Treatment and Uses

As described herein, the antibodies, variants, or derivatives of the present disclosure may be used in certain treatment and diagnostic methods.

The present disclosure is further directed to antibody-based therapies which involve administering the antibodies or fragments of the disclosure to a patient such as an animal, a mammal, and a human for treating one or more of the disorders or conditions described herein. Therapeutic compounds of the disclosure include, but are not limited to, antibodies of the disclosure (including variants and derivatives thereof as described herein) and nucleic acids or polynucleotides encoding antibodies of the disclosure (including variants and derivatives thereof as described herein).

The antibodies of the disclosure can also be used to treat or inhibit cancer. As provided above, 5T4 is rarely expressed in normal adult tissues, but is present at high levels in placenta and in most common tumors, typically more than 80% of carcinomas of the kidney, breast, colon, prostate, and ovary.

Accordingly, in some embodiments, provided are methods for treating a cancer in a patient in need thereof. The method, in one embodiment, entails administering to the patient an effective amount of an antibody or fragment or antibody-drug conjugate of the present disclosure. In some embodiments, at least one of the cancer cells (*e.g.,* stromal cells) in the patient over-express 5T4.

Cellular therapies, such as chimeric antigen receptor (CAR) T-cell therapies, are also provided in the present disclosure. A suitable cell can be used, that is transduced with a vector that encodes, or put in contact with, an CAR that includes an anti-5T4 antibody of the present disclosure (or alternatively engineered to express an anti-5T4 antibody of the present disclosure). Upon such contact or engineering, the cell can then be introduced to a cancer patient in need of a treatment. The cancer patient may have a cancer of any of the types as disclosed herein. The cell (*e.g.,* T cell) can be, for instance, a tumor-infiltrating T lymphocyte, a CD4+ T cell, a CD8+ T cell, or the combination thereof, without limitation.

In some embodiments, the cell was isolated from the cancer patient him- or her-self. In some embodiments, the cell was provided by a donor or from a cell bank. When the cell is isolated from the cancer patient, undesired immune reactions can be minimized.

Non-limiting examples of cancers include bladder cancer, breast cancer, colorectal cancer, endometrial cancer, esophageal cancer, head and neck cancer, kidney cancer, leukemia, liver cancer, lung cancer, lymphoma, melanoma, pancreatic cancer, prostate cancer, and thyroid cancer. In some embodiments, the cancer is one or more of gastric, pancreatic, esophageal, ovarian, and lung cancers.

Additional diseases or conditions associated with increased cell survival, that may be treated, prevented, diagnosed and/or prognosed with the antibodies or variants, or derivatives thereof of the disclosure include, but are not limited to, progression, and/or metastases of malignancies and related disorders such as leukemia (including acute leukemias (*e.g.,* acute lymphocytic leukemia, acute myelocytic leukemia (including myeloblastic, promyelocytic, myelomonocytic, monocytic, and erythroleukemia)) and chronic leukemias (*e.g.,* chronic myelocytic (granulocytic) leukemia and chronic lymphocytic leukemia)), polycythemia vera, lymphomas (*e.g.,* Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyo sarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

A specific dosage and treatment regimen for any particular patient will depend upon a variety of factors, including the particular antibodies, variant or derivative thereof used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

Methods of administration of the antibody or fragment include but are not limited to intradermal, intramuscular, intraperitoneal, intravenous, subcutaneous, intranasal, epidural, and oral routes. The antigen-binding polypeptides or compositions may be administered by any convenient route, for example by infusion or bolus injection, by absorption through epithelial or mucocutaneous linings (*e.g.,* oral mucosa, rectal and intestinal mucosa, etc.) and may be administered together with other biologically active agents. Thus, pharmaceutical compositions containing the antigen-binding polypeptides of the disclosure may be administered orally, rectally, parenterally, intracisternally, intravaginally, intraperitoneally, topically (as by powders, ointments, drops or transdermal patch), buccally, or as an oral or nasal spray.

The term "parenteral" as used herein refers to modes of administration which include intravenous, intramuscular, intraperitoneal, intrasternal, subcutaneous and intra-articular injection and infusion.

Administration can be systemic or local. In addition, it may be desirable to introduce the antibodies of the disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.,* by use of an inhaler or nebulizer, and formulation with an aerosolizing agent.

It may be desirable to administer the antigen-binding polypeptides or compositions of the disclosure locally to the area in need of treatment; this may be achieved by, for example, and not by way of limitation, local infusion during surgery, topical application, *e.g.,* in conjunction, with a wound dressing after surgery, by injection, by means of a catheter, by means of a suppository, or by means of an implant, said implant being of a porous, nonporous, or gelatinous material, including membranes, such as sialastic membranes, or fibers. Preferably, when administering a protein, including an antibody, of the disclosure, care must be taken to use materials to which the protein does not absorb.

The amount of the antibodies or fragments or antibody-drug conjugate of the disclosure which will be effective in the treatment, inhibition and prevention of an inflammatory, immune or malignant disease, disorder or condition can be determined by standard clinical techniques. In addition, *in vitro* assays may optionally be employed to help identify optimal dosage ranges. The precise dose to be employed in the formulation will also depend on the route of administration, and the seriousness of the disease, disorder or condition, and should be decided according to the judgment of the practitioner and each patient's circumstances. Effective doses may be extrapolated from dose-response curves derived from *in vitro* or animal model test systems.

As a general proposition, the dosage administered to a patient of the antibodies or fragments of the present disclosure is typically 0.001 mg/kg to 100 mg/kg of the patient's body weight, between 0.01 mg/kg and 20 mg/kg of the patient's body weight, or 0.5 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the disclosure may be reduced by enhancing uptake and tissue penetration (*e.g.,* into the brain) of the antibodies by modifications such as, for example, lipidation.

In an additional embodiment, the compositions of the disclosure are administered in combination with cytokines. Cytokines that may be administered with the compositions of the disclosure include, but are not limited to, IL-2, IL-3, IL-4, IL-5, IL-6, IL-7, IL-10, IL-12, IL-13, IL-15, anti-CD40, CD40L, and TNF-α.

In additional embodiments, the compositions of the disclosure are administered in combination with other therapeutic or prophylactic regimens, such as, for example, radiation therapy.

### Compositions

The present disclosure also provides pharmaceutical compositions. Such compositions comprise an effective amount of an antibody or fragment or antibody-drug conjugate and an acceptable carrier. In some embodiments, the composition further includes a second anticancer agent (*e.g.,* an immune checkpoint inhibitor).

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Further, a "pharmaceutically acceptable carrier" will generally be a non-toxic solid, semisolid or liquid filler, diluent, encapsulating material or formulation auxiliary of any type.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose are also envisioned. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences by E. W. Martin, incorporated herein by reference. Such compositions will contain a therapeutically effective amount of the antigen-binding polypeptide, preferably in purified form, together with a suitable amount of carrier so as to provide the form for proper administration to the patient. The formulation should suit the mode of administration. The parental preparation can be enclosed in ampoules, disposable syringes or multiple dose vials made of glass or plastic.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the disclosure can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

### EXAMPLES

### Example 1: Generation of Mouse Monoclonal Antibodies against Human 5T4

This example describes the generation of anti-human-5T4 mouse monoclonal antibodies using the hybridoma technology.

*Antigen*: human 5T4-His protein and CHO-K1-expressing human 5T4.

Immunization: To generate mouse monoclonal antibodies targeting human 5T4, SJL mice, Balb/c mice and C57BL/6 mice were first immunized with the 5T4-His protein. The immunized mice were subsequently boosted with the 5T4-His protein or CHO-K1-expressing human 5T4. To select mice producing antibodies that bound to 5T4 protein, the serum of immunized mice was subjected to the antibody titer evaluation by ELISA and FACS. Briefly, microtiter plates were coated with human 5T4 or cyno 5T4 protein at 0.5 or 1 µg/ml in ELISA coating buffer, 100 µl/well at 4°C overnight, then blocked with 150 µl/well of 1% BSA. Dilutions of serum from immunized mice were added to each well and incubated for 1 hours at 37°C. The plates were washed with PBS/Tween and then incubate with anti-mouse IgG antibody conjugated with Horse Radish Peroxidase (HRP) for 30 min at 37°C. After washing, the plates were developed with TMB substrate and analyzed by spectrophotometer at OD 450nm. Immune responses were also tested by serum FACS against CHOK1-hu5T4 cell line with CHOK1 parental cell line served as negative control. The resulting mice were used for fusions. The hybridoma supernatants were screened by ELISA.

Cell fusion: Fusion was performed by electro fusion. Fused cells were plated into 50 96-well plates for each fusion.

Screening: The supernatants were screened by ELISA against recombinant human (rh) 5T4-His protein and recombinant cyno 5T4-His protein. Then, positive supernatants from primary screening underwent confirmative screening with FACS binding against CHOK1-hu5T4 cell line as well as protein binding with ELISA.

Subcloning and screening: Positive primary clones from each fusion were subcloned by limiting dilution to ensure that the subclones were derived from a single parental cell. Subcloning were screened in the same approach as primary clones and culture supernatant of positive clones underwent additional confirmative screening by affinity ranking.

Hybridoma clones 14G12, 393E9, 113H5, 159D5, 24F10, 493E10, 257F1, 353H11, 367B8, 389G2, 109H7, 286B4, 37G6, 267B5, 425G1, 449H9, 49C5, 119G5, 85B10 and 95F10 were selected for further analysis. The amino acid sequences of the variable regions of these clones are listed in **Table 1** below. In **Tables 1-1 to 1-20,** in addition to the original CDR sequences from this murine antibodies, versions in which potential post-translational modifications (PTM) are removed or that are affinity-matured are also included.

**Table 1. Sequences of the variable regions of selected clones**

| **Name** | **Sequence** | **SEQ ID NO:** |
|---|---|---|
| 14G12 VH | EVQLQQSGPELVKPGASVKISCKASGYAFSSSWMNWVKQRPGKGLE WIGRIYREDGHTNYNGKFKGKATLTADKSSTTAYMQLTSLTSEDSAV YFCANGGAMDYWGQGTSVTVSS | 1 |
| 14G12 VL | DIQMTQSTSSLSASLGDRVTISCRASQDISNYLNWYQQKPDGTVKLLIY YTSRLHSGVPSRFSGSGSGTDYSLTISNLEQEDIATYFCQQDNTLPWTF GGGTKLEIK | 2 |
| 393E9 VH | EVKLVESGGGLVQPGGSLSLSCAASGFTFTDYYMSWVRQPPGKALEW LGFIRNKGNGYTTENSASVKGRFTISRDNSQSILYLQMNALRAEDSAT YYCARYRGNPHYAMDYWGQGTSVTVSS | 3 |
| 393E9 VL | DIVLTQFPASLAVSLGQRATISCRASESVEFYGTSFLQWYQQKPGQPP KLLIYGASNVESGVPARFSGSGSGTDFSLSIHPVEEDDIAMYFCQQSRK VPSTFGGGTKLEIK | 4 |
| 113H5 VH | DVQLQESGPGLVKPSQSLSLTCSVTGYSITSGYYWNWIRQFPGNKLEW MGYINSDGSNNYNPSLKNRISITRDTSKNQFFLKLNSVTTEDTATYYC AREEYDYWFAYWGQGTLVTVSA | 5 |
| 113H5 VL | DIVMTQSQKFMSTSVGDRVSVTCKASQNVGTNVAWYQQKPGQSPKA LIYSASNRYSGVPDRFTGSGFGTDFTLTISNVQSEDLTEYFCQQYNSYP YTFGGGTNLEIK | 6 |
| 159D5 VH | DVQLVESGGGLVQPGGSRKLSCTASGFTFSNFGMHWVRQAPETGLE WVAYISSGSSTFYYADSVKGRFTISRDNPKNTLFLQMTSLRSEDTAMY FCARSPAYYRYGLDYWGQGTTLTVSS | 7 |
| 159D5 VL | DIVLTQSPASLAVSLGQRATISCRASQSVSSSTFSYMHWYQQKPGQPP KLLIKSSSNLESGVPARFSGSGSGTDFTLNIHPVEEEDTATYYCQHSWE IPYTFGGGTKLEIK | 8 |
| 24F10 VH | EVQLVESGGGLVKPGGSLKLSCAASGFTFSDYGMHWVRQAPEKGLE WVAYISSGTSTIYYTDTVKGRFTISRDNAKNTLFLQMTSLRSEDTAMY YCARGGDGYYRSTMDYWGQGISVTVSS | 9 |
| 24F10 VL | DIVLTQSPASLAVSLGQRATISCRASQSVSSSSYSYMHWYQQKPGQPP KLLIKSASNLASGVPARFSGSGSGTDFTLNIHPVEEEDSATYYCQHSWE VPRTFGGGTKLEIK | 10 |
| 493E10 VH | DVQLVESGGGLVQPGGSRKLSCAASGFTFSSFGMHWVRQAPEKGLE WVAYISSGSSTIYYADTVKGRFTISRDNPKNTLFLQMTSLRSEDTAMY YCARNRAYHREAMDYWGQGTSVTVSS | 11 |
| 493E10 VL | DIVLTQSPASLTVSLGQRATISCRASQSVSSSSYNYMHWYQQKPGQPP KLLIKSASNLESGVPARFSGSGSGTDFTLNIHPVEEEDTATYYCQHSWE IPYTFGGGTKLEIK | 12 |
| 257F1 VH | QVLLQQSGAEVARPGASVKLSCKASGYTFTSYGLNWLRQRTGQGLE WIGEIYPRSENTHYNEKFKGKATLTADKSSSTAYMELRSLTSEDSAV YFCARGDWDFDHWGQGTTLTVSS | 13 |
| 257F1 VL | EILLTQSPAIIPASPGEKVTITCSASSSVSYMNWYQQKPGSSPKIWIYGIS NLASGVPARFSGSGSGTFFSFTINSMEAEDVATYYCQQRSSYPRTFGG GTKLEIK | 14 |
| 353H11 VH | QVQLQQPGTELVKPGASVKLSCKASDYTFTSYWMHWVKQRPEQGLE WIGNINPSNGNTNYNEKFKNKATLTVDKSSSTAYMQLSSLTFEDSAV YYCARGGWDFDYWGQGTTLTVSS | 15 |
| 353H11 VL | QVVLTQSPAIMSASPGEKVTMTCSASSSVRYMHWYQQRSGTSPKRWI YDTSKLTSGVPARFSGSGSGTSYSLTISSMEAEDAATYYCQLWTSKPP WTFGGGTKLEIR | 16 |
| 367B8 VH | QVQLQQPGTELVKPGASVKLSCKASGYTFTSYWMHWVKQRPEQGLE WIGNINPSNGNTNYNENFKSQATLTVDKSSSTAYMQLSSLTSEDSAVY YCARGGWDFDYWGQGTTLTVSS | 17 |
| 367B8 VL | QVVLTQSPAIMSASPGEKVTMTCSASSSVRYIHWYQQRSGTSPKRWIY DTSKLTSGVPVRFSGSGSGTSYSLTISSMEAEDAATYYCQQWTSKPP WTFGGGTKLEIR | 18 |
| 389G2 VH | QVQLQQPGTELVKPGASVKLSCKASGYTFTSYWMHWVKQRPEQGLE WIGNINPSNGNTNYNERFKSKATLTVDKSSSTAYMQLSSLTSEDSTVY YCARGGWDFDYWGQGTTLTVSS | 19 |
| 389G2 VL | QVVLTQSPAIMSASPGEKVTMTCSASSSVRYIHWYQQRSGTSPKRWIY DTSKLTSGVPVRFSGSGSGTSFSLTISSMEAEDAATYYCQQWTSKPP WTFGGGTKLEIK | 20 |
| 109H7 VH | QVQLQQSGAELMKPGASVKLSCKATGYTFTGYWIEWVKQRPGQGLE WIGETLPGRGSTNYNEKFKGKATFTADTSSNIVYMQLSSLTIEDSAIY YCARGRRDFDYWGQGTTLTVSS | 21 |
| 109H7 VL | DIQMTQTTSSLSASLGDEVTISCSASQGINNYLNWYQQKPDGTVKLLI YYTSSLHSGVPSRFSGSGSGTDYSLTIINLEPEDIATYYCQQFSKLPFTF GSGTKLEIR | 22 |
| 286B4 VH | DVQLHESGPGLVKPSQSLSLTCSVTGYSITNNYYWNWIRQFPGNNLEW MGYITYDGSNNYNPSLKNRISVTRDTSKNQFFLKLSSVTTEDTATYYC TRGGGQLRFDYWGQGTTLTVSS | 23 |
| 286B4 VL | SIVMTQTPKFLIVSAGDRVTITCKASQSVSNEVTWYQQKPGQSPKMLI YYASNRYTGVPDRFTGSGYGTDFTFTINTVQAEDLAIYFCQQDYSSPW TFGGGTKLEIK | 24 |
| 37G6 VH | QVQLQQPGAELVKPGASVKMSCKASGYTFTSYWITWVKQRPGQGLE RIGDIYPGSGTINYNEKFKSKATLTVDTSSSTAYMQLSSLTSEDSAVYC CARSDGNYYFDYWGQGTTLKVSS | 25 |
| 37G6 VL | QIVLSQSPAILSASPGEKVTMTCRASSSVNYMHWYQQKPGSSPKPWIY ATSNLASGVPVRFSGSGSGTSYSLTISRVEAEDAATYYCQQWSSNPPT FGGGTKLEIK | 26 |
| 267B5 VH | DVQFQESGPGLVKPSQSLSLTCSVTGYSITSSYYWNWIRQFPGDKLEW MGYISYDGSNNYNPSLKNRISITRDTSKKQFFLKLNSVTTEDTATYYC ARSWDGGYAMDNWGQGTSVTVSS | 27 |
| 267B5 VL | SIVMTQTPKFLLVSAGDRVTITCKASQSVSNDVAWYQQKPGQSPKLLI YFASNRYTGVPDRFTGSGYGTVFTFTISTVQADDLAVYFCQQDYTSP WTFGGGTRLEIK | 28 |
| 425G1 VH | EVQLQQSGAELVKPGASVKLSCTASGFNIKDTYMHWVKQRPEQGLE WIGRIDPANGHTIFASKFQGKATITADTSSNTAYMQLSSLTSGDTAVY YCGLFTMVVVPWYFDVWGTGTTVTVSS | 29 |
| 425G1 VL | DIVMTQSHKFMSTSVGDRVSITCKASQDVSTAVAWYQQKPGQSPKLLI YWASTRHTGVPDRFTGSGSGTDYTLTISSVQAEDLALYYCQQHYSSP LTFGAGTKLELK | 30 |
| 449H9 VH | QVQLQQSGAELVRPGSSVRISCKASGYAFSNYWMNWVKQRPGQGLE WIGQIYPGDGDTNYNGKFKNKATLTTDKSSSTAYMQLSSLTSEDSAV YFCARHYDYPYYYAMDYWGQGTSVTVSS | 31 |
| 449H9 VL | DIQMTQSPSSLSASLGERVSLTCRASQDIGIALTWLQQEPDGTIKRLIY ATSSLDSGVPKRFSGSRSGSDYTLTISSLESEDFVDYYCLQYIISPYTFG GGTKLEIK | 32 |
| 49C5 VH | EVQLQQSGPELVKPGASVKISCKASGYTFTDYYMNWVKQSHGKSLEW IGDINPYSGGATNNQKFKGKATLTVDKSSSTAYIELRSLTSEDSAVYF CARGSIYYDSTANYFDYWGQGTTLTVSS | 33 |
| 49C5 VL | DIQMTQSPASLSASVGETVTITC**RASENIYSYLA**WYQQKQGKSPQLLV Y**NAKSLTE**GVPSRFSGSRSGTQFSLKINSLQPEDFGSYYC**QHHYVTPW** **T**FGGGTKLEIK | 34 |
| 119G5 VH | QVQLQQSGPEVVKPGASVKISCKASGYTFTDYYMNWVKQSRGKSLE WIGDINPNNGGTNYNQKFKGKATLTVDKSSSTAYMELRSLTSEDSAV YYCAKEGPPGAMDYWGQGTSVTVSS | 35 |
| 119G5 VL | NIVMTQTPKFLLVSAGDRVTITCKASQSVSNHVAWYQQKPGQSPKLLI YYVSNRSIGVPDRFTGSGYGTDFTFTISTVQAEDLAVYFCQQVYSSPFT FGGGTRLEMK | 36 |
| 85B10 VH | QVQLQQSGPELVKPGASVKISCKASGYTFTAYYVNWVKQSHGKSLEW IGDINPNNGGTNYNQKFKGKATLTVDMSSSTAYMELRSLTSEDSAVY YCAKEGPPGAMDYWGQGTSVTVSS | 37 |
| 85B10 VL | SIVLTQTPKFLLVSAGDRVPITCKASQSVSNNVAWYQQKPGQSPKLLIY YASNPYTGVPDRFTGSGYGTDFTFTITTVQAEDLAVYFCQQVYSSPFT FGGGTKLEMK | 38 |
| 95F10 VH | QVQLQQSGPEVVNPGASVKISCKASGYTFTDYYMNWVKQSRGKSLE WIGDINPNNGGTNYNQKFKDKATLTVDKSSNTAYMELRSLTSENSAV YYCAKEGPPGAMDYWGQGTSVTVSS | 39 |
| 95F10 VL | NIVMTQTPKFLLVSAGDRVTITCKASQSVSNHVAWYQQKPGQSPKLLI YYVSNRYIGVPDRFTGSGYGTDFTFTISTVQAEDLAVYFCQQVYSSPF TFGGGTRLEMK | 40 |

**Table 1-1. CDR sequences of 14G12**

| **14G12** | **Sequence** | **SEQ ID NO:** |
|---|---|---|
| CDRH1 | SSWMN | 41 |
| CDRH2 | RIYREDGHTNYNGKFKG | 42 |
| | RIYREDAHTNYNGKFKG | 47 |
| | RIYREDGHTNYNAKFKG | 48 |
| | RIYREDGHTNYNQKFKG | 49 |
| | RIYREEGHTNYNQKFKG | 50 |
| | RIYRE EGHTNYN E KFKG | 51 |
| | RIYRESGHTNYNQKFKG | 52 |
| | RIYRESGHTNYNEKFKG | 53 |
| CDRH3 | GGAMDY | 43 |
| CDRL1 | RASQDISNYLN | 44 |
| CDRL2 | YTSRLHS | 45 |
| CDRL3 | QQDNTLPWT | 46 |
| | GNDNTLPWT | 264 |
| | N NTLPWT | 265 |

**Table 1-2. CDR sequences of 393E9**

| **393E9** | **Sequence** | **SEQ ID NO:** |
|---|---|---|
| CDRH1 | DYYMS | 54 |
| CDRH2 | FIRNKGNGYTTENSASVKG | 55 |
| | FIRNKGNAYTTEYSASVKG | 60 |
| | FIRNKGNTYTTEYSASVKG | 61 |
| | FIRNKGN AYTTENAASVK G | 62 |
| | | 63 |
| | FIRNKGNTYTTENAASVKG | 266 |
| | FIRNKGNAYTTENSASVKG | 267 |
| | FIRNKGNGYTTEYSASVKG | |
| CDRH3 | YRGNPHYAMDY | 56 |
| CDRL1 | RASESVEFYGTSFLQ | 57 |
| CDRL2 | GASNVES | 58 |
| CDRL3 | QQSRKVPST | 59 |

**Table 1-3. CDR sequences of 113H5**

| **113H5** | **Sequence** | **SEQ ID NO:** |
|---|---|---|
| CDRH1 | SGYYWN | 64 |
| CDRH2 | YINSDGSNNYNPSLKN | 65 |
| CDRH3 | EEYDYWFAY | 66 |
| CDRL1 | KASQNVGTNVA | 67 |
| CDRL2 | SASNRYS | 68 |
| CDRL3 | QQYNSYPYT | 69 |

**Table 1-4. CDR sequences of 159D5**

| **159D5** | **Sequence** | **SEQ ID NO:** |
|---|---|---|
| CDRH1 | NFGMH | 70 |
| CDRH2 | YISSGSSTFYYADSVKG | 71 |
| | YISSGSSTFYYADAVKG | 76 |
| CDRH3 | SPAYYRYGLDY | 72 |
| CDRL1 | RASQSVSSSTFSYMH | 73 |
| CDRL2 | SSSNLES | 74 |
| CDRL3 | QHSWEIPYT | 75 |

**Table 1-5. CDR sequences of 24F10**

| **24F10** | **Sequence** | **SEQ ID NO:** |
|---|---|---|
| CDRH1 | DYGMH | 77 |
| CDRH2 | YISSGTSTIYYTDTVKG | 78 |
| CDRH3 | GGDGYYRSTMDY | 79 |
| CDRL1 | RASQSVSSSSYSYMH | 80 |
| CDRL2 | SASNLAS | 81 |
| CDRL3 | QHSWEVPRT | 82 |

**Table 1-6. CDR sequences of 493E10**

| **493E10** | **Sequence** | **SEQ ID NO:** |
|---|---|---|
| CDRH1 | SFGMH | 83 |
| CDRH2 | YISSGSSTIYYADTVKG | 84 |
| CDRH3 | NRAYHREAMDY | 85 |
| CDRL1 | RASQSVSSSSYNYMH | 86 |
| CDRL2 | SASNLES | 87 |
| CDRL3 | QHSWEIPYT | 88 |

**Table 1-7. CDR sequences of 257F1**

| **257F1** | **Sequence** | **SEQ ID NO:** |
|---|---|---|
| CDRH1 | SYGLN | 89 |
| CDRH2 | EIYPRSENTHYNEKFKG | 90 |
| CDRH3 | GDWDFDH | 91 |
| CDRL1 | SASSSVSYMN | 92 |
| CDRL2 | GISNLAS | 93 |
| CDRL3 | QQRSSYPRT | 94 |

**Table 1-8. CDR sequences of 353H11**

| **353H11** | **Sequence** | **SEQ ID NO:** |
|---|---|---|
| CDRH1 | SYWMH | 95 |
| CDRH2 | NINPSNGNTNYNEKFKN | 96 |
| | NINPSNANTNYNEKFKN | 101 |
| | NINPSNQNTNYNEKFKN | 102 |
| | NINPSNENTNYNEKFKN | 103 |
| | NINPSQGNTNYNEKFKN | 104 |
| CDRH3 | GGWDFDY | 97 |
| CDRL1 | SASSSVRYMH | 98 |
| CDRL2 | DTSKLTS | 99 |
| CDRL3 | QLWTSKPPWT | 100 |

**Table 1-9. CDR sequences of 367B8**

| **367B8** | **Sequence** | **SEQ ID NO:** |
|---|---|---|
| CDRH1 | SYWMH | 105 |
| CDRH2 | NINPSNGNTNYNENFKS | 106 |
| CDRH3 | GGWDFDY | 107 |
| CDRL1 | SASSSVRYIH | 108 |
| CDRL2 | DTSKLTS | 109 |
| CDRL3 | QQWTSKPPWT | 110 |

**Table 1-10. CDR sequences of 389G2**

| **389G2** | **Sequence** | **SEQ ID NO:** |
|---|---|---|
| CDRH1 | SYWMH | 111 |
| CDRH2 | NINPSNGNTNYNERFKS | 112 |
| CDRH3 | GGWDFDY | 113 |
| CDRL1 | SASSSVRYIH | 114 |
| CDRL2 | DTSKLTS | 115 |
| CDRL3 | QQWTSKPPWT | 116 |

**Table 1-11. CDR sequences of 109H7**

| **109H7** | **Sequence** | **SEQ ID NO:** |
|---|---|---|
| CDRH1 | GYWIE | 117 |
| CDRH2 | ETLPGRGSTNYNEKFKG | 118 |
| CDRH3 | GRRDFDY | 119 |
| CDRL1 | SASQGINNYLN | 120 |
| CDRL2 | YTSSLHS | 121 |
| CDRL3 | QQFSKLPFT | 122 |

**Table 1-12. CDR sequences of 286B4**

| **286B4** | **Sequence** | **SEQ ID NO:** |
|---|---|---|
| CDRH1 | NNYYWN | 123 |
| CDRH2 | YITYDGSNNYNPSLKN | 124 |
| | YITYDASNNYNPSLKN | 129 |
| CDRH3 | GGGQLRFDY | 125 |
| CDRL1 | KASQSVSNEVT | 126 |
| CDRL2 | YASNRYT | 127 |
| CDRL3 | QQDYSSPWT | 128 |

**Table 1-13. CDR sequences of 37G6**

| **37G6** | **Sequence** | **SEQ ID NO:** |
|---|---|---|
| CDRH1 | SYWIT | 130 |
| CDRH2 | DIYPGSGTINYNEKFKS | 131 |
| CDRH3 | SDGNYYFDY | 132 |
| CDRL1 | RASSSVNYMH | 133 |
| CDRL2 | ATSNLAS | 134 |
| CDRL3 | QQWSSNPPT | 135 |

**Table 1-14. CDR sequences of 267B5**

| **267B5** | **Sequence** | **SEQ ID NO:** |
|---|---|---|
| CDRH1 | SSYYWN | 136 |
| CDRH2 | YISYDGSNNYNPSLKN | 137 |
| CDRH3 | SWDGGYAMDN | 138 |
| CDRL1 | KASQSVSNDVA | 139 |
| CDRL2 | FASNRYT | 140 |
| CDRL3 | QQDYTSPWT | 141 |

**Table 1-15. CDR sequences of 425G1**

| **425G1** | **Sequence** | **SEQ ID NO:** |
|---|---|---|
| CDRH1 | DTYMH | 142 |
| CDRH2 | RIDPANGHTIFASKFQG | 143 |
| CDRH3 | FTMVVVPWYFDV | 144 |
| CDRL1 | KASQDVSTAVA | 145 |
| CDRL2 | WASTRHT | 146 |
| CDRL3 | QQHYSSPLT | 147 |

**Table 1-16. CDR sequences of 449H9**

| **449H9** | **Sequence** | **SEQ ID NO:** |
|---|---|---|
| CDRH1 | NYWMN | 148 |
| CDRH2 | QIYPGDGDTNYNGKFKN | 149 |
| CDRH3 | HYDYPYYYAMDY | 150 |
| CDRL1 | RASQDIGIALT | 151 |
| CDRL2 | ATSSLDS | 152 |
| CDRL3 | LQYIISPYT | 153 |

**Table 1-17. CDR sequences of 49C5**

| **49C5** | **Sequence** | **SEQ ID NO:** |
|---|---|---|
| CDRH1 | DYYMN | 154 |
| CDRH2 | DINPYSGGATNNQKFKG | 155 |
| CDRH3 | GSIYYDSTANYFD Y | 156 |
| CDRL1 | RASENIYSYLA | 157 |
| CDRL2 | NAKSLTE | 158 |
| CDRL3 | QHHYVTPWT | 159 |

**Table 1-18. CDR sequences of 119G5**

| **119G5** | **Sequence** | **SEQ ID NO:** |
|---|---|---|
| CDRH1 | DYYMN | 160 |
| CDRH2 | DINPNNGGTNYNQKFKG | 161 |
| | DINPNQGGTNYNQKFKG | 166 |
| CDRH3 | EGPPGAMDY | 162 |
| CDRL1 | KASQSVSNHVA | 163 |
| CDRL2 | YVSNRSI | 164 |
| CDRL3 | QQVYSSPFT | 165 |

**Table 1-19. CDR sequences of 85B10**

| **85B10** | **Sequence** | **SEQ ID NO:** |
|---|---|---|
| CDRH1 | AYYVN | 167 |
| CDRH2 | DINPNNGGTNYNQKFKG | 168 |
| | DINPNQGGTNYNQKFKG | 173 |
| CDRH3 | EGPPGAMDY | 169 |
| CDRL1 | KASQSVSNNVA | 170 |
| CDRL2 | YASNPYT | 171 |
| CDRL3 | QQVYSSPFT | 172 |

**Table 1-20. CDR sequences of 95F10**

| **95F10** | **Sequence** | **SEQ ID NO:** |
|---|---|---|
| CDRH1 | DYYMN | 174 |
| CDRH2 | DINPNNGGTNYNQKFKD | 175 |
| | DINPNQGGTNYNQKFKD | 180 |
| CDRH3 | EGPPGAMDY | 176 |
| CDRL1 | KASQSVSNHVA | 177 |
| CDRL2 | YVSNRYI | 178 |
| CDRL3 | QQVYSSPFT | 179 |

### Example 2: Binding affinity of mAbs

The binding of the chimeric mAbs from these clones against recombinant 5T4 protein (human 5T4-his tag) was tested with Biacore using a capture method. The mAbs were captured using Protein A chip. A serial dilution of human 5T4-his tag protein was injected over captured antibody for 2-3 mins at a flow rate of 30 µl/min. The antigen was allowed to dissociate for 360-1000s. All the experiments were carried out on a Biacore T200. Data analysis was carried out using Biacore T200 evaluation software.

As shown in the results of **Table 2** below, most of the tested antibodies exhibited nanomolar or sub-nanomolar binding affinity to the recombinant human 5T4 protein.

**Table 2. Affinity measured by Biacore**

| **Abs** | **5T4-His** | | |
|---|---|---|---|
| | **ka (1/Ms)** | **kd (1/s)** | **KD (M)** |
| **14G12** | 5.38 x 10⁵ | 2.22 x 10⁻⁴ | 4.13 x 10⁻¹⁰ |
| **393E9** | 3.24 x 10⁶ | 1.83 x 10⁻⁴ | 5.65 x 10⁻¹¹ |
| **113H5** | 3.48 x 10⁵ | 1.92 x 10⁻⁴ | 5.52 x 10⁻¹⁰ |
| **159D5** | 1.16 x 10⁶ | 2.40 x 10⁻⁴ | 2.06 x 10⁻¹⁰ |
| **24F10** | 4.18 x 10⁵ | 1.26 x 10⁻⁴ | 3.02 x 10⁻¹⁰ |
| **493E10** | 6.42 x 10⁵ | 4.02 x 10⁻⁴ | 6.26 x 10⁻¹⁰ |
| **257F1** | 1.86 x 10⁵ | 2.75 x 10⁻⁴ | 1.48 x 10⁻⁹ |
| **353H11** | 4.01 x 10⁵ | 9.63 x 10⁻⁵ | 2.40 x 10⁻¹⁰ |
| **367B8** | 5.88 x 10⁵ | 1.67 x 10⁻⁴ | 2.85 x 10⁻¹⁰ |
| **389G2** | 2.82 x 10⁵ | 1.38 x 10⁻⁴ | 4.90 x 10⁻¹⁰ |
| **109H7** | 6.45 x 10⁵ | 3.91 x 10⁻⁴ | 6.06 x 10⁻¹¹ |
| **286B4** | 7.28 x 10⁴ | 6.99 x 10⁻⁵ | 9.60 x 10⁻¹⁰ |
| **37G6** | 8.23 x 10⁵ | 4.86 x 10⁻⁴ | 5.90 x 10⁻¹⁰ |
| **267B5** | 1.58 x 10⁵ | 2.55 x 10⁻⁴ | 1.62 x 10⁻⁹ |
| **425G1** | 1.31 x 10⁵ | 2.15 x 10⁻⁴ | 1.64 x 10⁻⁹ |
| **449H9** | 3.01 x 10⁵ | 1.15 x 10⁻⁴ | 3.81 x 10⁻¹⁰ |
| **49C5** | 5.44 x 10⁵ | 3.37 x 10⁻⁴ | 6.20 x 10⁻¹⁰ |
| **119G5** | 1.30 x 10⁵ | 1.59 x 10⁻⁴ | 1.22 x 10⁻⁹ |
| **85B10** | 1.58 x 10⁵ | 1.25 x 10⁻⁴ | 7.92 x 10⁻¹⁰ |
| **95F10** | 3.55 x 10⁵ | 1.20 x 10⁻⁴ | 3.37 x 10⁻¹⁰ |

ELISA testing was carried out to evaluate the binding of chimeric antibodies to human and cynomolgus, respectively. The antibody portion of naptumomab (NeoTX) that targets 5T4 (abbreviated as naptumomab in the present disclosure), was generated and used as a benchmark.

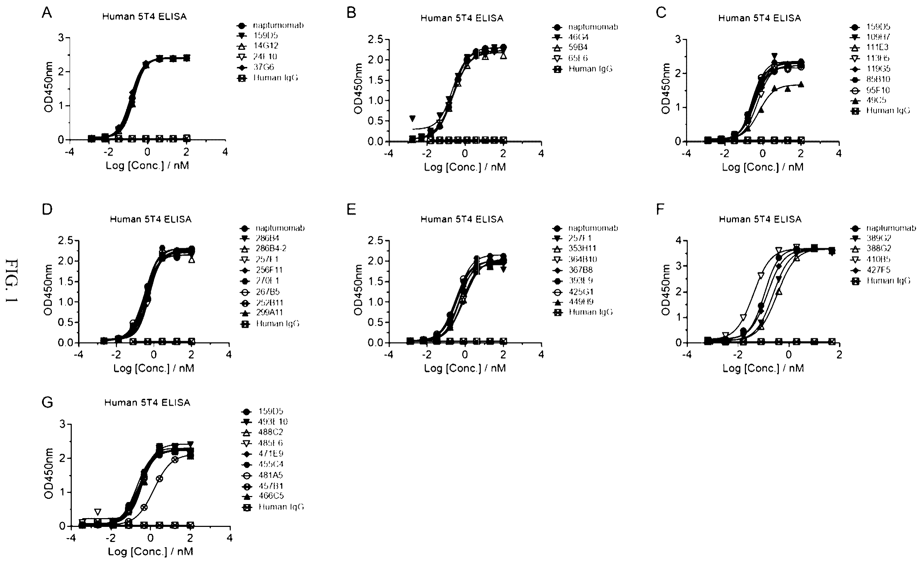

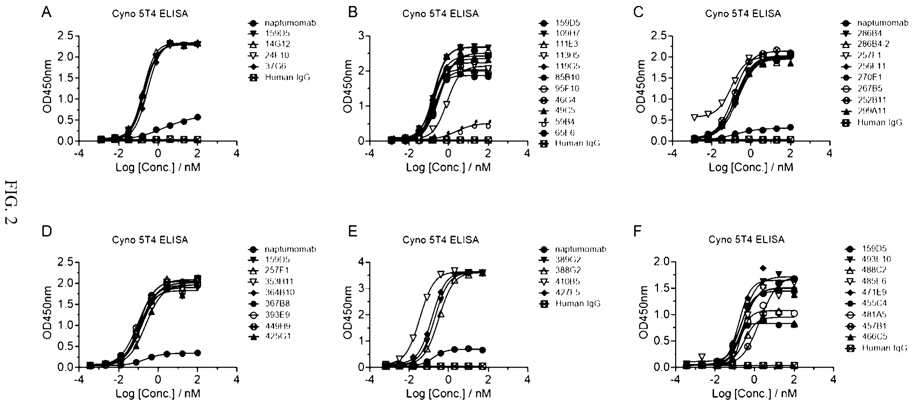

Briefly, microtiter plates were coated with human and cynomolgus 5T4 proteins at 1 µg/ml in PBS, 100 µl/well at 4°C overnight, then blocked with 150 µl/well of 1% BSA. Serial dilutions of chimeric antibodies were added to each well and incubated for 1 hour at RT. The plates were washed with PBS/Tween and then incubate with mouse-anti-human IgG Fc antibody conjugated with Horse Radish Peroxidase (HRP) for 30 mins at RT. After washing, the plates were developed with TMB substrate and analyzed by spectrophotometer at OD 450nm. All the 5T4 chimeric mAbs bound to human and cynomolgus 5T4 **(****FIG. 1-2** and **Table 3).** Naptumomab showed very weak cross-activity towards cynomolgus 5T4.

### Example 3: The binding activity to 5T4 antigen

### 3.1 Cross species activity

**Table 3. Cross species activity of the clones evaluated by ELISA**

| **Clone** / **EC₅₀ (nM)** | **Human 5T4** | **Cynomolgus 5T4** |
|---|---|---|
| **naptumomab** | **0.15** | **2.02** |
| **159D5** | 0.20 | 0.19 |
| **14G12** | 0.19 | 0.17 |
| **24F10** | 0.18 | 0.21 |
| **37G6** | 0.13 | 0.28 |
| **159D5** | **0.39** | **0.20** |
| **109H7** | 0.31 | 0.19 |
| **113H5** | 0.52 | 0.82 |
| **119G5** | 0.32 | 0.20 |
| **85B10** | 0.34 | 0.17 |
| **95F10** | 0.24 | 0.17 |
| **49C5** | 0.60 | 0.18 |
| **Naptumomab** | **0.48** | **0.16** |
| **286B4** | 0.35 | 0.22 |
| **257F1** | 0.56 | - |
| **267B5** | 0.31 | 0.17 |
| **Naptumomab** | **0.52** | - |
| **159D5** | - | **0.11** |
| **257F1** | - | 0.11 |
| **353H11** | 0.73 | 0.13 |
| **367B8** | 0.86 | 0.13 |
| **393E9** | 0.37 | 0.14 |
| **425G1** | 0.29 | 0.23 |
| **449H9** | 0.37 | 0.10 |
| **Naptumomab** | **0.11** | **0.17** |
| **389G2** | 0.23 | 0.19 |
| **159D5** | **0.24** | **0.18** |
| **493E10** | 0.28 | 0.25 |

### 3.2 Epitope binning by competitive ELSA

Competitive ELISA was carried out to classify the test 5T4 mAbs based on the binding epitope to human 5T4.

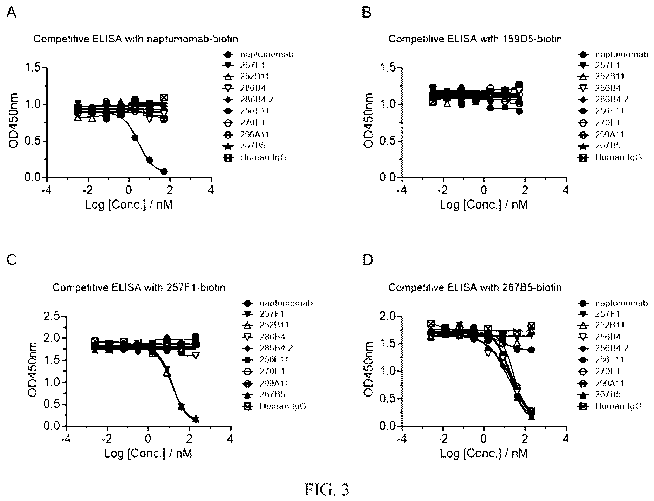

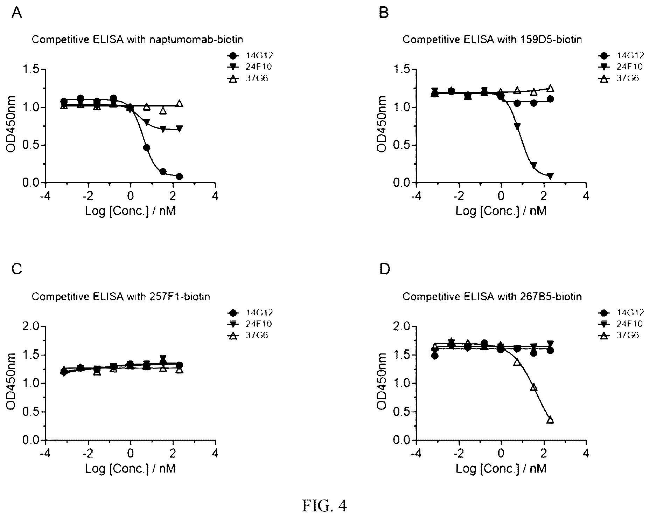

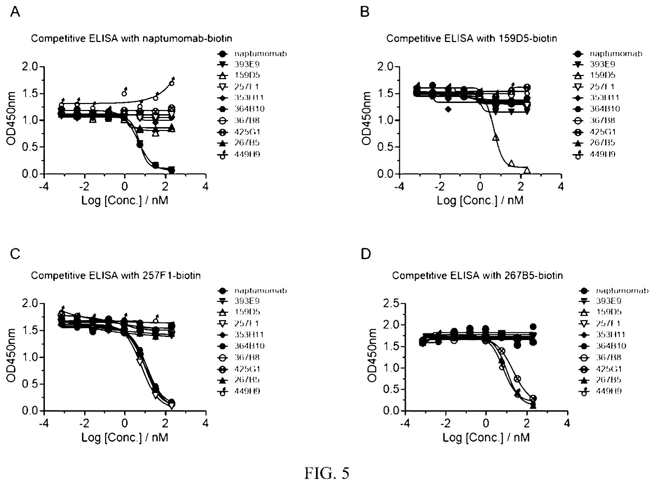

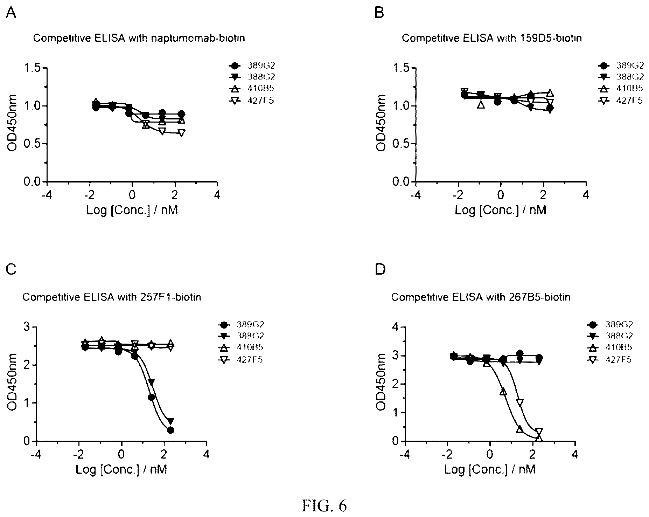

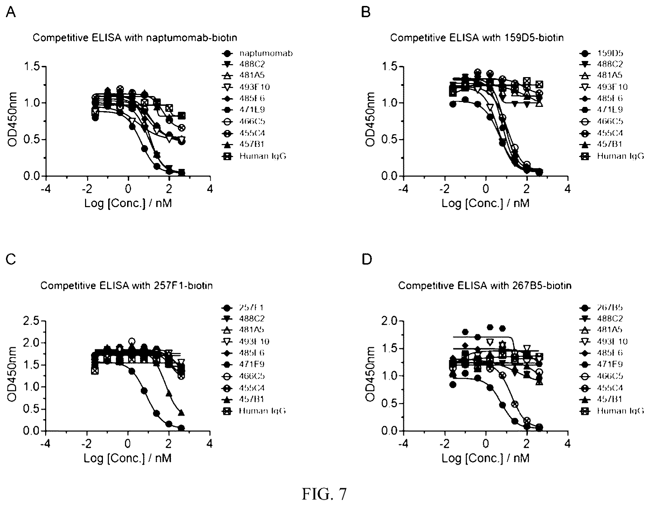

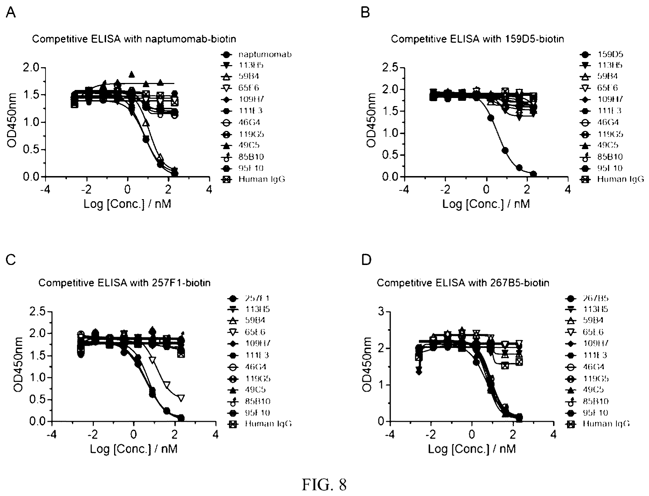

Briefly, microtiter plates were coated with human 5T4 proteins at 0.5 µg/ml in PBS, 100 µl/well at 4°C overnight, then blocked with 150 µl/well of 1% BSA. Serial dilutions of chimeric antibodies as well as 0.2 ug/ml biotin-conjugated reference mAb were added to each well and incubated for 1 hour at RT. The plates were washed with PBS/Tween and then incubate with streptavidin-HRP for 15 mins at RT. After washing, the plates were developed with TMB substrate and analyzed by spectrophotometer at OD 450nm. According to the competition performance with reference mAbs, the 5T4 mAbs were divided into four bins (Bin A-D), as shown in **FIG. 3-8** and **Table 4.**

**Table 4. Epitope Categories**

| **Category** | **Example clones** |
|---|---|
| **Bin A** | naptumomab, 14G12, 393E9, 113H5 |
| **Bin B** | 159D5, 24F10, 493E10 |
| **Bin C** | 257F1, 353H11, 367B8, 389G2, 109H7 |
| **Bin D** | 286B4, 37G6, 267B5, 425G1, 449H9, 49C5, 119G5, 85B10, 95F10 |

### 3.3 FACS Testing

Cell-based binding: FACS was used to evaluate the binding activity of all the tested chimeric mAbs on CHO-K1-expressing human 5T4 (CHOK1-hu5T4).

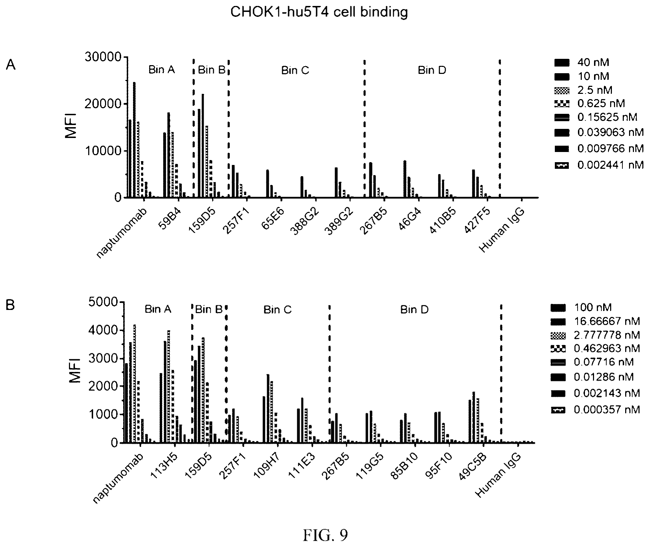

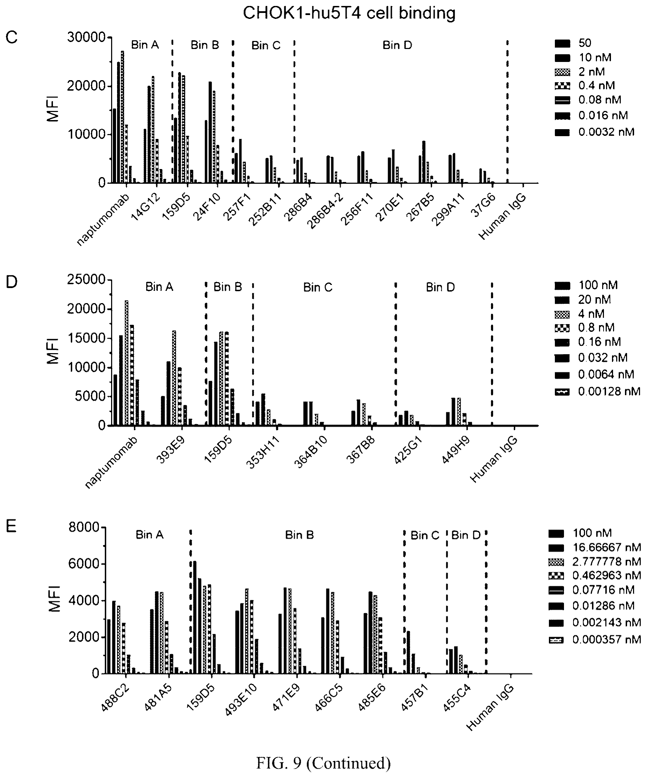

Briefly, CHOK1-hu5T4 cells were washed by FACS buffer and divided to each well with serially diluted 5T4 chimeric mAbs at 4°C for 30 mins. After washing by FACS buffer, PE Goat anti-Human IgG Fc Secondary Antibody (eBioscience^{™}, Invitrogen) was added to each well and incubated at 4°C for 30 mins. Samples were washed twice with FACS buffer. The mean florescence intensity (MFI) of PE was evaluated by MACSQuant Analyzer 16. As shown in **FIG. 9****,** all the tested 5T4 chimeric mAbs bound to CHOK1-hu5T4 cells, and antibodies in different bins showed different binding activity.

### Example 4. Humanization of 14G12

The 14G12 variable region genes were employed to create humanized mAbs. In the first step of this process, the amino acid sequences of the VH and VK of 14G12 were compared against the available database of human Ig gene sequences to find the overall best-matching human germline Ig gene sequences.

The sequences of the human germlines used for CDR grafting, as well as the resulting humanized sequences are listed in **Table 5.**

**Table 5-1. Humanization of 14G12 - 1^{st} run**

| **Name** | **Sequence** | **SEQ ID NO:** |
|---|---|---|
| 14G12 VH | EVQLQQSGPELVKPGASVKISCKASGYAFSSSWMNWVKQRPGKGLE WIGRIYREDGHTNYNGKFKGKATLTADKSSTTAYMQLTSLTSEDSAV YFCANGGAMDYWGQGTSVTVSS | 1 |
| 14G12 VH.V1 (grafted) | EVQLVQSGAEVKKPGATVKISCKVSGYAFSSSWMNWVQQAPGKGLE WMGRIYREDGHTNYNGKFKGRVTITADTSTDTAYMELSSLRSEDTA VYYCATGGAMDYWGQGTLVTVSS | 181 |
| 14G12 VH.V2 | EVQLVQSGAEVKKPGATVKISCKVSGYAFSSSWMNWVQQAPGKGLE WIGRIYREDGHTNYNGKFKGRVTITADTSTDTAYMELSSLRSEDTAV YFCANGGAMDYWGQGTLVTVSS | 182 |
| 14G12 VH.V3 | EVQLVQSGAEVKKPGATVKISCKVSGYAFSSSWMNWVKQAPGKGLE WIGRIYREDGHTNYNGKFKGRATITADTSTTAYMELSSLRSEDTAV YFCANGGAMDYWGQGTLVTVSS | 183 |
| 14G12 VH.V4 | EVQLVQSGAEVKKPGATVKISCKASGYAFSSSWMNWVKQAPGKGLE WIGRIYREDGHTNYNGKFKGRATITADTSTTAYMELSSLRSEDSAV YFCANGGAMDYWGQGTLVTVSS | 184 |
| 14G12 VL | DIQMTQSTSSLSASLGDRVTISCRASQDISNYLNWYQQKPDGTVKLLI YYTSRLHSGVPSRFSGSGSGTDYSLTISNLEQEDIATYFCQQDNTLPWT FGGGTKLEIK | 2 |
| 14G12 VL.V1 (grafted) | DIQMTQSPSSLSASVGDRVTITCRASQDISNYLNWYQQKPGKAPKLLI YYTSRLHSGVPSRFSGSGSGTDFTFTISSLQPEDIATYYCQQDNTLPWT FGGGTKLEIK | 185 |
| 14G12 VL.V2 | DIQMTQSPSSLSASVGDRVTITCRASQDISNYLNWYQQKPGKAPKLLI YYTSRLHSGVPSRFSGSGSGTDYTFTISSLQPEDIATYFCQQDNTLPWT FGGGTKLEIK | 186 |
| 14G12 VL.V3 | DIQMTQSPSSLSASVGDRVTISCRASQDISNYLNWYQQKPGKAVKLLI YYTSRLHSGVPSRFSGSGSGTDFTFTISSLQPEDIATYFCQQDNTLPWT FGGGTKLEIK | 187 |
| 14G12 VL.V4 | DIQMTQSPSSLSASVGDRVTISCRASQDISNYLNWYQQKPGKTVKLLI YYTSRLHSGVPSRFSGSGSGTDYTFTISSLQPEDIATYFCQQDNTLPWT FGGGTKLEIK | 188 |

**Table 5-2. Humanized antibodies from 14G12 - 1^{st} run**

| **VL VH** | **14G12-VL.V1** | **14G12-VL.V2** | **14G12-VL.V3** | **14G12-VL.V4** | **14G12-VL** |
|---|---|---|---|---|---|
| **14G12-VH.V1** | Hu14G12-1 | Hu14G12-2 | Hu14G12-3 | Hu14G12-4 | |
| **14G12-VH.V2** | Hu14G12-5 | Hu14G12-6 | Hu14G12-7 | Hu14G12-8 | |
| **14G12-VH.V3** | Hu14G12-9 | Hu14G12-10 | Hu14G12-11 | Hu14G12-12 | |
| **14G12-VH.V4** | Hu14G12-13 | Hu14G12-14 | Hu14G12-15 | Hu14G12-16 | |
| **14G12-VH** | | | | | 14G12-C |

**Table 5-3. Humanization of 14G12 - 2^{nd} run**

| **Name** | **Sequence** | **SEQ ID NO:** |
|---|---|---|
| 14G12 VH | EVQLQQSGPELVKPGASVKISCKASGYAFSSSWMNWVKQRPGKGLE WIGRIYREDGHTNYNGKFKGKATLTADKSSTTAYMQLTSLTSEDSAV YFCANGGAMDYWGQGTSVTVSS | 1 |
| 14G12 VH0 (grafted) | QVQLVQSGAEVKKPGSSVKVSCKASGGTFSSSWMNWVRQAPGQGLE WMGRIYREDGHTNYNGKFKGRATITADKSTSTAYMELSSLRSEDTAV YYCARGGAMDYWGQGTTVTVSS | 189 |
| 14G12 VH1 | QVQLVQSGAEVKKPGSSVKVSCKASGGTFSSSWMNWVKQAPGQGLE WIGRIYREDGHTNYNGKFKGKATLTADKSTSTAYMELSSLRSEDTAV YFCANGGAMDYWGQGTTVTVSS | 190 |
| 14G12 VH2 | QVQLVQSGAEVKKPGSSVKVSCKASGGTFSSSWMNWVKQAPGQGLE WIGRIYREDGHTNYNGKFKGRATLTADKSTSTAYMELSSLRSEDTAV YFCANGGAMDYWGQGTTVTVSS | 191 |
| 14G12 VH3 | QVQLVQSGAEVKKPGSSVKVSCKASGGTFSSSWMNWVKQAPGQGLE WIGRIYREDGHTNYNGKFKGRATITADKSTSTAYMELSSLRSEDTAVY FCANGGAMDYWGQGTTVTVSS | 192 |
| 14G12 VL | DIQMTQSTSSLSASLGDRVTISCRASQDISNYLNWYQQKPDGTVKLLIY YTSRLHSGVPSRFSGSGSGTDYSLTISNLEQEDIATYFCQQDNTLPWTFG GGTKLEIK | 2 |
| 14G12 VL1 (grafted) | DIQMTQSPSSLSASVGDRVTITCRASQDISNYLNWYQQKPGKVPKLLIY YTSRLHSGVPSRFSGSGSGTDFTLTISSLQPEDVATYYCQQDNTLPWTF GGGTKLEIK | 193 |
| 14G12 VL2 | DIQMTQSPSSLSASVGDRVTITCRASQDISNYLNWYQQKPGKVPKLLIY YTSRLHSGVPSRFSGSGSGTDYTLTISSLQPEDVATYFCQQDNTLPWTF GGGTKLEIK | 194 |

**Table 5-4. Humanized antibodies from 14G12 - 2^{nd} run**

| **VL VH** | **14G12-VL1** | **14G12-VL2** | **14G12-VL** |
|---|---|---|---|
| **14G12-VH1** | Hu14G12-17 | Hu14G12-18 | |
| **14G12-VH2** | Hu14G12-19 | Hu14G12-20 | |
| **14G12-VH3** | Hu14G12-21 | Hu14G12-22 | |
| **14G12-VH** | | | 14G12-C |

**Table 5-5. Humanization of 14G12 - 3^{rd} run**

| **Name** | **Sequence** | **SEQ ID NO:** |
|---|---|---|
| 14G12 VH | EVQLQQSGPELVKPGASVKISCKASGYAFSSSWMNWVKQRPGKGL EWIGRIYREDGHTNYNGKFKGKATLTADKSSTTAYMQLTSLTSEDS AVYFCANGGAMDYWGQGTSVTVSS | 1 |
| 14G12 hVH1 | QVQLVQSGAEVKKPGSSVKVSCKASGYAFSSSWMNWVRQAPGQG LEWMGRIYREDGHTNYNGKFKGRVTITADKSTTTAYMELSSLRSED TAVYYCANGGAMDYWGQGTLVTVSS | 195 |
| hVH1 grafted | QVQLVQSGAEVKKPGSSVKVSCKASGGTFSSSWMNWVRQAPGQG LEWMGRIYREDGHTNYNGKFKGRVTITADKSTSTAYMELSSLRSED TAVYYCANGGAMDYWGQGTLVTVSS | 196 |
| 14G12 hVH2 | QVQLVQSGAEVKKPGASVKVSCKASGYAFSSSWMNWVRQAPGQG LEWMGRIYREDGHTNYNGKFKGRVTMTADKSITTAYMELSRLRSD DTAVYYCANGGAMDYWGQGTLVTVSS | 197 |
| hVH2 grafted | QVQLVQSGAEVKKPGASVKVSCKASGYTFSSSWMNWVRQAPGQG LEWMGRIYREDGHTNYNGKFKGRVTMTADKSITTAYMELSRLRSD DTAVYYCANGGAMDYWGQGTLVTVSS | 198 |
| 14G12 hVH3 | QVQLVQSGAEVKKPGASVKVSCKASGYAFSSSWMNWVRQAPGQG LEWMGRIYREDGHTNYNGKFKGRVTMTADKSTTTAYMELSSLRSE DTAVYYCANGGAMDYWGQGTLVTVSS | 199 |
| hVH3 grafted | QVQLVQSGAEVKKPGASVKVSCKASGYTFSSSWMNWVRQAPGQG LEWMGRIYREDGHTNYNGKFKGRVTMTADKSTTTGYMELSSLRSE DTAVYYCANGGAMDYWGQGTLVTVSS | 200 |
| 14G12 hVH4 | QVQLVQSGAEVKKPGASVKVSCKASGYAFSSSWMNWVRQAPGQR LEWMGRIYREDGHTNYNGKFKGRVTITADKSATTAYMELSSLRSED TAVYYCANGGAMDYWGQGTLVTVSS | 201 |
| hVH4 grafted | QVQLVQSGAEVKKPGASVKVSCKASGYTFSSSWMNWVRQAPGQR LEWMGRIYREDGHTNYNGKFKGRVTITADKSATTAYMELSSLRSED TAVYYCANGGAMDYWGQGTLVTVSS | 202 |
| 14G12 hVH5 | QVQLVQSGAEVKKPGASVKVSCKASGYAFSSSWMNWVRQAPGQG LEWMGRIYREDGHTNYNGKFKGRVTMTADKSTTTAYMELRSLRSD DTAVYYCANGGAMDYWGQGTLVTVSS | 203 |
| hVH5 grafted | QVQLVQSGAEVKKPGASVKVSCKASGYTFSSSWMNWVRQAPGQG LEWMGRIYREDGHTNYNGKFKGRVTMTADKSTSTAYMELRSLRSD DTAVYYCANGGAMDYWGQGTLVTVSS | 204 |
| 14G12 VL | DIQMTQSTSSLSASLGDRVTISCRASQDISNYLNWYQQKPDGTVKL LIYYTSRLHSGVPSRFSGSGSGTDYSLTISNLEQEDIATYFCQQDNTL PWTFGGGTKLEIK | 2 |
| 14G12 hVL1 | DIQMTQSPSSLSASVGDRVTITCRASQDISNYLNWYQQKPGKAPKL LIYYTSRLHSGVPSRFSGSGSGTDYTFTISSLQPEDIATYFCQQDNTL PWTFGGGTKVEIK | 205 |
| **hVL1** grafted | DIQMTQSPSSLSASVGDRVTITCRASQDISNYLNWYQQKPGKAPKL LIYYTSRLHSGVPSRFSGSGSGTDYTFTISSLQPEDIATYYCQQDNTL PWTFGGGTKVEIK | 206 |
| 14G12 hVL2 | DIQMTQSPSSLSASVGDRVTITCRASQDISNYLNWYQQKPGKAPKL LIYYTSRLHSGVPSRFSGSGSGTDYTLTISSLQPEDIATYFCQQDNTL PWTFGGGTKVEIK | 207 |
| **hVL2** grafted | DIQMTQSPSSLSASVGDRVTITCRASQDISNYLNWYQQKPGKAPKL LIYYTSRLHSGVPSRFSGSGSGTDYTLTISSLQPEDFATYFCQQDNTL PWTFGGGTKVEIK | 208 |
| 14G12 hVL3 | EIVMTQSPATLSLSPGERATLSCRASQDISNYLNWYQQKPGQAPRLL IYYTSRLHSGIPARFSGSGSGTDYTLTISSLEPEDIAVYFCQQDNTLP WTFGGGTKVEIK | 209 |
| hVL3 grafted | EIVMTQSPATLSLSPGERATLSCRASQDISNYLNWYQQKPGQAPRLL IYYTSRLHSGIPARFSGSGSGTDYTLTISSLEPEDFAVYYCQQDNTLP WTFGGGTKVEIK | 210 |
| 14G12 hVL4 | DIVMTQSPDSLAVSLGERATINCRASQDISNYLNWYQQKPGQPPKL LIYYTSRLHSGVPDRFSGSGSGTDYTLTISSLQAEDIAVYFCQQDNTL PWTFGGGTKVEIK | 211 |
| hVL4 grafted | DIVMTQSPDSLAVSLGERATINCRASQDISNYLNWYQQKPGQPPKL LIYYTSRLHSGVPDRFSGSGSGTDYTLTISSLQAEDIAVYYCQQDNT LPWTFGGGTKVEIK | 212 |
| 14G12 hVL5 | DIQMTQSPSTLSASVGDRVTITCRASQDISNYLNWYQQKPGKAPKL LIYYTSRLHSGVPSRFSGSGSGTEYTLTISSLQPDDIATYFCQQDNTL PWTFGGGTKVEIK | 213 |
| hVL5 grafted | DIQMTQSPSTLSASVGDRVTITCRASQDISNYLNWYQQKPGKAPKL LIYYTSRLHSGVPSRFSGSGSGTEYTLTISSLQPDDFATYFCQQDNTL PWTFGGGTKVEIK | 214 |

**Table 5-6. Humanized antibodies from 14G12 - 3^{rd} run**

| **VL VH** | **14G12-hVL1** | **14G12-hVL2** | **14G12-hVL3** | **14G12-hVL4** | **14G12-hVL5** | **14G12-VL** |
|---|---|---|---|---|---|---|
| **14G12-hVH1** | Hu14G12-23 | Hu14G12-24 | Hu14G12-25 | Hu14G12-26 | Hu14G12-27 | |
| **14G12-hVH2** | Hu14G12-28 | Hu14G12-29 | Hu14G12-30 | Hu14G12-31 | Hu14G12-32 | |
| **14G12-hVH3** | Hu14G12-33 | Hu14G12-34 | Hu14G12-35 | Hu14G12-36 | Hu14G12-37 | |
| **14G12-hVH4** | Hu14G12-38 | Hu14G12-39 | Hu14G12-40 | Hu14G12-41 | Hu14G12-42 | |
| **14G12-hVHS** | Hu14G12-43 | Hu14G12-44 | Hu14G12-45 | Hu14G12-46 | Hu14G12-47 | |
| **14G12-VH** | | | | | | 14G12-C |

### Example 5: Binding activity of 14G12 humanized antibodies to human 5T4 antigen

This example tested the binding activities of the 14G12 humanized antibodies to the human 5T4 protein.

### 5.1 ELISA binding of 14G12 humanized antibodies to 5T4

To evaluate the binding activity of 14G12 humanized antibodies to human 5T4, the 14G12 chimeric antibody along with 14G12 humanized mAbs were subjected to ELISA test.

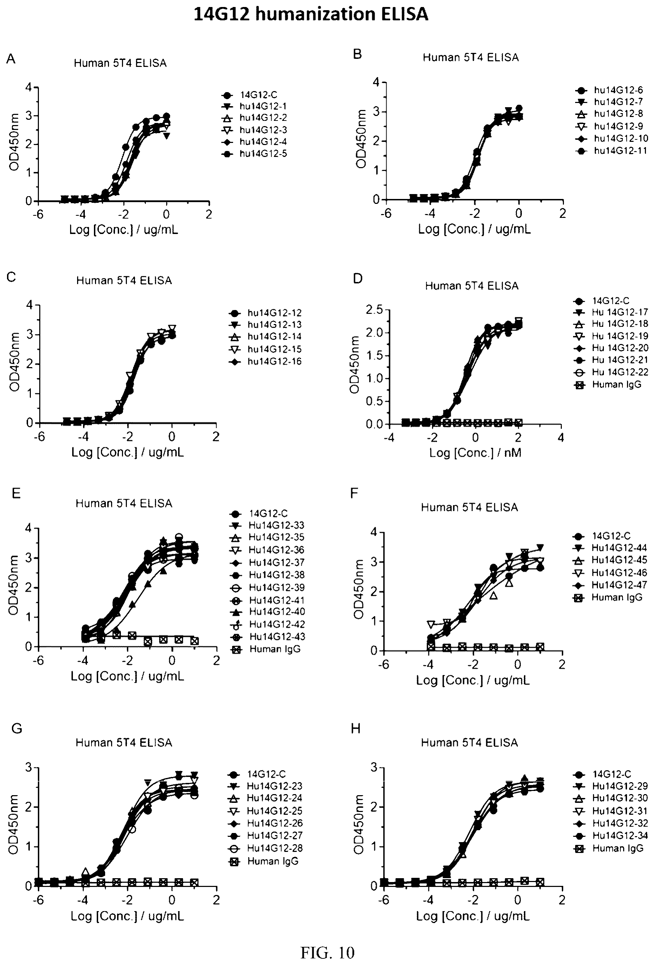

Briefly, microtiter plates were coated with human 5T4-His protein at 1 µg/ml in PBS, 100µl/well at 4°C overnight, then blocked with 150µl/well of 1% BSA. Serial dilutions of antibodies were added to each well and incubated for 1 hour at 37°C. The plates were washed with PBS/Tween and then incubated with Goat Anti-Human IgG-HRP for 30 mins at 37°C. After washing, the plates were developed with TMB substrate and analyzed by spectrophotometer at OD 450nm. As shown in **FIG. 10** and **Table 6,** most of the humanized antibodies bound to human 5T4 at high activity.

**Table 6. Binding activity of humanized 14G12 antibodies to human 5T4**

| **Abs** | **EC50 (µg/ml)** |
|---|---|
| 14G12-C | 0.0084 |
| Hu14G12-1 | 0.021 |
| Hu14G12-2 | 0.021 |
| Hu14G12-3 | 0.026 |
| Hu14G12-4 | 0.019 |
| Hu14G12-5 | 0.014 |
| Hu14G12-6 | 0.010 |
| Hu14G12-7 | 0.015 |
| Hu14G12-8 | 0.015 |
| Hu14G12-9 | 0.012 |
| Hu14G12-10 | 0.015 |
| Hu14G12-11 | 0.015 |
| Hu14G12-12 | 0.019 |
| Hu14G12-13 | 0.018 |
| Hu14G12-14 | 0.014 |
| Hu14G12-15 | 0.014 |
| Hu14G12-16 | 0.017 |
| 14G12-C | 0.40 |
| Hu14G12-17 | 0.52 |
| Hu14G12-18 | 0.31 |
| Hu14G12-19 | 0.40 |
| Hu14G12-20 | 0.32 |
| Hu14G12-21 | 0.40 |
| Hu14G12-22 | 0.45 |
| 14G12-C | 0.0076 |
| Hu14G12-33 | 0.011 |
| Hu14G12-35 | 0.013 |
| Hu14G12-36 | 0.0068 |
| Hu14G12-37 | 0.011 |
| Hu14G12-38 | 0.0064 |
| Hu14G12-39 | 0.0069 |
| Hu14G12-40 | 0.028 |
| Hu14G12-41 | 0.0047 |
| Hu14G12-42 | 0.0078 |
| Hu14G12-43 | 0.0064 |
| 14G12-C | 0.0082 |
| Hu14G12-44 | 0.0075 |
| Hu14G12-45 | 0.0082 |
| Hu14G12-46 | 0.023 |
| Hu14G12-47 | 0.014 |
| 14G12-C | 0.0057 |
| Hu14G12-23 | 0.0071 |
| Hu14G12-24 | 0.0049 |
| Hu14G12-25 | 0.0060 |
| Hu14G12-26 | 0.0069 |
| Hu14G12-27 | 0.0078 |
| Hu14G12-28 | 0.010 |
| 14G12-C | 0.010 |
| Hu14G12-29 | 0.0067 |
| Hu14G12-30 | 0.010 |
| Hu14G12-31 | 0.0084 |
| Hu14G12-32 | 0.0093 |
| Hu14G12-34 | 0.0087 |

### 5.2 Cell-based binding of 14G12 humanized antibodies to 5T4

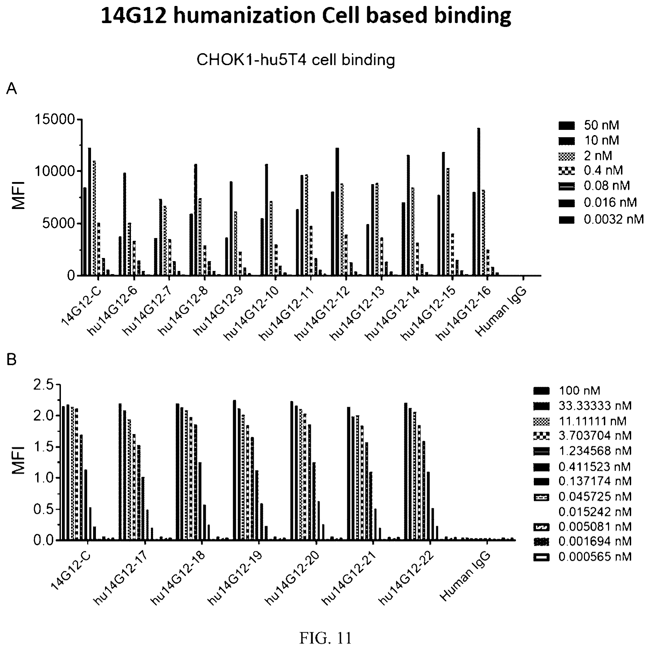

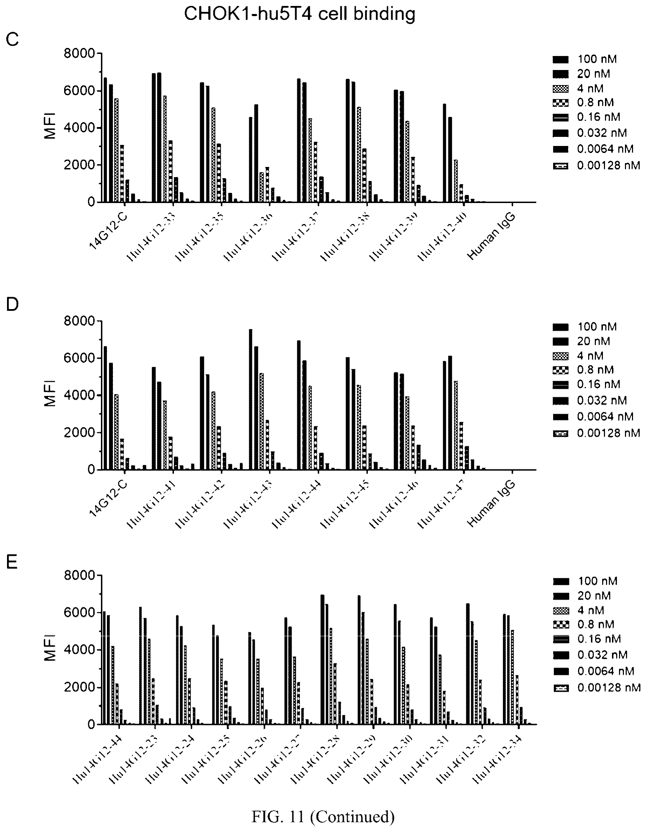

To evaluate the cell-based binding property of 14G12 humanized antibodies to human 5T4, the tested antibodies were analyzed by FACS in CHOKl-hu5T4 cells. A total number of 1X10⁵ cHOK1-hu5T4 cells in each well were incubated with serial diluted antibodies for 30 minutes at 4°C in FACS buffer. After wash by FACS buffer, PE conjugated-anti-human IgG antibody was added to each well and incubated at 4°C for 30 minutes. After wash, MFI of PE was evaluated by MACSQuant Analyzer 16. As shown in **FIG. 11****,** some of the listed 14G12 humanized antibodies showed comparable binding abilities to 14G12 chimeric antibody.

### 5.3 Protein affinity ranking of 14G12 humanized antibodies for 5T4

The binding of the 14G12 humanized antibodies to recombinant 5T4 protein (human 5T4-his tag) was tested with Biacore using a capture method. The mAbs were captured using Protein A chip. Serial dilution of human 5T4-his tag protein was injected over captured antibody for 120s-180s at a flow rate of 30 µl/min. The antigen was allowed to dissociate for 360s-1000s. All the experiments were carried out on a Biacore T200. Data analysis was carried out using the Biacore T200 evaluation software. The results are shown in **Table 7** below.

**Table 7. Affinity ranking measured by Biacore**

| **Abs** | **Human 5T4-His** | | |
|---|---|---|---|
| | **ka (1/Ms)** | **kd (1/s)** | **KD (M)** |
| 14G12-C | 4.01 x 10⁵ | 3.36 x 10⁻⁴ | 8.39 x 10⁻¹⁰ |
| Hu14G12-1 | 2.83 x 10⁵ | 1.21 x 10⁻² | 4.27 x 10⁻⁸ |
| Hu14G12-2 | 2.03 x 10⁵ | 4.36 x 10⁻³ | 2.15 x 10⁻⁸ |
| Hu14G12-3 | 2.00 x 10⁵ | 5.50 x 10⁻³ | 2.75 x 10⁻⁸ |
| Hu14G12-4 | 2.14 x 10⁵ | 2.67 x 10⁻³ | 1.25 x 10⁻⁸ |
| Hu14G12-5 | 2.48 x 10⁵ | 1.46 x 10⁻³ | 5.88 x 10⁻⁹ |
| Hu14G12-6 | 2.88 x 10⁵ | 7.70 x 10⁻⁴ | 2.68 x 10⁻⁹ |
| Hu14G12-7 | 3.36 x 10⁵ | 8.46 x 10⁻⁴ | 2.52 x 10⁻⁹ |
| Hu14G12-8 | 3.40 x 10⁵ | 4.64 x 10⁻⁴ | 1.36 x 10⁻⁹ |
| Hu14G12-9 | 2.02 x 10⁵ | 1.07 x 10⁻³ | 5.31 x 10⁻⁹ |
| Hu14G12-10 | 2.12 x 10⁵ | 5.12 x 10⁻⁴ | 2.42 x 10⁻⁹ |
| Hu14G12-11 | 2.41 x 10⁵ | 5.23 x 10⁻⁴ | 2.17 x 10⁻⁹ |
| Hu14G12-12 | 2.74 x 10⁵ | 6.55 x 10⁻⁴ | 2.39 x 10⁻⁹ |
| Hu14G12-13 | 1.98 x 10⁵ | 1.21 x 10⁻³ | 6.12 x 10⁻⁹ |
| Hu14G12-14 | 2.07 x 10⁵ | 7.01 x 10⁻⁴ | 3.39 x 10⁻⁹ |
| Hu14G12-15 | 2.62 x 10⁵ | 8.37 x 10⁻⁴ | 3.19 x 10⁻⁹ |
| Hu14G12-16 | 2.69 x 10⁵ | 4.99 x 10⁻⁴ | 1.85 x 10⁻⁹ |
| 14G12-C | 6.15 x 10⁵ | 3.30 x 10⁻⁴ | 5.37 x 10⁻¹⁰ |
| Hu14G12-18 | 3.92 x 10⁵ | 1.78 x 10⁻³ | 4.55 x 10⁻⁹ |
| Hu14G12-23 | 3.52 x 10⁵ | 9.08 x 10⁻⁴ | 2.58 x 10⁻⁹ |
| Hu14G12-28 | 2.95 x 10⁵ | 7.14 x 10⁻⁴ | 2.42 x 10⁻⁹ |
| Hu14G12-29 | 5.14 x 10⁵ | 1.10 x 10⁻³ | 2.14 x 10⁻⁹ |
| Hu14G12-32 | 3.55 x 10⁵ | 9.79 x 10⁻⁴ | 2.76 x 10⁻⁹ |
| Hu14G12-37 | 1.34 x 10⁶ | 1.42 x 10⁻³ | 1.05 x 10⁻⁹ |
| Hu14G12-43 | 1.89 x 10⁶ | 1.60 x 10⁻³ | 8.44 x 10⁻¹⁰ |
| Hu14G12-44 | 1.40 x 10⁶ | 1.97 x 10⁻³ | 1.41 x 10⁻⁹ |
| Hu14G12-47 | 4.38 x 10⁵ | 9.21 x 10⁻⁴ | 2.10 x 10⁻⁹ |

### Example 6. Humanization of the 393E9 Antibodies

The 393E9 variable region genes were employed to create humanized mAbs. In the first step of this process, the amino acid sequences of the VH and VK of 393E9 were compared against the available database of human Ig gene sequences to find the overall best-matching human germline Ig gene sequences.

The sequences of the human germlines used for CDR grafting, as well as the resulting humanized sequences are listed in **Table 8.**

**Table 8. Humanization of 393E9**

| **Name** | **Sequence** | **SEQ ID NO:** |
|---|---|---|
| 393E9_VH | EVKLVESGGGLVQPGGSLSLSCAASGFTFTDYYMSWVRQPPGKAL EWLGFIRNKGNGYTTENSASVKGRFTISRDNSQSILYLQMNALRAE DSATYYCARYRGNPHYAMDYWGQGTSVTVSS | 3 |
| 393E9 VH-G57A (CDR2 mutated) | EVKLVESGGGLVQPGGSLSLSCAASGFTFTDYYMSWVRQPPGKAL EWLGFIRNKGNAYTTENSASVKGRFTISRDNSQSILYLQMNALRAE DSATYYCARYRGNPHYAMDYWGQGTSVTVSS | 215 |
| 393E9 VH.V1 | EVQLVESGGGLVQPGGSLRLSCAASGFTFTDYYMSWVRQAPGKG LEWVGFIRNKGNGYTTENSASVKGRFTISRDNSKSTLYLQMNSLR AEDTAVYYCARYRGNPHYAMDYWGQGTLVTVSS | 216 |
| 393E9 VH.V2 | EVQLVESGGGLIQPGGSLRLSCAASGFTFTDYYMSWVRQPPGKGL EWVGFIRNKGNGYTTENSASVKGRFTISRDNSKSTLYLQMNSLRA EDTAVYYCARYRGNPHYAMDYWGQGTLVTVSS | 217 |
| 393E9 VH.V3 | QVQLVESGGGVVQPGGSLRLSCAASGFTFTDYYMSWVRQAPGKG LEWVGFIRNKGNGYTTENSASVKGRFTISRDNSKSTLYLQMNSLR AEDTAVYYCARYRGNPHYAMDYWGQGTLVTVSS | 218 |
| 393E9 VH.V4 | EVQLVESGGGLVQPGGSLRLSCAASGFTFTDYYMSWVRQAPGKG LEWVGFIRNKGNGYTTENSASVKGRFTISRDNSKSSLYLQMNSLK TEDTAVYYCARYRGNPHYAMDYWGQGTLVTVSS | 219 |
| 393E9 VH.V5 | EVQLVESGGGLVQPGRSLRLSCTASGFTFTDYYMSWVRQAPGKGL EWVGFIRNKGNGYTTENSASVKGRFTISRDNSKSILYLQMNSLKTE DTAVYYCARYRGNPHYAMDYWGQGTLVTVSS | 220 |
| 393E9 VH.V6 | EVQLVESGGGLVQPGGSLRLSCAASGFTFTDYYMSWVRQAPGKG LEWVGFIRNKGNGYTTENSASVKGRFTISRDNAKSSLYLQMNSLR AEDTAVYYCARYRGNPHYAMDYWGQGTLVTVSS | 221 |
| 393E9 VL | DIVLTQFPASLAVSLGQRATISCRASESVEFYGTSFLQWYQQKPGQP PKLLIYGASNVESGVPARFSGSGSGTDFSLSIHPVEEDDIAMYFCQ QSRKVPSTFGGGTKLEIK | 4 |
| 393E9 VL.V1 | DIVLTQSPDSLAVSLGERATINCRASESVEFYGTSFLQWYQQKPGQ PPKLLIYGASNVESGVPDRFSGSGSGTDFTLTISSLQAEDIAVYFCQ QSRKVPSTFGGGTKVEIK | 222 |
| 393E9 VL.V2 | EIVLTQSPATLSVSPGERATLSCRASESVEFYGTSFLQWYQQKPGQ APRLLIYGASNVESGIPARFSGSGSGTEFTLTISSLQSEDIAVYFCQQ SRKVPSTFGGGTKVEIK | 223 |
| 393E9 VL.V3 | DIQLTQSPSSLSASVGDRVTITCRASESVEFYGTSFLQWYQQKPGK APKLLIYGASNVESGVPSRFSGSGSGTDFTLTISSLQPEDIATYFCQ QSRKVPSTFGGGTKVEIK | 224 |
| 393E9 VL.V4 | DIVLTQSPLSLPVTPGEPASISCRASESVEFYGTSFLQWYLQKPGQS PQLLIYGASNVESGVPDRFSGSGSGTDFTLKISRVEAEDIGVYFCQ QSRKVPSTFGGGTKVEIK | 225 |
| 393E9 VL.V5 | EIVLTQSPGTLSLSPGERATLSCRASESVEFYGTSFLQWYQQKPGQ APRLLIYGASNVESGIPDRFSGSGSGTDFTLTISRLEPEDIAVYFCQQ SRKVPSTFGGGTKVEIK | 226 |

**Table 8-2. Humanized antibody from 393E9**

| | 393E9 VH.V1 | 393E9 VH.V2 | 393E9 VH.V3 | 393E9 VH.V4 | 393E9 VH.V5 | 393E9 VH.V6 |
|---|---|---|---|---|---|---|
| 393E9 VL.V1 | 393E9_hVH1 _hVL1 | 393E9_hVH 2_hVL1 | 393E9_hVH3 _hVL1 | 393E9_hVH 4_hVL1 | 393E9_hVH 5_hVL1 | 393E9_hVH6 _hVL1 |
| 393E9 VL.V2 | 393E9_hVH1 _hVL2 | 393E9_hVH 2_hVL2 | 393E9_hVH3 _hVL2 | 393E9_hVH 4_hVL2 | 393E9_hVH 5_hVL2 | 393E9_hVH6 _hVL2 |
| 393E9 VL.V3 | 393E9 hVH1 _hVL3 | 393E9_hVH 2_hVL3 | 393E9 hVH3 _hVL3 | 393E9_hVH 4_hVL3 | 393E9_hVH 5_hVL3 | 393E9_hVH6 _hVL3 |
| 393E9 VL.V4 | 393E9_hVH1 _hVL4 | 393E9_hVH 2_hVL4 | 393E9_hVH3 _hVL4 | 393E9_hVH 4_hVL4 | 393E9_hVH 5_hVL4 | 393E9_hVH6 _hVL4 |
| 393E9 VL.V5 | 393E9_hVH1 _hVL5 | 393E9_hVH 2_hVL5 | 393E9_hVH3 _hVL5 | 393E9_hVH 4_hVL5 | 393E9_hVH 5_hVL5 | 393E9_hVH6 _hVL5 |

### Example 7: Binding activity of 393E9 humanized antibodies to human 5T4 antigen

This example tested the binding activities of the 393E9 humanized antibodies to the human 5T4 protein.

### 7.1 ELISA binding of 393E9 humanized antibodies to 5T4

To evaluate the binding activity of 393E9 humanized antibodies to human 5T4, the 393E9 chimeric antibody (393E9-C) along with 393E9 humanized mAbs were subjected to ELISA test.

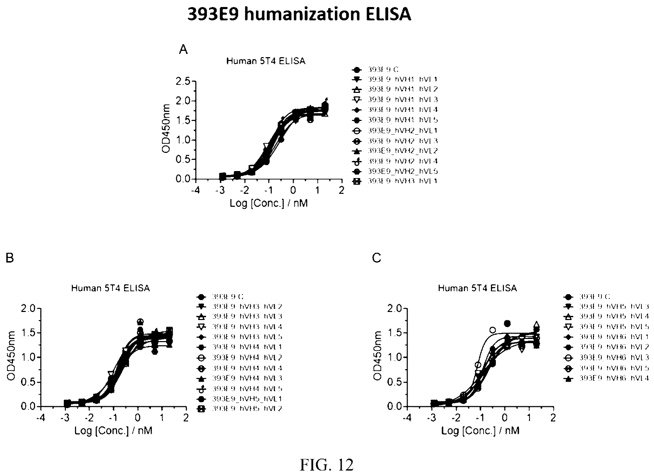

Briefly, microtiter plates were coated with human 5T4-His protein at 1 µg/ml in PBS, 100µl/well at 4°C overnight, then blocked with 150µl/well of 1% BSA. 4-fold dilutions of antibodies starting from 20 nM were added to each well and incubated for 1 hour at 37°C. The plates were washed with PBS/Tween and then incubated with Goat Anti-Human IgG-HRP for 30 mins at 37°C. After washing, the plates were developed with TMB substrate and analyzed by spectrophotometer at OD 450nm. As shown in **FIG. 12** and **Table 9,** most of the humanized antibodies bound to human 5T4 with high activity.

**Table 9. Binding activity of humanized 393E9 antibodies to human 5T4**

| **Abs** | **EC50 (nM)** |
|---|---|
| 393E9-C | 0.27 |
| 393E9_hVH1_hVL1 | 0.18 |
| 393E9_hVH1_hVL2 | 0.16 |
| 393E9_hVH1_hVL3 | 0.085 |
| 393E9_hVH1_hVL4 | 0.13 |
| 393E9_hVH1_hVL5 | 0.13 |
| 393E9_hVH2_hVL1 | 0.17 |
| 393E9_hVH2_hVL2 | 0.12 |
| 393E9_hVH2_hVL3 | 0.11 |
| 393E9_hVH2_hVL4 | 0.12 |
| 393E9_hVH2_hVL5 | 0.11 |
| 393E9_hVH3_hVL1 | 0.16 |
| 393E9-C | 0.13 |
| 393E9_hVH3_hVL2 | 0.20 |
| 393E9_hVH3_hVL3 | 0.14 |
| 393E9_hVH3_hVL4 | 0.095 |
| 393E9_hVH3_hVL5 | 0.12 |
| 393E9_hVH4_hVL1 | 0.13 |
| 393E9_hVH4_hVL2 | 0.12 |
| 393E9_hVH4_hVL3 | 0.13 |
| 393E9_hVH4_hVL4 | 0.16 |
| 393E9_hVH4_hVL5 | 0.22 |
| 393E9_hVH5_hVL1 | 0.16 |
| 393E9_hVH5_hVL2 | 0.27 |
| 393E9-C | 0.23 |
| 393E9_hVH5_hVL3 | 0.12 |
| 393E9_hVH5_hVL4 | 0.17 |
| 393E9_hVH5_hVL5 | 0.11 |
| 393E9_hVH6_hVL1 | 0.11 |
| 393E9_hVH6_hVL2 | 0.11 |
| 393E9_hVH6_hVL3 | 0.052 |
| 393E9_hVH6_hVL4 | 0.14 |
| 393E9_hVH6_hVL5 | 0.19 |

### 7.2 Cell-based binding of 393E9 humanized antibodies to 5T4

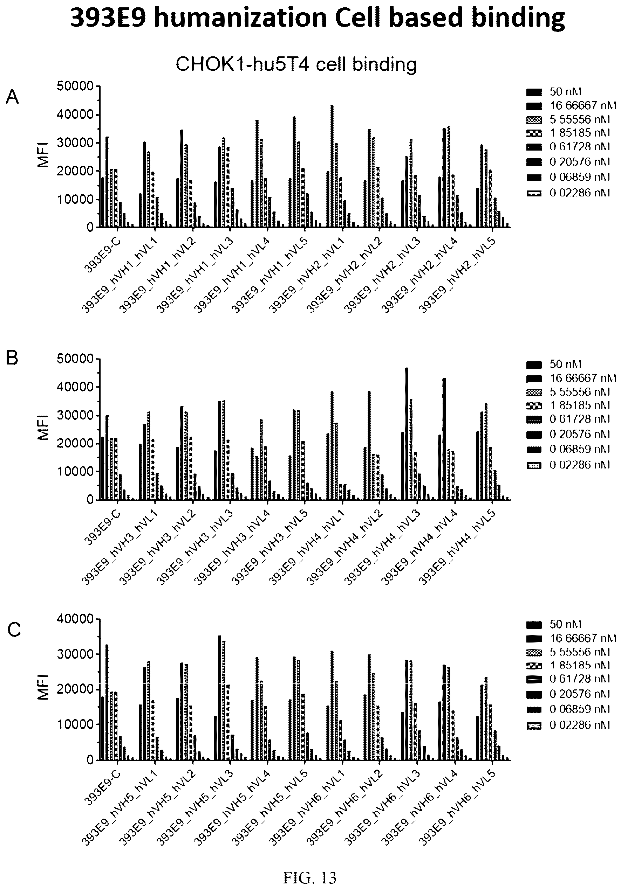

To evaluate the cell-based binding property of 393E9 humanized antibodies to human 5T4, the tested antibodies were analyzed by FACS in CHOK1-hu5T4 cells. A total number of 1X10⁵ CHOK1-hu5T4 cells in each well were incubated with 3-fold serial diluted antibodies starting from 50 nM for 30 minutes at 4°C in FACS buffer. After wash by FACS buffer, PE conjugated-anti-human IgG antibody was added to each well and incubated at 4°C for 30 minutes. After wash, MFI of PE was evaluated by MACSQuant Analyzer 16. As shown in **FIG. 13****,** some of the listed 393E9 humanized antibodies showed comparable binding abilities to 393E9 chimeric antibody.

### 7.3 Protein affinity ranking of 393E9 humanized antibodies for 5T4

The binding of the 393E9 humanized antibodies to recombinant 5T4 protein (human 5T4-his tag) was tested with Biacore using a capture method. The mAbs were captured using Protein A chip. A serial dilution of human 5T4-his tag protein was injected over captured antibody for 3 mins at a flow rate of 30 µl/min. The antigen was allowed to dissociate for 280-800s. All the experiments were carried out on a Biacore T200. Data analysis was carried out using the Biacore T200 evaluation software. The results are shown in **Table 10** below.

**Table 10. Affinity ranking measured by Biacore**

| **Abs** | **Human 5T4-His** | | |
|---|---|---|---|
| | **ka (1/Ms)** | **kd (1/s)** | **KD (M)** |
| 393E9-C | 2.31 x 10⁵ | 1.07 x 10⁻⁴ | 4.63 x 10⁻¹⁰ |
| 393E9_hVH1_hVL4 | 1.99 x 10⁵ | 1.19 x 10⁻⁴ | 5.99 x 10⁻¹⁰ |
| 393E9_hVH1_hVL5 | 2.17 x 10⁵ | 1.12 x 10⁻⁴ | 5.15 x 10⁻¹⁰ |
| 393E9_hVH2_hVL1 | 1.90 x 10⁵ | 1.04 x 10⁻⁴ | 5.47 x 10⁻¹⁰ |
| 393E9_hVH2_hVL2 | 2.01 x 10⁵ | 1.20 x 10⁻⁴ | 5.94 x 10⁻¹⁰ |
| 393E9_hVH2_hVL3 | 1.74 x 10⁵ | 1.20 x 10⁻⁴ | 6.84 x 10⁻¹⁰ |
| 393E9_hVH2_hVL4 | 1.95 x 10⁵ | 1.27 x 10⁴ | 6.50 x 10⁻¹⁰ |
| 393E9_hVH2_hVL5 | 2.17 x 10⁵ | 1.22 x 10⁻⁴ | 5.60 x 10⁻¹⁰ |
| 393E9_hVH3_hVL1 | 2.13 x 10⁵ | 1.08 x 10⁻⁴ | 5.06 x 10⁻¹⁰ |
| 393E9_hVH3_hVL3 | 1.93 x 10⁵ | 1.11 x 10⁻⁴ | 5.74 x 10⁻¹⁰ |
| 393E9_hVH3_hVL4 | 2.15 x 10⁵ | 1.23 x 10⁻⁴ | 5.73 x 10⁻¹⁰ |
| 393E9_hVH3_hVL5 | 2.38 x 10⁵ | 1.20 x 10⁻⁴ | 5.02 x 10⁻¹⁰ |
| 393E9_hVH4_hVL2 | 2.04 x 10⁵ | 1.23 x 10⁻⁴ | 6.03 x 10⁻¹⁰ |
| 393E9_hVH4_hVL3 | 1.81 x 10⁵ | 1.28 x 10⁻⁴ | 7.06 x 10⁻¹⁰ |
| 393E9_hVH4_hVL5 | 2.17 x 10⁵ | 1.29 x 10⁻⁴ | 5.95 x 10⁻¹⁰ |
| 393E9_hVH5_hVL3 | 1.70 x 10⁵ | 1.19 x 10⁻⁴ | 7.00 x 10⁻¹⁰ |
| 393E9_hVH5_hVL4 | 1.84 x 10⁵ | 1.25 x 10⁻⁴ | 6.79 x 10⁻¹⁰ |
| 393E9_hVH5_hVL5 | 2.02 x 10⁵ | 1.21 x 10⁻⁴ | 5.98 x 10⁻¹⁰ |
| 393E9_hVH6_hVL2 | 1.99 x 10⁵ | 1.37 x 10⁻⁴ | 6.87 x 10⁻¹⁰ |
| 393E9_hVH6_hVL3 | 1.79 x 10⁵ | 1.24 x 10⁻⁴ | 6.91 x 10⁻¹⁰ |
| 393E9_hVH6_hVL5 | 2.12 x 10⁵ | 1.22 x 10⁻⁴ | 5.74 x 10⁻¹⁰ |

### Example 8. Humanization of the 159D5 Antibodies

The 159D5 variable region genes were employed to create humanized mAbs. In the first step of this process, the amino acid sequences of the VH and VK of 159D5 were compared against the available database of human Ig gene sequences to find the overall best-matching human germline Ig gene sequences.

The sequences of the human germlines used for CDR grafting, as well as the resulting humanized sequences are listed in **Table 11.**

**Table 11-1. Humanization of 159D5**

| **Name** | **Sequence** | **SEQ ID NO:** |
|---|---|---|
| 159D5 VH | DVQLVESGGGLVQPGGSRKLSCTASGFTFSNFGMHWVRQAPETGL EWVAYISSGSSTFYYADSVKGRFTISRDNPKNTLFLQMTSLRSEDTA MYFCARSPAYYRYGLDYWGQGTTLTVSS | 7 |
| 159D5 mVH-S62A (CDR2 mutated) | DVQLVESGGGLVQPGGSRKLSCTASGFTFSNFGMHWVRQAPETGL EWVAYISSGSSTFYYADAVKGRFTISRDNPKNTLFLQMTSLRSEDTA MYFCARSPAYYRYGLDYWGQGTTLTVSS | 227 |
| 159D5 VH.V0 (grafted) | EVQLVESGGGVVQPGRSLRLSCAASGFTFSNFGMHWVRQAPGKGL EWVAYISSGSSTFYYADSVKGRFTISRDNSKNTLYLQMNSLRAEDTA VYYCARSPAYYRYGLDYWGQGTTVTVSS | 228 |
| 159D5 VH.V1 | EVQLVESGGGVVQPGRSLRLSCAASGFTFSNFGMHWVRQAPGKGL EWVAYISSGSSTFYYADSVKGRFTISRDNSKNTLYLQMNSLRAEDTA VYFCARSPAYYRYGLDYWGQGTTVTVSS | 229 |
| 159D5 VL | DIVLTQSPASLAVSLGQRATISCRASQSVSSSTFSYMHWYQQKPGQP PKLLIKSSSNLESGVPARFSGSGSGTDFTLNIHPVEEEDTATYYCQHS WEIPYTFGGGTKLEIK | 8 |
| 159D5 VL.V0 (grafted) | DIVMTQTPLSLSVTPGQPASISCRASQSVSSSTFSYMHWYQQKPGQP PQLLIYSSSNLESGVPDRFSGSGSGTDFTLKISRVEAEDVGVYYCQH SWEIPYTFGGGTKVEIK | 230 |
| 159D5 VL.V1 | DIVMTQTPLSLSVTPGQPASISCRASQSVSSSTFSYMHWYQQKPGQP PQLLIKSSSNLESGVPDRFSGSGSGTDFTLKISRVEAEDVGVYYCQH SWEIPYTFGGGTKVEIK | 231 |
| 159D5 VL.V2 | DIVLTQTPLSLSVTPGQPASISCRASQSVSSSTFSYMHWYQQKPGQP PQLLIKSSSNLESGVPDRFSGSGSGTDFTLKISRVEAEDVGVYYCQH SWEIPYTFGGGTKVEIK | 232 |
| 159D5 VL.V3 (grafted) | DIVMTQSPDSLAVSLGERATINCRASQSVSSSTFSYMHWYQQKPGQ PPKLLIYSSSNLESGVPDRFSGSGSGTDFTLTISSLQAEDVAVYYCQH SWEIPYTFGGGTKVEIK | 233 |
| 159D5 VL.V4 | DIVMTQSPDSLAVSLGERATINCRASQSVSSSTFSYMHWYQQKPGQ PPKLLIKSSSNLESGVPDRFSGSGSGTDFTLTISSLQAEDVAVYYCQH SWEIPYTFGGGTKVEIK | 234 |
| 159D5 VL.V5 | DIVLTQSPDSLAVSLGERATINCRASQSVSSSTFSYMHWYQQKPGQP PKLLIKSSSNLESGVPDRFSGSGSGTDFTLTISSLQAEDVAVYYCQHS WEIPYTFGGGTKVEIK | 235 |

**Table 11-2. Humanized antibodies from 159D5**

| **VL VH** | **159D5V L.V0** | **159D5V L.V1** | **159D5V L.V2** | **159D5V L.V3** | **159D5V L.V4** | **159D5V L.V5** | **159D5 VL** |
|---|---|---|---|---|---|---|---|
| **159D5-VH.V0** | Hu159D 5-1 | Hu159D 5-2 | Hu159D 5-3 | Hu159D 5-4 | Hu159D 5-5 | Hu159D 5-6 | |
| **159D5-VH.V1** | Hu159D 5-7 | Hu159D 5-8 | Hu159D 5-9 | Hu159D 5-10 | Hu159D 5-11 | Hu159D 5-12 | |
| **159D5-VH.S62A** | | | | | | | 159D5-P1 |
| **159D5-VH** | | | | | | | 159D5-C |

### Example 9: Binding activity of 159D5 humanized antibodies to human 5T4 antigen

This example tested the binding activities of the 159D5 humanized antibodies to the human 5T4 protein.

### 9.1 ELISA binding of 159D5 humanized antibodies to 5T4

To evaluate the binding activity of 159D5 humanized antibodies to human 5T4, the 159D5 chimeric antibody along with 159D5 humanized mAbs were subjected to ELISA test.

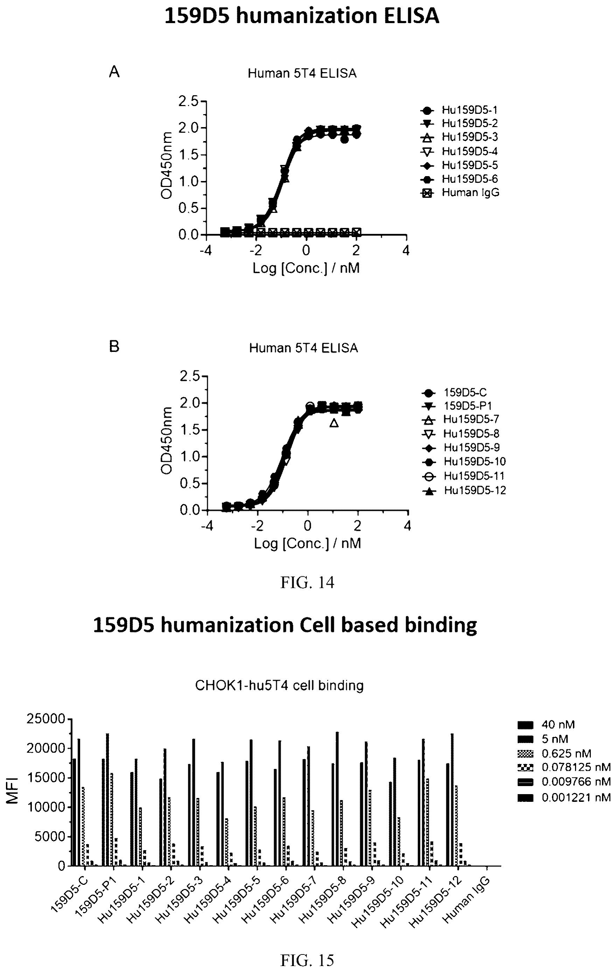

Briefly, microtiter plates were coated with human 5T4-His protein at 1 µg/ml in PBS, 100µl/well at 4°C overnight, then blocked with 150µl/well of 1% BSA. Serial dilutions of antibodies were added to each well and incubated for 1 hour at 37°C. The plates were washed with PBS/Tween and then incubated with Goat Anti-Human IgG-HRP for 30 mins at 37°C. After washing, the plates were developed with TMB substrate and analyzed by spectrophotometer at OD 450nm. As shown in **FIG. 14** and **Table 12,** most of the humanized antibodies bound to human 5T4 with high activity.

**Table 12. Binding activity of humanized 159D5 antibodies to human 5T4**

| **Abs** | **EC50 (nM)** |
|---|---|
| Hu159D5-1 | 0.088 |
| Hu159D5-2 | 0.12 |
| Hu159D5-3 | 0.12 |
| Hu159D5-4 | 0.094 |
| Hu159D5-5 | 0.099 |
| Hu159D5-6 | 0.10 |
| Hu159D5-7 | 0.097 |
| Hu159D5-8 | 0.15 |
| Hu159D5-9 | 0.14 |
| Hu159D5-10 | 0.094 |
| Hu159D5-11 | 0.12 |
| Hu159D5-12 | 0.12 |
| 159D5-P1 | 0.13 |
| 159D5-C | 0.13 |

### 9.2 Cell-based binding of 159D5 humanized antibodies to 5T4

To evaluate the cell-based binding property of 159D5 humanized antibodies to human 5T4, the tested antibodies were analyzed by FACS in CHOK1-hu5T4 cells. A total number of 1X10⁵ CHOK1-hu5T4 cells in each well were incubated with serial diluted antibodies for 30 minutes at 4°C in FACS buffer. After wash by FACS buffer, PE conjugated-anti-human IgG antibody was added to each well and incubated at 4°C for 30 minutes. After wash, MFI of PE was evaluated by MACSQuant Analyzer 16. As shown in **FIG.15****,** most of the listed 159D5 humanized antibodies showed comparable binding abilities to 159D5 chimeric antibody.

### 9.3 Protein affinity ranking of 159D5 humanized antibodies for 5T4

The binding of the 159D5 humanized antibodies to recombinant 5T4 protein (human 5T4-his tag) was tested with Biacore using a capture method. The mAbs were captured using Protein A chip. 50 nM of human 5T4-his tag protein was injected over captured antibody for 3 mins at a flow rate of 30 µl/min. The antigen was allowed to dissociate for 600s. All the experiments were carried out on a Biacore T200. Data analysis was carried out using the Biacore T200 evaluation software. The results are shown in **Table 13** below.

**Table 13. Affinity ranking measured by Biacore**

| **Abs** | **Human 5T4-His** | | |
|---|---|---|---|
| | **ka (1/Ms)** | **kd (1/s)** | **KD (M)** |
| 159D5-C | 3.30 x 10⁵ | 2.04 x 10⁻⁴ | 6.18 x 10⁻¹⁰ |
| Hu159D5-1 | 7.37 x 10⁴ | 2.94 x 10⁻⁴ | 3.99 x 10⁻⁹ |
| Hu159D5-2 | 2.87 x 10⁵ | 2.27 x 10⁻⁴ | 7.89 x 10⁻¹⁰ |
| Hu159D5-3 | 2.35 x 10⁵ | 2.40 x 10⁻⁴ | 1.02 x 10⁻⁹ |
| Hu159D5-4 | 6.02 x 10⁴ | 4.26 x 10⁻⁴ | 7.07 x 10⁻⁹ |
| Hu159D5-5 | 5.56 x 10⁴ | 5.61 x 10⁻⁴ | 1.01 x 10⁻⁸ |
| Hu159D5-6 | 2.52 x 10⁵ | 1.79 x 10⁻⁴ | 7.10 x 10⁻¹⁰ |
| Hu159D5-7 | 2.21 x 10⁵ | 2.53 x 10⁻⁴ | 1.14 x 10⁻⁹ |
| Hu159D5-8 | 8.40 x 10⁴ | 3.52 x 10⁻⁴ | 4.19 x 10⁻⁹ |
| Hu159D5-9 | 2.95 x 10⁵ | 2.21 x 10⁻⁴ | 7.47 x 10⁻¹⁰ |
| Hu159D5-10 | 5.24 x 10⁴ | 4.88 x 10⁻⁴ | 9.31 x 10⁻⁹ |
| Hu159D5-11 | 2.57 x 10⁵ | 2.73 x 10⁻⁴ | 1.06 x 10⁻⁹ |
| Hu159D5-12 | 2.22 x 10⁵ | 2.67 x 10⁻⁴ | 1.21 x 10⁻⁹ |
| 159D5-P1 | 3.34 x 10⁵ | 1.63 x 10⁻⁴ | 4.89 x 10⁻¹⁰ |

### Example 10. Humanization of the 286B4 Antibodies

The 286B4 variable region genes were employed to create humanized mAbs. In the first step of this process, the amino acid sequences of the VH and VK of 286B4 were compared against the available database of human Ig gene sequences to find the overall best-matching human germline Ig gene sequences.

For the heavy chain of 286B4, VH4-28/JH6 was chosen as the humanization backbone, and for the light chain of 286B4, VL-O18/JK4 is the best fit germline. Humanized 286B4 CDR grafting antibody was then designed where the CDRL1, L2, and L3 were grafted onto framework sequences of the VL-O18-JK4, and the CDRH1, H2, and H3 were grafted onto framework sequences of the VH4-28-JH6. A 3D model was then generated to determine the amino acids in the original mouse FR region sequences that are essential for antibody binding and conformation. Based on the 286B4 CDR grafting antibody sequence, 4 additional humanized heavy chains and 5 additional light chains were created.

The sequences of the human germlines used for CDR grafting, as well as the resulting humanized sequences are listed in **Table 14.**

**Table 14-1. Humanization of 286B4**

| **Name** | **Sequence** | **SEQ ID NO:** |
|---|---|---|
| 286B4 VH | DVQLHESGPGLVKPSQSLSLTCSVTGYSITNNYYWNWIRQFPGNNLEW MGYITYDGSNNYNPSLKNRISVTRDTSKNQFFLKLSSVTTEDTATYYCT RGGGQLRFDYWGQGTTLTVSS | 23 |
| 286B4 VH-G55A (CDR2 mutated) | DVQLHESGPGLVKPSQSLSLTCSVTGYSITNNYYWNWIRQFPGNNLEW MGYITYDASNNYNPSLKNRISVTRDTSKNQFFLKLSSVTTEDTATYYCT RGGGQLRFDYWGQGTTLTVSS | 236 |
| 286B4 VH.V0 (grafted) | EVQLQESGPGLVKPSDTLSLTCAVSGYSISNNYYWNWIRQPPGKGLEWI GYITYDGSNNYNPSLKNRVTMSVDTSKNQFSLKLSSVTAVDTAVYYCA RGGGQLRFDYWGQGTTVTVSS | 237 |
| 286B4 VH.V1 | EVQLQESGPGLVKPSDTLSLTCAVSGYSISNNYYWNWIRQPPGKGLEWI GYITYDGSNNYNPSLKNRVTMSRDTSKNQFSLKLSSVTAVDTAVYYCA RGGGQLRFDYWGQGTTVTVSS | 238 |
| 286B4 VH.V2 | EVQLQESGPGLVKPSDTLSLTCAVSGYSISNNYYWNWIRQPPGKGLEWI GYITYDGSNNYNPSLKNRITMSRDTSKNQFSLKLSSVTAVDTAVYYCA RGGGQLRFDYWGQGTTVTVSS | 239 |
| 286B4 VH.V3 | EVQLQESGPGLVKPSDTLSLTCAVSGYSISNNYYWNWIRQPPGKGLEW MGYITYDGSNNYNPSLKNRITVSRDTSKNQFSLKLSSVTAVDTAVYYC ARGGGQLRFDYWGQGTTVTVSS | 240 |
| 286B4 VH.V4 | EVQLQESGPGLVKPSDTLSLTCAVSGYSITNNYYWNWIRQPPGKGLEW MGYITYDGSNNYNPSLKNRITVSRDTSKNQFSLKLSSVTAVDTAVYYCT RGGGQLRFDYWGQGTTVTVSS | 241 |
| 286B4 VL | SIVMTQTPKFLIVSAGDRVTITCKASQSVSNEVTWYQQKPGQSPKMLIY YASNRYTGVPDRFTGSGYGTDFTFTINTVQAEDLAIYFCQQDYSSPWTF GGGTKLEIK | 24 |
| 286B4 VL.V0 (grafted) | DIQMTQSPSSLSASVGDRVTITCKASQSVSNEVTWYQQKPGKAPKLLI YYASNRYTGVPSRFSGSGSGTDFTFTISSLQPEDIATYYCQQDYSSPWTF GGGTKVEIK | 242 |
| 286B4 VL.V1 | DIQMTQSPSSLSASVGDRVTITCKASQSVSNEVTWYQQKPGKAPKLLI YYASNRYTGVPDRFSGSGSGTDFTFTISSLQPEDIATYYCQQDYSSPWT FGGGTKVEIK | 243 |
| 286B4 VL.V2 | DIQMTQSPSSLSASVGDRVTITCKASQSVSNEVTWYQQKPGKAPKMLI YYASNRYTGVPDRFSGSGSGTDFTFTISSLQPEDIATYYCQQDYSSPWT FGGGTKVEIK | 244 |
| 286B4 VL.V3 | DIQMTQSPSSLSASVGDRVTITCKASQSVSNEVTWYQQKPGKAPKMLI YYASNRYTGVPDRFSGSGYGTDFTFTISSLQPEDIATYYCQQDYSSPWT FGGGTKVEIK | 245 |
| 286B4 VL.V4 | DIQMTQSPSSLSASVGDRVTITCKASQSVSNEVTWYQQKPGKSPKMLI YYASNRYTGVPDRFSGSGYGTDFTFTISSLQPEDIATYFCQQDYSSPWT FGGGTKVEIK | 246 |
| 286B4 VL.V5 | SIVMTQSPSSLSASVGDRVTITCKASQSVSNEVTWYQQKPGKSPKMLIY YASNRYTGVPDRFSGSGYGTDFTFTISSLQPEDIATYFCQQDYSSPWTF GGGTKVEIK | 247 |

**Table 14-2. Humanized antibodies from 286B4**

| **VL VH** | **286B4VL.V1** | **286B4VL.V2** | **286B4VL.V3** | **286B4VL.V4** | **286B4VL.V5** | **286B4 VL** |
|---|---|---|---|---|---|---|
| **286B4-VH.V1** | Hu286B4-1 | Hu286B4-2 | Hu286B4-3 | Hu286B4-4 | Hu286B4-5 | |
| **286B4-VH.V2** | Hu286B4-6 | Hu286B4-7 | Hu286B4-8 | Hu286B4-9 | Hu286B4-10 | |
| **286B4-VH.V3** | Hu286B4-11 | Hu286B4-12 | Hu286B4-13 | Hu286B4-14 | Hu286B4-15 | |
| **286B4-VH.V4** | Hu286B4-16 | Hu286B4-17 | Hu286B4-18 | Hu286B4-19 | Hu286B4-20 | |
| **286B4-VH.G55A** | | | | | | 286B4-P1 |
| **286B4-VH** | | | | | | 286B4-C |

### Example 11: Binding activity of 286B4 humanized antibodies to human 5T4 antigen

This example tested the binding activities of the 286B4 humanized antibodies to the human 5T4 protein.

### 11.1 ELISA binding to 5T4

To evaluate the binding activity of clones, the 286B4 chimeric antibody along with 286B4 humanized mAbs were subjected to ELISA test.

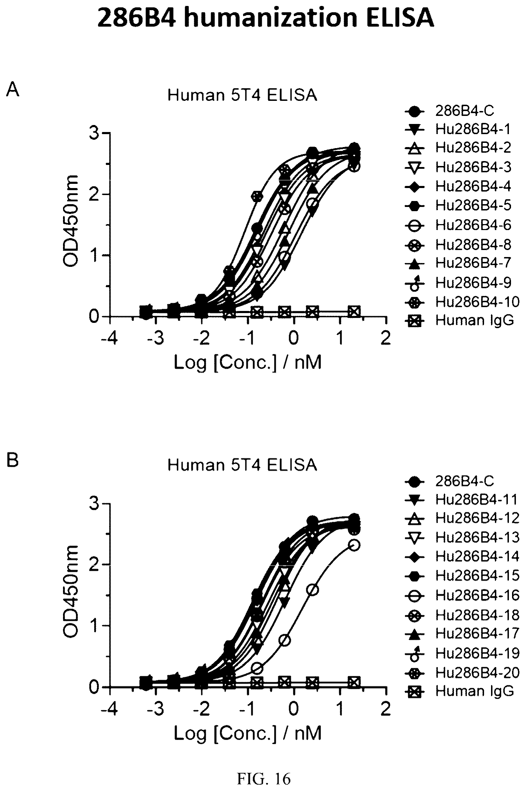

Briefly, microtiter plates were coated with human 5T4-His protein at 1 ug/ml in PBS, 100 µl/well at 4°C overnight, then blocked with 150 µl/well of 1% BSA.4-fold dilutions of tested antibodies starting from 20 nM were added to each well and incubated for 1 hour at 37°C. The plates were washed with PBS/Tween and then incubated with Goat Anti-Human IgG-HRP for 30 mins at 37°C. After washing, the plates were developed with TMB substrate and analyzed by spectrophotometer at OD 450nm. As shown in **FIG. 16** and **Table 15,** most of the humanized clones bound to human 5T4 with high potency.

**Table 15. Binding activity of humanized 286B4 humanized antibodies to human 5T4**

| **Abs** | **EC50 (nM)** |
|---|---|
| 284B4-C | 0.32 |
| Hu286B4-1 | 3.81 |
| Hu286B4-2 | 1.35 |
| Hu286B4-3 | 0.51 |
| Hu286B4-4 | 0.46 |
| Hu286B4-5 | 0.28 |
| Hu286B4-6 | 2.64 |
| Hu286B4-7 | 1.76 |
| Hu286B4-8 | 0.68 |
| Hu286B4-9 | 0.33 |
| Hu286B4-10 | 2.61 |
| 284B4-C | 0.32 |
| Hu286B4-11 | 1.42 |
| Hu286B4-12 | 0.89 |
| Hu286B4-13 | 0.57 |
| Hu286B4-14 | 0.30 |
| Hu286B4-15 | 0.25 |
| Hu286B4-16 | 4.03 |
| Hu286B4-17 | 0.71 |
| Hu286B4-18 | 0.42 |
| Hu286B4-19 | 0.27 |
| Hu286B4-20 | 0.42 |

### 11.2 Cell-based binding to 5T4

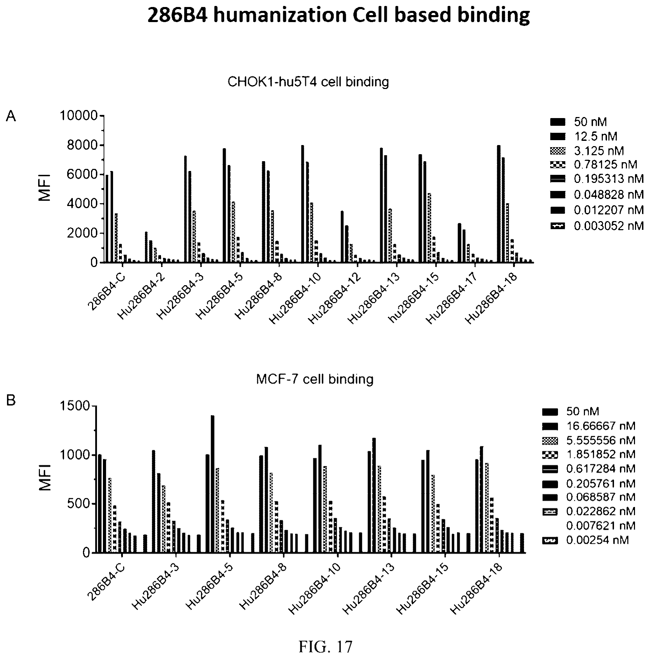

To evaluate the cell-based binding property of 286B4 humanized antibodies to human 5T4, the tested antibodies were analyzed for their binding to CHOK1-hu5T4 (human 5T4 high-expression cell line) or MCF-7 cells (human 5T4 low-expression cell line) by FACS. A total number of 1X10⁵ CHOK1-hu5T4 or MCF7 cells in each well were incubated with 4-fold or 3-fold serial diluted antibodies starting from 50 nM for 30 minutes at 4°C in FACS buffer. After wash by FACS buffer, PE conjugated-anti-human IgG antibody was added to each well and incubated at 4°C for 30 minutes. After wash, MFI of PE was evaluated by MACSQuant Analyzer 16. As shown in **FIG. 17A** **(CHO-hu5T4 cells) and** **Fig. 17B** **(MCF-7 cells),** the 286B4-3/8/13/18/5/10/15 humanized antibodies showed comparable binding abilities to 286B4 chimeric antibody.

### 11.3 Protein affinity ranking for human 5T4

The binding of the 286B4 humanized antibodies to recombinant 5T4 protein (human 5T4-his tag) was tested with Biacore using a capture method. The mAbs were captured using Protein A chip. A serial dilution of human 5T4-his tag protein was injected over captured antibody for 3 mins at a flow rate of 30 µl/min. The antigen was allowed to dissociate for 280-800s. All the experiments were carried out on a Biacore T200. Data analysis was carried out using the Biacore T200 evaluation software. The results are shown in **Table 16** below.

**Table 16. Affinity ranking measured by Biacore**

| **Abs** | **Human 5T4-His** | | |
|---|---|---|---|
| | **ka (1/Ms)** | **kd (1/s)** | **KD (M)** |
| 286-C | 9.09 x 10⁴ | 1.13 x 10⁻⁴ | 1.25 x 10⁻⁹ |
| Hu286B4-3 | 9.34 x 10⁴ | 1.07 x 10⁻⁴ | 1.14 x 10⁻⁹ |
| Hu286B4-5 | 1.06 x 10⁵ | 1.28 x 10⁻⁴ | 1.20 x 10⁻⁹ |
| Hu286B4-8 | 9.46 x 10⁴ | 1.03 x 10⁻⁴ | 1.08 x 10⁻⁹ |
| Hu286B4-10 | 1.06 x 10⁵ | 2.04 x 10⁻⁴ | 1.93 x 10⁻⁹ |
| Hu286B4-13 | 9.55 x 10⁴ | 1.07 x 10⁻⁴ | 1.12 x 10⁻⁹ |
| Hu286B4-15 | 1.10 x 10⁵ | 1.14 x 10⁻⁴ | 1.04 x 10⁻⁹ |
| Hu286B4-18 | 1.13 x 10⁵ | 1.33 x 10⁻⁴ | 1.18 x 10⁻⁹ |

### Example 12. PTM removal confirmation of 393E9, 159D5 and 286B4

### 12.1 PTM removal confirmation of 393E9

The 393E9 VH CDR2 includes NG and NS residues (Kabat numbering) which are at risk of post-translational modifications (PTM) and pose challenges for future manufacturing. Therefore, this example mutated NG to NA and NS to YS on the VH to prevent PTM. The sequences of the potential PTM removal site are listed in **Table 17.**

**Table 17-1: Humanization of 393E9 with removal of potential PTM site**

| **Name** | **Sequence** | **SEQ ID NO:** |
|---|---|---|
| 393E9 VH.V4 NG-NA | EVQLVESGGGLVQPGGSLRLSCAASGFTFTDYYMSWVRQAPGKGLE WVGFIRNKGNAYTTENSASVKGRFTISRDNSKSSLYLQMNSLKTEDT AVYYCARYRGNPHYAMDYWGQGTLVTVSS | 248 |
| 393E9 VH.V4 NS-YS | EVQLVESGGGLVQPGGSLRLSCAASGFTFTDYYMSWVRQAPGKGLE WVGFIRNKGNGYTTEYSASVKGRFTISRDNSKSSLYLQMNSLKTEDT AVYYCARYRGNPHYAMDYWGQGTLVTVSS | 249 |
| 393E9 VH.V4 NG-NA NS-YS | EVQLVESGGGLVQPGGSLRLSCAASGFTFTDYYMSWVRQAPGKGLE WVGFIRNKGNAYTTEYSASVKGRFTISRDNSKSSLYLQMNSLKTEDT AVYYCARYRGNPHYAMDYWGQGTLVTVSS | 250 |
| 393E9 VH.V5 NG-NA | EVQLVESGGGLVQPGRSLRLSCTASGFTFTDYYMSWVRQAPGKGLE WVGFIRNKGNAYTTENSASVKGRFTISRDNSKSILYLQMNSLKTEDTA VYYCARYRGNPHYAMDYWGQGTLVTVSS | 251 |
| 393E9 VH.V5 NS-YS | EVQLVESGGGLVQPGRSLRLSCTASGFTFTDYYMSWVRQAPGKGLE WVGFIRNKGNGYTTEYSASVKGRFTISRDNSKSILYLQMNSLKTEDT AVYYCARYRGNPHYAMDYWGQGTLVTVSS | 252 |
| 393E9 VH.V5 NG-NA NS-YS | EVQLVESGGGLVQPGRSLRLSCTASGFTFTDYYMSWVRQAPGKGLE WVGFIRNKGNAYTTEYSASVKGRFTISRDNSKSILYLQMNSLKTEDT AVYYCARYRGNPHYAMDYWGQGTLVTVSS | 253 |
| 393E9 VH.V6 NG-NA | EVQLVESGGGLVQPGGSLRLSCAASGFTFTDYYMSWVRQAPGKGLE WVGFIRNKGNAYTTENSASVKGRFTISRDNAKSSLYLQMNSLRAEDT AVYYCARYRGNPHYAMDYWGQGTLVTVSS | 254 |
| 393E9 VH.V6 NS-YS | EVQLVESGGGLVQPGGSLRLSCAASGFTFTDYYMSWVRQAPGKGLE WVGFIRNKGNGYTTEYSASVKGRFTISRDNAKSSLYLQMNSLRAEDT AVYYCARYRGNPHYAMDYWGQGTLVTVSS | 255 |
| 393E9 VH.V6 NG-NA NS-YS | EVQLVESGGGLVQPGGSLRLSCAASGFTFTDYYMSWVRQAPGKGLE WVGFIRNKGNAYTTEYSASVKGRFTISRDNAKSSLYLQMNSLRAEDT AVYYCARYRGNPHYAMDYWGQGTLVTVSS | 256 |

**Table 17-2. 393E9 Humanized antibodies with removal of potential PTM site**

| **VL VH** | 393E9 hVL2 | 393E9 hVL3 | 393E9 hVL5 |
|---|---|---|---|
| 393E9 VH.V4 | | | Hu393E9-45 |
| 393E9 VH.V4 (NG-NA) | | | Hu393E9-45-P1 |
| 393E9 VH.V4 (NS-YS) | | | Hu393E9-45-P2 |
| 393E9 VH.V4(NG-NA/ NS-YS) | | | Hu393E9-45-P3 |
| 393E9 VH.V5 | | Hu393E9-53 | |
| 393E9 VH.V5(NG-NA) | | Hu393E9-53-P1 | |
| 393E9 VH.V5(NS-YS) | | Hu393E9-53-P2 | |
| 393E9 VH.V5(NG-NA/ NS-YS) | | Hu393E9-53-P3 | |
| 393E9 VH.V6 | Hu393E9-62 | | |
| 393E9 VH.V6(NG-NA) | Hu393E9-62-P1 | | |
| 393E9 VH.V6(NS-YS) | Hu393E9-62-P2 | | |
| 393E9 VH.V6(NG-NA/ NS-YS) | Hu393E9-62-P3 | | |

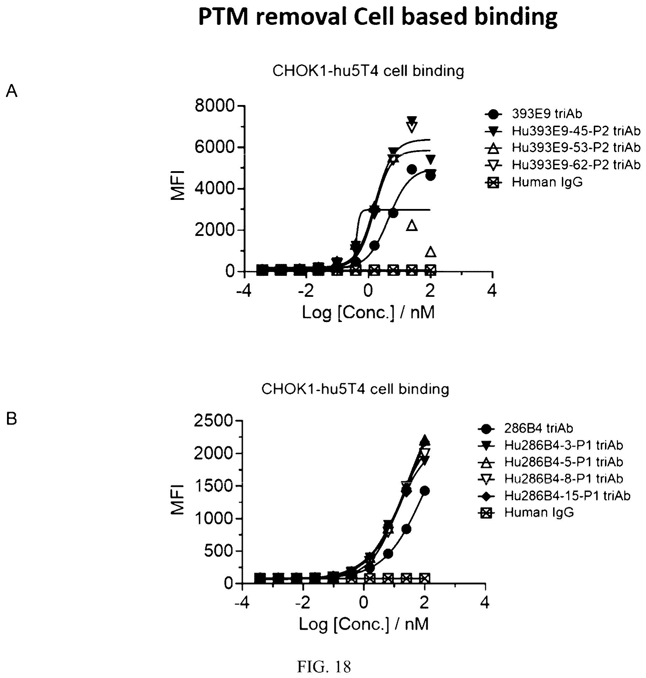

To evaluate the binding property of PTM removal antibodies to surface 5T4, trispecific antibodies with anti-5T4 portion of Hu393E9-45-P2, Hu393E9-53-P2, Hu393E9-62-P2 or chimeric 393E9 were analyzed for their binding to CHOK1-hu5T4 cells by FACS. A total number of 1X10⁵ cells in each well were incubated with 4-fold serial diluted antibodies starting from 100 nM for 30 minutes at 4°C. After wash by FACS buffer, PE conjugated-anti-human IgG antibody was added to each well and incubated at 4°C for 30 minutes. MFI of PE was evaluated by MACSQuant Analyzer 16. As shown in **FIG. 18A****,** the trispecific antibodies with anti-5T4 portion of Hu393E9-45-P2 or Hu393E9-62-P2 showed enhanced binding capacity on 5T4-expressing cells than that with chimeric 393E9. The only difference among tested trispesicifc antibodies was the anti-5T4 portion.

### 12.2 PTM removal confirmation of 159D5

The 159D5 VH CDR2 include DS residues (Kabat numbering) which are at risk of post-translational modifications (PTM) and pose challenges for future manufacturing. Therefore, this example mutated DS to DA on the VH to prevent PTM. The sequences of the potential PTM removal site are listed in **Table 18.** As shown in **FIG. 14B, FIG. 15** and **Table 13,** PTM removal antibody of 159D5-P1 showed comparable binding ability to 159D5 chimeric antibody of 159D5-C.

**Table 18-1. PTM removal of 159D5 CDRH2**

| **VH VL** | **159D5-VH DS-DA** | **159D5-VH** |
|---|---|---|
| **159D5-VL** | 159D5-P1 | 159D5-C |

**Table 18-2. Humanization of 159D5 with removal of potential PTM site**

| **Name** | **Sequence** | **SEQ ID NO:** |
|---|---|---|
| 159D5 VH.V0 (grafted) DS-DA | EVQLVESGGGVVQPGRSLRLSCAASGFTFSNFGMHWVRQAPGKGLEW VAYISSGSSTFYYADAVKGRFTISRDNSKNTLYLQMNSLRAEDTAVYYC ARSPAYYRYGLDYWGQGTTVTVSS | 257 |
| 159D5 VL.V1 | DIVMTQTPLSLSVTPGQPASISCRASQSVSSSTFSYMHWYQQKPGQPPQ LLIKSSSNLESGVPDRFSGSGSGTDFTLKISRVEAEDVGVYYCQHSWEIP YTFGGGTKVEIK | 258 |

**Table 18-3. 159D5 Humanized antibodies with removal of potential PTM site**

| **VH VL** | 159D5 VH.V0 DS-DA |
|---|---|
| 159D5 VL.V1 | Hu159D5-2-P1 |

### 12.3 PTM removal confirmation of 286B4

The 286B4 VH CDR2 include DG residues (Kabat numbering) which are at risk of post-translational modifications (PTM) and pose challenges for future manufacturing. Therefore, this example mutated DG to DA on the VH to prevent PTM. The sequences of the potential PTM removal site are listed in **Table 19.**

**Table 19-1. Humanization of 286B4 with removal of potential PTM site**

| **Name** | **Sequence** | **SEQ ID NO:** |
|---|---|---|
| 286B4 VH.V1 DG-DA | EVQLQESGPGLVKPSDTLSLTCAVSGYSISNNYYWNWIRQPPGKGLEWIG YITYDASNNYNPSLKNRVTMSRDTSKNQFSLKLSSVTAVDTAVYYCARG GGQLRFDYWGQGTTVTVSS | 259 |
| 286B4 VH.V2 DG-DA | EVQLQESGPGLVKPSDTLSLTCAVSGYSISNNYYWNWIRQPPGKGLEWIG YITYDASNNYNPSLKNRITMSRDTSKNQFSLKLSSVTAVDTAVYYCARG GGQLRFDYWGQGTTVTVSS | 260 |
| 286B4 VH.V3 DG-DA | EVQLQESGPGLVKPSDTLSLTCAVSGYSISNNYYWNWIRQPPGKGLEWM GYITYDASNNYNPSLKNRITVSRDTSKNQFSLKLSSVTAVDTAVYYCARG GGQLRFDYWGQGTTVTVSS | 261 |

**Table 19-2: 286B4 Humanized antibodies with removal of potential PTM site**

| **VL VH** | 286B4 hVL3 | 286B4 hVL5 |
|---|---|---|
| 286B4 VH.V1 | Hu286B4-3 | Hu286B4-5 |
| 286B4 VH.V1 DG-DA | Hu286B4-3-P1 | Hu286B4-5-P1 |
| 286B4 VH.V2 | Hu286B4-8 | |
| 286B4 VH.V2 DG-DA | Hu286B4-8-P1 | |
| 286B4 VH.V3 | | Hu286B4-15 |
| 286B4 VH.V3 DG-DA | | Hu286B4-15-P1 |

To evaluate the binding property of PTM removal antibodies to surface 5T4, trispecific antibodies with anti-5T4 portion of Hu286B4-3-P1, Hu286B4-5-P1, Hu286B4-8-P1, Hu286B4-15-P1 or chimeric 286B4 were analyzed for their binding to CHOK1-hu5T4 cells by FACS. A total number of 1X10⁵ cells in each well were incubated with 4-fold serial diluted antibodies starting from 100 nM for 30 minutes at 4°C. After wash by FACS buffer, PE conjugated-anti-human IgG antibody was added to each well and incubated at 4°C for 30 minutes. MFI of PE was evaluated by MACSQuant Analyzer 16. As shown in **FIG. 18B****,** the trispecific antibodies with PTM removal 286B4 fragment showed enhanced binding capacity on 5T4-expressing cells than that with chimeric 286B4 fragment. The only difference among tested trispecific antibodies was the anti-5T4 portion.

### Example 13. Affinity Maturation of Humanized Antibodies Hu14G12-28

In order to enhance the affinity of humanized antibodies of Hu14G12-28, affinity maturation was performed. Briefly, 4 phage libraries containing single-point or two-point saturation mutation in CDR regions of Hu14G12-28 were constructed. Two candidates with unique mutations in CDRs were obtained by 1 round of screening with solid or liquid panning. CDRs of these candidates were engrafted into Hu14G12-28 frameworks to generate antibodies for further binding and affinity validation.

The amino acid sequences of the variable regions of frameworks engrafted affinity maturated candidates are listed in **Table 20** below.

**Table 20-1. Sequences of the variable regions of affinity maturated antibodies**

| **Name** | **Sequence** | **SEQ ID NO:** |
|---|---|---|
| 14G12 hVH2 | QVQLVQSGAEVKKPGASVKVSCKASGYAFSSSWMNWVRQAPGQG LEWMGRIYREDGHTNYNGKFKGRVTMTADKSITTAYMELSRLRSD DTAVYYCANGGAMDYWGQGTLVTVSS | 197 |
| 14G12 hVL1-88# | DIQMTQSPSSLSASVGDRVTITCRASQDISNYLNWYQQKPGKAPKL LIYYTSRLHSGVPSRFSGSGSGTDYTFTISSLQPEDIATYFCGNDNTL PWTFGGGTKVEIK | 262 |
| 14G12 hVH2 | QVQLVQSGAEVKKPGASVKVSCKASGYAFSSSWMNWVRQAPGQG LEWMGRIYREDGHTNYNGKFKGRVTMTADKSITTAYMELSRLRSD DTAVYYCANGGAMDYWGQGTLVTVSS | 197 |
| 14G12 hVL1-108# | DIQMTQSPSSLSASVGDRVTITCRASQDISNYLNWYQQKPGKAPKL LIYYTSRLHSGVPSRFSGSGSGTDYTFTISSLQPEDIATYFCANDNTL PWTFGGGTKVEIK | 263 |

**Table 20-2. Affinity maturated antibodies from Hu14G12-28**

| **VL VH** | **14G12-hVL1-88#** | **14G12-hVL1-108#** |
|---|---|---|
| **14G12-hVH2** | Hu14G12-28-88# | Hu14G12-28-108# |

**Table 20-3. CDR sequences of Hu14G12-28-88#**

| **Hu14G12-28-88#** | **Sequence** | **SEQ ID NO:** |
|---|---|---|
| CDRH1 | SSWMN | 41 |
| CDRH2 | RIYREDGHTNYNGKFK G | 42 |
| CDRH3 | GGAMDY | 43 |
| CDRL1 | RASQDISNYLN | 44 |
| CDRL2 | YTSRLHS | 45 |
| CDRL3 | GNDTLPWT | 264 |

**Table 20-4. CDR sequences of Hu14G12-28-108#**

| **Hu14G12-28-108#** | **Sequence** | **SEQ ID NO:** |
|---|---|---|
| CDRH1 | SSWMN | 41 |
| CDRH2 | RIYREDGHTNYNGKFK G | 42 |
| CDRH3 | GGAMDY | 43 |
| CDRL1 | RASQDISNYLN | 44 |
| CDRL2 | YTSRLHS | 45 |
| CDRL3 | ANDNTLPWT | 265 |

### 13.1 ELISA binding to 5T4

To evaluate the binding activity of affinity maturated antibodies to human 5T4, the Hu14G12-28 along with Hu14G12-28-88# and Hu14G12-28-108# were subjected to ELISA test.

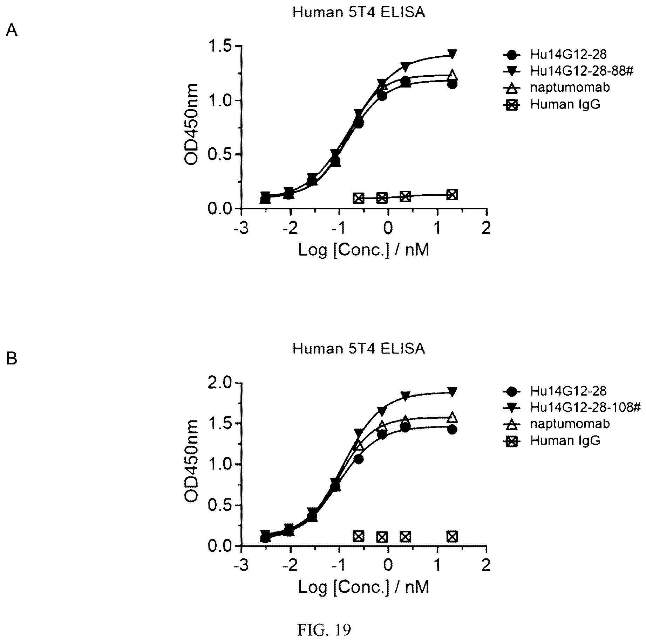

Briefly, microtiter plates were coated with human 5T4-His protein at 2 µg/ml in PBS, 30µl/well at 4°C overnight, then blocked with 5% PBS/Milk. Serial dilutions of antibodies were added to each well and incubated for 1 hour at room temperature. The plates were washed with PBS/Tween and then incubated with Goat Anti-Human IgG-HRP for 50 mins at room temperature. After washing, the plates were developed with TMB substrate and analyzed by spectrophotometer at OD 450nm. As shown in **FIG. 19****,** the affinity maturated antibodies bound to human 5T4 with higher potency than the parental Hu14G12-28 antibody.

### 13.2 Cell-based binding to 5T4

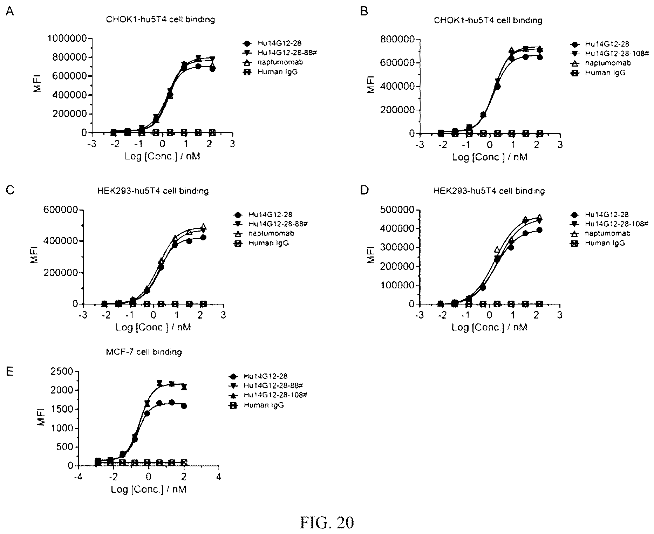

To evaluate the binding property of affinity maturated antibodies to surface 5T4, tested antibodies were analyzed for their binding to CHOK1-hu5T4, HEK293-hu5T4 or MCF-7 cells by FACS. A total number of 1X10⁵ cells in each well were incubated with 4-fold or 5-fold serial diluted antibodies starting from 133 nM or 100 nM for 60 minutes at 4°C. After wash by FACS buffer, PE conjugated-anti-human IgG antibody was added to each well and incubated at 4°C for 30 minutes. MFI of PE was evaluated by MACSQuant Analyzer 16. As shown in **FIG. 20A-B** (CHOK1-hu5T4 cells), **FIG. 20C-D** **(HEK293-hu5T4 cells)** and **FIG. 20E** **(MCF-7 cells),** the affinity maturated antibodies showed enhanced binding capacity on 5T4-expressing cells than the parental Hu14G12-28 antibody.

### 13.3 Binding affinity to 5T4

The binding affinity of tested antibodies against recombinant 5T4 protein (human 5T4-his tag) was tested with Biacore using a capture method. The antibodies were captured using Protein A chip. A serial dilution of human 5T4-his tag protein was injected over captured antibody for 3 mins at a flow rate of 30 µl/min. The antigen was allowed to dissociate for 300s. All the experiments were carried out on a Biacore T200. Data analysis was carried out using Biacore T200 evaluation software.

As shown in the results of **Table 21** below, the affinity maturated antibodies Hu14G12-28-88# and Hu14G12-28-108# significantly increased binding affinity against human 5T4 protein by 9.45 fold and 7.41 fold respectively, compared to the parental Hu14G12-28 antibody, which makes them ideal anti-5T4 antibody for antibody-drug conjugates (ADC) and for bispecific antibodies.

**Table 21. Affinity measured by Biacore**

| **Abs** | **Human 5T4-His** | | |
|---|---|---|---|
| | **ka (1/Ms)** | **kd (1/s)** | **KD (M)** |
| Hu14G12-28 | 1.80 x 10⁵ | 7.38 x 10⁻⁴ | 4.09 x 10⁻⁹ |
| Hu14G12-28-88# | 1.91 x 10⁵ | 8.28 x 10⁻⁵ | 4.33 x 10⁻¹⁰ |
| Hu14G12-28-108# | 1.83 x 10⁵ | 1.01 x 10⁻⁴ | 5.52 x 10⁻¹⁰ |

The present disclosure is not to be limited in scope by the specific embodiments described which are intended as single illustrations of individual aspects of the disclosure, and any compositions or methods which are functionally equivalent are within the scope of this disclosure. It will be apparent to those skilled in the art that various modifications and variations can be made in the methods and compositions of the present disclosure without departing from the spirit or scope of the disclosure. Thus, it is intended that the present disclosure cover the modifications and variations of this disclosure provided they come within the scope of the appended claims and their equivalents.

All publications and patent applications mentioned in this specification are herein incorporated by reference to the same extent as if each individual publication or patent application was specifically and individually indicated to be incorporated by reference.

## Claims

1. An antibody or antigen-binding fragment thereof which has specificity to the human 5T4 oncofetal trophoblast glycoprotein (5T4) protein and comprises a heavy chain variable region (VH) comprising a VH CDR1, a VH CDR2 and a VH CDR3, and a light chain variable region (VL) comprising a VL CDR1, a VL CDR2, and a VL CDR3, wherein the VH CDR1, VH CDR2, VH CDR3, VL CDR1, VL CDR2 and VL CDR3 comprise, respectively, the amino acid sequences of
SEQ ID NO: 54, 55 (or any one of 60-63, or 266 or 267), 56, 57, 58 and 59;
SEQ ID NO: 123, 124 or 129, 125, 126, 127 and 128; or
SEQ ID NO: 160, 161 or 166, 162, 163, 164 and 165.

2. The antibody or antigen-binding fragment thereof of claim 1, wherein:
the VH CDR1 comprises the amino acid sequence of SEQ ID NO: 54;
the VH CDR2 comprises an amino acid sequence selected from the group consisting of SEQ ID NO: 55, 60-63 and 266-267;
the VH CDR3 comprises the amino acid sequence of SEQ ID NO: 56;
the VL CDR1 comprises the amino acid sequence of SEQ ID NO: 57;
the VL CDR2 comprises the amino acid sequence of SEQ ID NO: 58; and
the VL CDR3 comprises the amino acid sequence of SEQ ID NO: 59.

3. The antibody or antigen-binding fragment thereof of claim 2, wherein the VH comprises an amino acid sequence selected from the group consisting of SEQ ID NO: 3, 215-221 and 248-256, and the VL comprises an amino acid sequence selected from the group consisting of SEQ ID NO: 4 and 222-226.

4. The antibody or antigen-binding fragment thereof of claim 1, wherein:
the VH CDR1 comprises the amino acid sequence of SEQ ID NO: 123;
the VH CDR2 comprises an amino acid sequence selected from the group consisting of SEQ ID NO: 124 and 129;
the VH CDR3 comprises the amino acid sequence of SEQ ID NO: 125;
the VL CDR1 comprises the amino acid sequence of SEQ ID NO: 126;
the VL CDR2 comprises the amino acid sequence of SEQ ID NO: 127; and
the VL CDR3 comprises the amino acid sequence of SEQ ID NO: 128.

5. The antibody or antigen-binding fragment thereof of claim 4, wherein the VH comprises an amino acid sequence selected from the group consisting of SEQ ID NO: 23, 236-241 and 259-261, and the VL comprises an amino acid sequence selected from the group consisting of SEQ ID NO: 24 and 242-247.

6. The antibody or antigen-binding fragment thereof of claim 1, wherein:
the VH CDR1 comprises the amino acid sequence of SEQ ID NO: 160;
the VH CDR2 comprises an amino acid sequence selected from the group consisting of SEQ ID NO: 161 and 166;
the VH CDR3 comprises the amino acid sequence of SEQ ID NO: 162;
the VL CDR1 comprises the amino acid sequence of SEQ ID NO: 163;
the VL CDR2 comprises the amino acid sequence of SEQ ID NO: 164; and
the VL CDR3 comprises the amino acid sequence of SEQ ID NO: 165.

7. The antibody or antigen-binding fragment thereof of claim 6, wherein the VH comprises an amino acid sequence selected from the group consisting of SEQ ID NO: 35, and the VL comprises an amino acid sequence selected from the group consisting of SEQ ID NO: 36.

8. The antibody or fragment thereof of any one of claims 1-7, wherein the antibody or fragment thereof is a bivalent Fab antibody, or a fragment selected from the group consisting of F(ab')2, F(ab)2, Fab', Fab, Fv, and scFv.

9. An antibody-drug conjugate comprising an antibody or fragment thereof of any one of claims 1-8 conjugated to a drug moiety.

10. The antibody-drug conjugate of claim 9, wherein the drug moiety is a cytotoxic or cytostatic agent.

11. The antibody-drug conjugate of claim 10, wherein the drug moiety is a maytansinoid, an auristatin, or a macrocyclic ketone analogue.

12. The antibody-drug conjugate of claim 11, wherein the drug moiety comprises monomethyl auristatin E (MMAE) or monomethyl auristatin F (MMAF).

13. The antibody-drug conjugate of any one of claims 9-12, wherein the drug moiety is attached to the antibody or fragment thereof through a linker that is hydrolyzable under acidic conditions.

14. A multispecific antibody comprising an antigen-binding fragment of any one of claims 1-8 and one or more antibody or antigen-binding fragment having binding specificity to a target antigen that is not 5T4.

15. A chimeric antigen receptor (CAR) comprising an antigen-binding fragment of any one of claims 1-8, a transmembrane domain, a costimulatory domain, and a CD3ξ intracellular domain.

16. One or more polynucleotide(s) encoding the antibody or antigen-binding fragment thereof of any one of claims 1-8 or the CAR of claim 15.

17. The polynucleotide(s) of claim 16, which is one or more mRNA.

18. The polynucleotide(s) of claim 17, wherein the mRNA is chemically modified.

19. A cell comprising the polynucleotide(s) of any one of claims 16-18.

20. A composition comprising the antibody or antigen-binding fragment thereof of any one of claims 1-8, the antibody-drug conjugate of any one claims 9-13, the multispecific antibody of claim 14, the CAR of claim 15, the polynucleotide(s) of any one of claims 16-18, or the cell of claim 19, and a pharmaceutically acceptable carrier.

21. A method of treating cancer in a patient in need thereof, comprising administering to the patient an effective amount of the antibody or antigen-binding fragment thereof of any one of claims 1-8, the antibody-drug conjugate of any one claims 9-13, the multispecific antibody of claim 14, the CAR of claim 15, the polynucleotide(s) of any one of claims 16-18, or the cell of claim 19.

22. Use of the antibody or antigen-binding fragment thereof of any one of claims 1-8, the antibody-drug conjugate of any one claims 9-13, the multispecific antibody of claim 14, the CAR of claim 15, the polynucleotide(s) of any one of claims 16-18, or the cell of claim 21 for the preparation of a medicament for treating cancer.
